# EUROPEAN PATENT APPLICATION

(11) **EP 4 342 471 A1**
(43) Date of publication of application: **27.03.2024**
(21) Application number: 22804085.3
(22) Date of filing: 20.05.2022
(51) Int. Cl.: A61K 31/517, A61K 31/428, A61K 31/138, A61P 25/02, A61P 25/28, A61P 25/00, A61P 9/10, A61P 27/02, A61P 29/00, A61P 35/00

(54) **METHOD FOR MODULATING NEUROPATHIES**

(30) Priority: 21.05.2021 CN 202110560773; 28.01.2022 CN 202210110224
(71) Applicant: Chengdu Wending Technology Development Co., Ltd., Chengdu, Sichuan 610041 (CN)
(72) Inventor: ZENG, Wen, Chengdu City, Sichuan 610041 (CN); GONG, Li, Chengdu City, Sichuan 610041 (CN)
(74) Representative: Jones Day
(86) International application number: PCT/CN2022/094251
(87) International publication number: WO 2022/242766

(57) **Abstract**

The present invention relates to a method for modulating neuropathies. The method comprises: administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

## Description

### Cross-Reference to Related Applications

The present application claims priority to Chinese Invention Patent Application No. 202110560773.5, filed on May 21, 2021, the content of which is hereby incorporated by reference in its entirety.

The present application claims priority to Chinese Invention Patent Application No. 202210110224.2, filed on January 28, 2022, the content of which is hereby incorporated by reference in its entirety.

The present application claims that the Chinese Invention Patent Application No. 202010794864.0, filed on August 10, 2020, is incorporated herein by reference in its entirety.

The present application claims that the International Patent Application No. PCT/CN2021/106355, filed on July 14, 2021, is incorporated herein by reference in its entirety.

### Technical Field

The present invention disclosure relates to the field of neuropathy, in particular to methods for modulating neuropathy, especially to methods for treating, preventing, and improving a neuropathy-related disease, disorder or condition.

### Background

Neuropathy includes central neuropathy and peripheral neuropathy. Clinically common and serious central neuropathy includes cerebrovascular diseases (such as stroke, traumatic brain injury), neurodegenerative diseases, optic neuropathy, etc. Cerebrovascular diseases cause more than 1.5 million deaths every year, in which stroke is one of the main clinical types. About 82% of stroke patients are ischemic stroke. Clinical therapeutic measures mainly include intravenous thrombolysis and intravascular interventional therapy, to timely recover cerebral blood flow, reconstruct blood circulation in ischemic areas and rescue ischemic penumbra. Traumatic brain injury is a main cause of death and disability. In 2019, there were about 61,000 TBI-related deaths in the U.S. Every day, there are about 166 deaths related to traumatic brain injury (TBI).

In current clinical practice, intravenous thrombolysis (IVT) such as injectable alteplase (produced by Boehringer Ingelheim Pharma GmbH&Co.KG) is given within 3 hours after the onset of acute ischemic stroke. Alteplase is the only drug approved by the FDA to treat acute ischemic stroke. Since there is a strict time window limit for intravenous thrombolysis, only about 3% of the patients benefit from this treatment. At present, there are still 70% of stroke patients with varying degrees of labor loss, and more than 40% of them are severely disabled. At the same time, there are studies that have shown that intravenous thrombolysis cannot reduce the mortality rate of patients 3 to 6 months after stroke (EMBERSON J, LEES K R, LYDEN P, et al. Effect of treatment delay, age, and stroke severity on the effects of intravenous thrombolysis with alteplase for acute ischaemic stroke: a meta-analysis of individual patient data from randomised trials [J]. Lancet, 2014, 384(9958): 1929-1935.), especially in patients with large vessel occlusion or severe illness, intravenous thrombolysis tends to have unsatisfactory effects, with a recanalization rate of only 13%-18% (GUPTA R, JOVIN T G. Endovascular management of acute ischemic stroke: advances in patient and treatment selection [J]. Expert Rev Neurother, 2007, 7 (2): 143-153).

One of the main goals of treating stroke is neuroprotection. For patients who have already suffered from stroke, it is extremely important to prevent and delay the death of nerve cells, which may preserve more nerve cells, so as to restore the functions and gain more time for treatments. However, at present, there is a lack of safe and effective neuroprotective agents, and neuroprotection is a seriously unmet clinical medical need (Wu, S. et al. Stroke in China: advances and challenges in epidemiology, prevention, and management. Lancet Neurol. 18, 394-405 (2019).; Kim, A.S., Cahill, E. & Cheng, N.T. Global Stroke Belt: Geographic Variation in Stroke Burden Worldwide. Stroke 46, 3564-3570 (2015)).

Dementia, a cognitive, emotional disorder and social hypofunction syndrome, is the most common neurodegenerative disease and is currently the seventh leading cause of death of all diseases, and one of the leading causes of disability and dependency among the elderly population globally. According to the statistics of the World Health Organization (WHO), about 55 million people worldwide are currently living with dementia, with nearly 10 million new cases each year, and the global annual burden is estimated to be about 1 trillion US dollars. It is estimated that by 2050 there will be 135 million people with dementia.

There are many types of dementia, including Alzheimer's disease, vascular dementia, Lewy body dementia and frontotemporal dementia. Dementia may also develop after stroke or in cases of certain infections, repetitive physical injuries to the brain, or nutritional deficiencies. The boundaries between different forms of dementia are blurred, but cerebrovascular injury is often present in various dementia (Gg A, Cq A, Lfa B. Prevention of dementia in an ageing world: Evidence and biological rationale. Ageing Research Reviews, 2020, 64:101045).

In the past 40 years, a large number of clinical trials for treating dementia have all failed. At present, there is a lack of safe and effective neuroprotective agents in clinic, and neuroprotection is a seriously unmet clinical medical need (Jca B, Gl B, Ar B, et al. Alzheimer's disease drug development pipeline: 2019. Alzheimer's & Dementia: Translational Research & Clinical Interventions, 2019, 5:272-293; Panza F, Lozupone M, Logroscino G, et al. A critical appraisal of amyloid-β-targeting therapies for Alzheimer disease. Nature Reviews Neurology, 2019, 15(2):73-88).

Retinal ischemia-reperfusion (I/R) injury is a pathophysiological process of cell injury leading to many eye diseases such as optic neuropathy (including glaucoma, ischemic optic neuropathy, retinal vascular occlusion, and various retinopathies) (Khanh Vu, Dong Feng Chen. "CD4+ T-Cell Responses Mediate Progressive Neurodegeneration in Experimental Ischemic Retinopathy." The American Journal of Pathology 190, no. 8 (August 2020): 1723-34). Retinal ischemia-reperfusion (I/R) induced retinal ganglion cells (RGCs) and internal retinal injuries are common causes of visual impairment in middle-aged people, irreversible visual impairment and blindness in the elderly, which are seriously unmet medical needs and have no effective therapeutic methods (Soares R O S, Losada D M, Jordani M C, et al. Ischemia/Reperfusion Injury Revisited: An Overview of the Latest Pharmacological Strategies [J]. International Journal of Molecular Sciences, 2019, 20(20):5034).

Glaucoma is a group of eye diseases characterized by progressive retinal ganglion cell (RGC) death and typical visual field defect, which has become a main cause of irreversible blindness in the world. At present, the clinical therapeutic regimen is mainly to reduce intraocular pressure, which only has protective effect on the visual function of some glaucoma patients. Therefore, it is very important to develop new therapeutic methods to provide safe and effective RGC neuroprotection. In a word, there is still a lack of safe and effective drugs for these diseases in clinic, so it is urgent to find new drugs and therapies.

Retinal vein occlusion (RVO) is a retinal vascular disease which leads to severe visual loss. Patients with RVO may develop neovascular glaucoma (NVG), leading to complete blindness. At present, intravitreal injection of an anti-VEGF drug is a first-line therapeutic regimen. However, due to the short half-life period of the drug, the drug needs to be intraocularly injected frequently for treatment, which increases the potential risk of endophthalmitis and causes problems such as easy relapse, and will incur partial atrophy of chromatophore and photoreceptor cell layers after long-term use. Therefore, it is urgent to find new RVO therapeutic drugs in clinic.

Diabetes is an increasingly prevalent disease, which has a great impact on human health and quality of life. It is estimated that in 2040, the number of diabetic patients aged between 20 and 79 in the world will reach 642 million, accounting for 8.8% of the total population in the world. Up to 50% of diabetic patients will develop Diabetic Peripheral Neuropathy (DPN). DPN patients need relatively high nursing costs, and patients will have huge physiological, psychological, and economic burdens.

Existing therapeutic means and drugs are still unsatisfactory in prevention and control of DPN progress and effectiveness. Epalrestat is a novel aldose reductase inhibitor via a polyol pathway, which can improve the symptoms of diabetic peripheral neuropathy and peripheral nerve conduction velocity. However, the use of epalrestat will lead to some side effects, especially allergy, liver and kidney function and digestive system adverse reactions after long-term use, which will lead to poor therapeutic effect, and it cannot control the progression of neuropathy in long term (Hotta, Nigishi, Akanuma et al. Long-Term Clinical Effects of Epalrestat, an Aldose Reductase Inhibitor, on Diabetic Peripheral Neuropathy. [J]. Diabetes Care, 2006; Sato K, Yama K, Yu M, et al. Epalrestat increases intracellular glutathione levels in Schwann cells through transcription regulation[J]. Redox Biology, 2014; Epalrestat. Reactions Weekly, 2011, 1369(1): 18-18; Fujise Y, Koda M, Kato J, et al. Drug-induced hepatic injury caused by an aldose reductase inhibitor, epalrestat[J]. Kanzo, 2011, 52(6):351-355.). At present, epalrestat has not been approved by the US FDA. Therefore, there is still no drug that can control the progress of neuropathy in DPN patients in long term, and it is urgent to find new drugs and therapies for clinical prevention and treatment of diabetic peripheral neuropathy (Zakin E, Abrams R, Simpson D M. Diabetic Neuropathy [J]. Seminars in Neurology, 2019, 39(05):560-569).

A considerable number of DPN patients are accompanied by severe neuropathic pain, namely Diabetic Peripheral Neuropathy Pain (DPNP). This puts a huge burden on patients' lives, leading to an increase in unemployment rate and an increase in the proportion of mental health disorders and physical complications. Therapies for diabetic painful peripheral neuropathy are aimed at relieving symptoms, but the current drugs are effective in only one-third of patients, and cannot relieve pain completely, accompanied by side effects, poor efficacy, etc. Therefore, it is very important to develop potential new therapeutic targets to alleviate diabetic painful peripheral neuropathy (Gordon S, Pallai S, Dinesh S, et al. A new look at painful diabetic neuropathy[J]. Diabetes Research and Clinical Practice, 2018, 144:177-191.).

Doxazosin is used clinically for symptomatic treatment of benign prostatic hyperplasia and hypertension. Adults are often administered orally, with an initial treatment dosage of 1 mg once daily, and after 1-2 weeks, the dosage is adjusted according to the clinical response and tolerance. The maintenance treatment dosage is 1-8 mg once daily, the maximum dosage is up to 16 mg/day, and a dosage of more than 4 mg is prone to cause postural hypotension.

Pramipexole is clinically used to treat the signs and symptoms of adult idiopathic Parkinson's disease, and is also used to treat the symptoms of moderate to severe idiopathic restless legs syndrome. Adults are often administered orally, 3 times a day, with an initial dosage of 0.375 mg per day, and then an increase of a dosage every 5-7 days, with a maximum daily dosage of 4.5 mg. The maintenance treatment dosage is between 0.375 mg and 4.5 mg per day.

Metoprolol is clinically an antiarrhythmic drug, and its indications are hypertension, angina pectoris, etc. The common dosage for adults to treat hypertension is 100-200 mg/time, twice a day. In the treatment of acute myocardial infarction, it is recommended that the drug be administered in the early stage, i.e., within the first few hours, with a general method of use of intravenously administering 2.5-5 mg/time of metoprolol (within 2 minutes) first, once every 5 minutes, for a total of 3 dosages of 10-15 mg; and 15 minutes later, administering orally at 25-50 mg/time, once every 6-12 hours for 24-48 hours, and then administering orally at 50-100 mg/time twice daily.

U.S. Patent No. US10117868 B2 mentions a method of using a composition of doxazosin and metoprolol for treating ocular diseases that are associated with light induced retinal degeneration, aberrant all-trans-retinal clearance, or reactive oxygen species generation. Some literature also reported the inhibitory effect of a composition of metoprolol, bromocriptine and doxazosin on early diabetic retinopathy (DR) in diabetic mice (Kern T S, Du Y, Tang J, et al. Regulation of adrenergic, serotonin and dopamine receptors to inhibit diabetic retinopathy: monotherapies versus combination therapies[J]. Molecular Pharmacology, 2021: MOLPHARM-AR-2021-000278.).

### Summary

The present invention provides a method for modulating neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The method for modulating neuropathy involves inhibiting neuroinflammation or repairing nerve injury. In certain embodiments, the method treats, prevents, or improves neuroinflammation-related diseases, including ischemic stroke, hemorrhagic stroke, transient ischemic attack, craniocerebral injury, vascular dementia, Alzheimer's disease, Lewy body dementia, frontotemporal dementia, glaucoma, ischemic optic neuropathy, optic neuritis, optic nerve tumor, traumatic optic neuropathy, retinal artery occlusion, retinal vein occlusion, and retinopathy of prematurity, by inhibiting neuroinflammation.

In certain embodiments, the method for modulating neuropathy further involves treating, preventing, or improving a neuropathy-related disease, disorder, or condition, wherein the disease, disorder, or condition includes a central nervous system disease, disorder, or condition, and peripheral neuropathy, wherein peripheral neuropathy includes diabetic peripheral neuropathy. In certain embodiments, the method for treating, preventing, and/or improving peripheral neuropathy involves improving nerve conduction velocity.

In certain embodiments, diabetic peripheral neuropathy is diabetic distal symmetrical polyneuropathy, diabetic mononeuropathy, or multiple mononeuropathy. In certain embodiments, the subject exhibits symmetrical symptoms, and symptomatic manifestations in the subject include distal limb sensory abnormalities, sensory abnormalities in both lower limbs, stocking anesthesia, glove anesthesia, median nerve, ulnar nerve, common peroneal nerve involvement, sensory dysfunction, motor dysfunction, insensitivity to trauma, and neuropathic pain. In certain embodiments, diabetic peripheral neuropathy is diabetic painful peripheral neuropathy. In certain embodiments, the subject's sleep is affected by pain caused by neuropathy and nerve conduction abnormalities, and the subject suffers from depression and anxiety caused by pain or neuropathy.

In certain embodiments, the method for treating, preventing, or improving peripheral neuropathy, particularly diabetic peripheral neuropathy, involves: improving peripheral nerve conduction velocity, and significantly improving sensory nerve conduction velocity of sural nerve. In certain embodiments, the effects of the method on the subject include: improving microcirculation, inhibiting neuroinflammation, inhibiting central nervous inflammation, repairing damaged nerve tissue, relieving symptoms of peripheral neuropathy for a long term, controlling progression of neuropathy for a long term, and controlling progression of neuropathic pain for a long term.

In certain embodiments, the central nervous system disease, disorder, or condition includes cerebrovascular diseases. Cerebrovascular diseases include stroke and transient ischemic attack, wherein the stroke includes ischemic stroke and hemorrhagic stroke, and hemorrhagic stroke includes conditions of cerebral hemorrhage and/or subarachnoid hemorrhage.

In certain embodiments, subjects having an ischemic stroke attack include subjects within 7 hours after the stroke attack, or subjects within 5 hours after the stroke attack. In certain embodiments, the subject has a National Institute of Health Stroke Scale (NIHSS) of 20 or within 20, or 15 or within 15, or 8 to 15. The subject may also be a subject with moderate to severe stroke accompanied by persistent neurologic deficit. In certain embodiments, the subject is a subject with anterior circulation large vessel occlusion. In certain embodiments, the subject has a symptom of occlusion in the M1 or M2 segment of the middle cerebral artery.

In certain embodiments, the subject having an ischemic stroke attack is a subject with ischemia-reperfusion injury, wherein reperfusion includes reperfusion by endovascular therapy, reperfusion by administration of a thrombolytic agent, and/or reperfusion by angioplasty. In certain embodiments, the subject having an ischemic stroke attack is a subject with ischemia non-recanalization and/or a subject with massive cerebral infarction.

In certain embodiments, the method for treating, preventing, or improving stroke, particularly ischemic stroke, involves: improving acute cerebral tissue perfusion in a subject, inhibiting reduction of regional cerebral blood volume (rCBV) around cerebral tissue infarction, improving acute ischemic condition and saving ischemic penumbra; reducing brain edema/infarction volume in the subject; inhibiting central nervous inflammation, and inhibiting overexpression of glial fibrillary acidic protein (GFAP, marker of astrocyte activation); reducing neurobehavioral score of stroke, improving neurologic deficit in the subject, improving and restoring finger function and limb strength of an affected limb in the subject, and reducing the disability rate of the subject; preventing, treating, and improving cognitive dysfunction of the subject, including aspects of memory, visuospatial, and comprehension judgment, and preventing and impeding development of dementia; and/or treating destructive effect of ischemia on the central nervous system of the subject.

In certain embodiments, the method for treating, preventing, or improving transient ischemic attack involves: improving acute cerebral tissue perfusion in a subject, inhibiting reduction of regional cerebral blood volume (rCBV) in areas of injured brain tissue, improving acute (75 hours after stroke) ischemic condition and saving ischemic penumbra; inhibiting central nervous inflammation, and inhibiting overexpression of glial fibrillary acidic protein (GFAP); reducing neurobehavioral score of stroke, improving neurologic deficit in the subject, improving and restoring finger function and limb strength of an affected limb in the subject, and reducing the disability rate of the subject; preventing, treating, and improving cognitive dysfunction of the subject, including aspects of memory, visuospatial, and comprehension judgment, and preventing and impeding development of dementia; and/or treating destructive effect of ischemia on the central nervous system of the subject.

In certain embodiments, the central nervous system disease, disorder, or condition includes craniocerebral injury, wherein craniocerebral injury includes traumatic brain injury. In certain embodiments, the method for treating, preventing, or improving traumatic brain injury involves: inhibiting central nervous inflammation and/or inhibiting overexpression of GFAP.

In certain embodiments, the central nervous system disease, disorder, or condition includes neurodegenerative diseases, and the neurodegenerative diseases include dementia, wherein dementia includes vascular dementia, Alzheimer's disease, Lewy body dementia, and frontotemporal dementia.

In certain embodiments, the method for preventing, treating, and improving dementia involves: preventing, treating, and improving cognitive dysfunction of a subject, including aspects of memory, visuospatial, and comprehension judgment, especially visuospatial cognitive dysfunction, preventing and impeding development of dementia; improving acute cerebral tissue perfusion in the subject, inhibiting reduction of regional cerebral blood volume (rCBV) around cerebral tissue infarction, improving ischemic condition and saving ischemic penumbra; reducing brain edema/infarction volume in the subject; inhibiting central nervous inflammation, and inhibiting GFAP overexpression; and reducing neurobehavioral score and improving neurological deficit, and/or reducing overall disability rate of the subject.

In certain embodiments, the central nervous system disease, disorder, or condition includes optic neuropathy, wherein optic neuropathy includes glaucoma, ischemic optic neuropathy, optic neuritis, optic nerve tumor, and traumatic optic neuropathy. In certain embodiments, the method for treating, preventing, and/or improving optic neuropathy involves inhibiting or relieving retinal ganglion cell (RGC) injury or severe axonal injury in a subject.

In certain embodiments, the glaucoma includes primary glaucoma, secondary glaucoma, and/or developmental glaucoma, wherein the primary glaucoma includes open-angle glaucoma, angle-closure glaucoma, and special types of glaucoma.

In certain embodiments, the method for preventing, treating, and improving glaucoma involves: mitigating retinal ganglion cell (RGC) loss, protecting visual function, and inhibiting or reducing severity of secondary neuronal injury; mitigating internal retinal injury, reducing retinal edema and mitigating retinal thickness reduction; reducing edema of optic nerve fiber layer, maintaining the thickness of retinal nerve fiber layer and preventing the thinning of optic nerve fiber layer; inhibiting optic nerve inflammation and GFAP overexpression, inhibiting the expression level of at least one inflammatory factor associated with nerve cell injury, the inflammatory factors include TNF-α, IL-1β, and/or IFN-γ; mitigating optic nerve function decline including improving functional indexes of full-field electroretinogram (FERG) and flash visual evoked potential (FVEP), and/or protecting overall retinal function.

In certain embodiments, the ischemic optic neuropathy includes anterior ischemic optic neuropathy and/or posterior ischemic optic neuropathy.

In certain embodiments, the method for preventing, treating, and improving ischemic optic neuropathy involves: mitigating retinal ganglion cell (RGC) loss, protecting visual function, and inhibiting or reducing severity of secondary neuronal injury; mitigating internal retinal injury, reducing retinal edema, and mitigating retinal thickness reduction; reducing edema of optic nerve fiber layer, maintaining thickness of retinal nerve fiber layer and preventing thinning of optic nerve fiber layer; inhibiting optic nerve inflammation and GFAP overexpression, inhibiting the expression level of at least one inflammatory factor associated with nerve cell injury, the inflammatory factors include TNF-α, IL-1β, and/or IFN-γ; mitigating optic nerve function decline including improving functional indexes of full-field electroretinogram (FERG), and flash visual evoked potential (FVEP), and protecting overall retinal function.

In certain embodiments, the method for preventing, treating, and improving optic neuritis involves: inhibiting or mitigating retinal ganglion cell (RGC) injury or severe axonal injury in a subject; inhibiting overexpression of GFAP including I/R induced GFAP; inhibiting the expression level of at least one inflammatory factor associated with nerve cell injury, the inflammatory factors include TNF-α, IL-1β, and/or IFN-γ.

In certain embodiments, the method for preventing, treating, and improving optic nerve tumors involves: inhibiting or mitigating retinal ganglion cell (RGC) injury or severe axonal injury in a subject; inhibiting overexpression of GFAP including I/R induced GFAP; and/or inhibiting the expression level of at least one inflammatory factor associated with nerve cell injury, the inflammatory factors include TNF-α, IL-1β, and IFN-γ.

In certain embodiments, the method for preventing, treating, and improving traumatic optic neuropathy involves: inhibiting or mitigating retinal ganglion cell (RGC) injury or severe axonal injury in a subject; inhibiting overexpression of GFAP including I/R induced GFAP; and/or inhibiting the expression level of at least one inflammatory factor associated with nerve cell injury, the inflammatory factors include TNF-α, IL-1β, and IFN-γ.

In certain embodiments, the central nervous system disease, disorder, or condition includes retinopathy. In certain embodiments, the method for treating, preventing, and/or improving retinopathy involves inhibiting or mitigating retinal ganglion cell (RGC) injury or severe axonal injury in a subject. Retinopathy includes retinal vascular occlusion diseases and/or retinopathy of prematurity. Retinal vascular occlusion diseases include retinal artery occlusion and/or retinal vein occlusion, wherein the retinal artery occlusion includes central artery occlusion, branch artery occlusion, ciliary artery occlusion and/or retinal anterior capillary arteriole occlusion; and the retinal vein occlusion includes central vein occlusion and/or branch vein occlusion.

In certain embodiments, the method for preventing, treating, and improving retinal vascular occlusion diseases and/or retinopathy of prematurity involves: mitigating retinal ganglion cell (RGC) loss, protecting visual function, and inhibiting or reducing severity of secondary neuronal injury; mitigating internal retinal injury, reducing retinal edema and slowing retinal thickness reduction; reducing edema of optic nerve fiber layer, maintaining the thickness of retinal nerve fiber layer and preventing thinning of optic nerve fiber layer; significantly inhibiting optic nerve inflammation and GFAP overexpression, inhibiting the expression level of inflammatory factors such as TNF-α, IL-1β, and IFN-γ that are associated with nerve cell injury; and/or mitigating optic nerve function decline including improving functional indexes of full-field electroretinogram (FERG) and flash visual evoked potential (FVEP), and protecting overall retinal function.

In certain embodiments, the subject includes mammals; wherein the mammals include primates, especially humans.

In certain embodiments, the method includes administering to a subject a therapeutically effective amount of doxazosin, a pharmaceutically acceptable salt or an acceptable form thereof, in combination with a therapeutically effective amount of pramipexole, a pharmaceutically acceptable salt or an acceptable form thereof, and further in combination with a therapeutically effective amount of metoprolol, a pharmaceutically acceptable salt or an acceptable form thereof.

In certain embodiments, the method involves administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof simultaneously or separately. When they are administered simultaneously, the three pharmaceutical components may be formulated into a single pharmaceutical composition for administration to the subject.

In certain embodiments, the three pharmaceutical components or the pharmaceutical composition described above are formulated into an oral dosage form, an intravenous dosage form, a subcutaneous dosage form, or a fundal injection dosage form, preferably an oral dosage form. The above three pharmaceutical components or pharmaceutical composition may be formulated into tablets, capsules, syrups, suspensions; intravenous injection, subcutaneous injection, intramuscular injection and intraperitoneal injection; creams, jellies, powders, patches; and inhalation powders, sprays, suspensions, or rectal suppositories, preferably tablets. The above three pharmaceutical components or pharmaceutical composition may be administered orally, intravenously, subcutaneously, or intramuscularly to the subject, preferably orally.

In certain embodiments, the pharmaceutically acceptable salt is selected from hydrochloride, sulfate, phosphate, pyrophosphate, hydrobromide or nitrate, citrate, fumarate, maleate, malate, ascorbate, succinate, tartrate, benzoate, acetate, methanesulfonate, ethanesulfonate, salicylate, stearate, benzenesulfonate, and/or p-toluenesulfonate. In certain embodiments, the pharmaceutically acceptable salt form of doxazosin is methanesulfonate. In certain embodiments, the pharmaceutically acceptable salt form of pramipexole is hydrochloride. In certain embodiments, the pharmaceutically acceptable salt form of metoprolol is tartrate.

In certain embodiments, the daily dosage of doxazosin ranges from 0.4 mg to 16 mg, preferably 0.5 mg to 8 mg, the daily dosage of pramipexole ranges from 0.03 mg to 4.5 mg, preferably 0.1 mg to 1mg, and the daily dosage of metoprolol ranges from 2 mg to 200 mg, preferably 10 mg to 100 mg.

In certain embodiments, the therapeutically effective amount of doxazosin, a pharmaceutically acceptable salt or an acceptable form thereof, the therapeutically effective amount of pramipexole, a pharmaceutically acceptable salt or an acceptable form thereof, and the therapeutically effective amount of metoprolol, a pharmaceutically acceptable salt or an acceptable form thereof are administered to the subject at a frequency of 1, 2, 3, or 4 times per day.

In certain embodiments, the therapeutically effective amount of doxazosin, a pharmaceutically acceptable salt or an acceptable form thereof, the therapeutically effective amount of pramipexole, a pharmaceutically acceptable salt or an acceptable form thereof, and the therapeutically effective amount of metoprolol, a pharmaceutically acceptable salt or an acceptable form thereof are administered to the subj ect in a form of a long-acting oral preparation.

The present invention also provides a method for inhibiting neuroinflammation, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides a method for repairing nerve injury, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides a method for treating, preventing, or improving cerebrovascular diseases, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. Cerebrovascular diseases include stroke and transient ischemic attack, wherein stroke includes ischemic stroke and hemorrhagic stroke, and hemorrhagic stroke includes conditions of cerebral hemorrhage and/or subarachnoid hemorrhage.

In certain embodiments, subjects having an ischemic stroke attack include subjects within 7 hours after the stroke attack, or subjects within 5 hours after the stroke attack. In certain embodiments, the subjects have a baseline National Institute of Health Stroke Scale (NIHSS) of 20 or within 20, or 15 or within 15, or 8 to 15. The subjects may also be subjects with moderate to severe stroke accompanied by persistent neurologic deficit. In certain embodiments, the subjects are subjects with anterior circulation large vessel occlusion. In certain embodiments, the subjects have a symptom of intracranial occlusion in the M1 or M2 segment of the middle cerebral artery.

In certain embodiments, the subjects having an ischemic stroke attack are subjects with ischemia-reperfusion injury, wherein reperfusion includes reperfusion by intravascular therapy, reperfusion by administration of a thrombolytic agent, and/or reperfusion by angioplasty. In certain embodiments, the subjects having an ischemic stroke attack are subjects with ischemia non-recanalization and/or subjects with massive cerebral infarction.

In certain embodiments, the method for treating, preventing, or improving stroke, particularly ischemic stroke, involves: improving acute cerebral tissue perfusion in a subject, inhibiting reduction of regional cerebral blood volume (rCBV) around cerebral tissue infarction, improving acute (75 hours after stroke) ischemic condition after stroke and saving ischemic penumbra; reducing brain edema/infarction volume in the subject; inhibiting central nervous inflammation, and inhibiting overexpression of glial fibrillary acidic protein (GFAP); reducing neurobehavioral score of stroke, improving neurologic deficit in the subject, improving and restoring finger function and limb strength of an affected limb in the subject, and reducing the disability rate of the subject; preventing, treating and improving cognitive dysfunction of the subject, including aspects of memory, visuospatial, and comprehension judgment, and preventing and impeding development of dementia; and/or treating destructive effect of ischemia on the central nervous system of the subject.

In certain embodiments, the method for treating, preventing, or improving transient ischemic attack involves: improving acute cerebral tissue perfusion in a subject, inhibiting reduction of regional cerebral blood volume (rCBV) in areas of injured brain tissue, improving acute ischemic condition and saving ischemic penumbra; inhibiting central nervous inflammation, and inhibiting overexpression of glial fibrillary acidic protein (GFAP); reducing neurobehavioral score of stroke, improving neurologic deficit in the subject, improving and restoring finger function and limb strength of an affected limb in the subject, and reducing the disability rate of the subject; preventing, treating, and improving cognitive dysfunction of the subject, including aspects of memory, visuospatial, and comprehension judgment, and preventing and impeding development of dementia; and/or treating destructive effect of ischemia on the central nervous system of the subject.

The present invention also provides a method for treating, preventing, or improving craniocerebral injury, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. Craniocerebral injury includes traumatic brain injury. In certain embodiments, the method for treating, preventing, or improving traumatic brain injury involves: inhibiting central nervous inflammation and/or inhibiting GFAP overexpression.

The present invention also provides a method for treating, preventing, or improving neurodegenerative diseases, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. The neurodegenerative diseases include dementia, wherein dementia includes vascular dementia, Alzheimer's disease, Lewy body dementia and/or frontotemporal dementia.

In certain embodiments, the method for preventing, treating, and improving dementia involves: preventing, treating, and improving cognitive dysfunction of a subject, including aspects of memory, visuospatial, and comprehension judgment, especially visuospatial cognitive dysfunction, preventing and impeding development of dementia; improving cerebral tissue perfusion in the subject, inhibiting reduction of regional cerebral blood volume (rCBV) around cerebral tissue infarction, improving ischemic condition and saving ischemic penumbra; reducing brain edema/infarction volume in the subject; inhibiting central nervous inflammation, and inhibiting GFAP overexpression; and reducing neurobehavioral score and improving neurologic deficit, and/or reducing an overall disability rate of the subject.

The present invention also provides a method for treating, preventing, or improving glaucoma, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. The glaucoma includes primary glaucoma, secondary glaucoma, and/or developmental glaucoma, wherein the primary glaucoma includes open-angle glaucoma, angle-closure glaucoma, and special types of glaucoma.

In certain embodiments, the method for preventing, treating, and improving glaucoma involves: mitigating retinal ganglion cell (RGC) loss, protecting visual function, and inhibiting or reducing severity of secondary neuronal injury; mitigating internal retinal injury, reducing retinal edema and mitigating retinal thickness reduction; reducing edema of optic nerve fiber layer, maintaining thickness of retinal nerve fiber layer and preventing thinning of optic nerve fiber layer; inhibiting optic nerve inflammation and GFAP overexpression, inhibiting the expression level of at least one inflammatory factor associated with nerve cell injury, the inflammatory factors include TNF-α, IL-1β, and IFN-γ; mitigating optic nerve function decline including improving functional indexes of full-field electroretinogram (FERG) and flash visual evoked potential (FVEP), and/or protecting overall retinal function.

The present invention also provides a method for treating, preventing, or improving ischemic optic neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. The ischemic optic neuropathy includes anterior ischemic optic neuropathy and/or posterior ischemic optic neuropathy.

In certain embodiments, the method for preventing, treating, and improving ischemic optic neuropathy involves: mitigating retinal ganglion cell (RGC) loss, protecting visual function, and inhibiting or reducing severity of secondary neuronal injury; mitigating internal retinal injury, reducing retinal edema and mitigating retinal thickness reduction; reducing edema of optic nerve fiber layer, maintaining thickness of retinal nerve fiber layer and preventing thinning of optic nerve fiber layer; inhibiting optic nerve inflammation and GFAP overexpression, inhibiting the expression level of at least one inflammatory factor associated with nerve cell injury, the inflammatory factors include TNF-α, IL-1β, and IFN-γ; mitigating optic nerve function decline including improving functional indexes of full-field electroretinogram (FERG) and flash visual evoked potential (FVEP), and/or protecting overall retinal function.

The present invention also provides a method for treating, preventing, or improving optic neuritis, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. In certain embodiments, the method for preventing, treating, and improving optic neuritis involves: inhibiting or mitigating retinal ganglion cell (RGC) injury or severe axonal injury in a subject; inhibiting overexpression of GFAP, wherein the GFAP includes I/R induced GFAP; and/or inhibiting the expression level of at least one inflammatory factor associated with nerve cell injury, the inflammatory factors include TNF-α, IL-1β, and IFN-γ.

The present invention also provides a method for treating, preventing, or improving optic nerve tumors, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. In certain embodiments, the method for preventing, treating, and improving optic nerve tumors involves: inhibiting or mitigating retinal ganglion cell (RGC) injury or severe axonal injury in a subject; inhibiting overexpression of GFAP including I/R induced GFAP; and/or inhibiting the expression level of at least one inflammatory factor associated with nerve cell injury, the inflammatory factors include TNF-α, IL-1β, and IFN-γ.

The present invention also provides a method for treating, preventing, or improving traumatic optic neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. In certain embodiments, the method for preventing, treating, and improving traumatic optic neuropathy involves: inhibiting or mitigating retinal ganglion cell (RGC) injury or severe axonal injury in a subject; inhibiting overexpression of GFAP including I/R induced GFAP; and/or inhibiting the expression level of at least one inflammatory factor associated with nerve cell injury, the inflammatory factors include TNF-α, IL-1β, and IFN-γ.

The present invention also provides a method for treating, preventing or improving retinopathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. In certain embodiments, the method for treating, preventing, and/or improving retinopathy involves inhibiting or mitigating retinal ganglion cell (RGC) injury or severe axonal injury in a subject. Retinopathy includes retinal vascular occlusion diseases and/or retinopathy of prematurity. Retinal vascular occlusion diseases include retinal artery occlusion and/or retinal vein occlusion, wherein the retinal artery occlusion includes central artery occlusion, branch artery occlusion, ciliary artery occlusion, and/or retinal anterior capillary arteriole occlusion; and the retinal vein occlusion includes central vein occlusion and/or branch vein occlusion.

In certain embodiments, the method for preventing, treating, and improving retinal vascular occlusion diseases and/or retinopathy of prematurity involves: mitigating retinal ganglion cell (RGC) loss, protecting visual function, and inhibiting or reducing severity of secondary neuronal injury; mitigating internal retinal injury, reducing retinal edema and mitigating retinal thickness reduction; reducing edema of optic nerve fiber layer, maintaining thickness of retinal nerve fiber layer and preventing thinning of optic nerve fiber layer; significantly inhibiting optic nerve inflammation and GFAP overexpression, inhibiting the expression level of inflammatory factors such as TNF-α, IL-1β, and IFN-γ that are associated with nerve cell injury; and mitigating optic nerve function decline including improving functional indexes of full-field electroretinogram (FERG) and flash visual evoked potential (FVEP), and/or protecting overall retinal function.

The present invention also provides a method for inhibiting or mitigating retinal ganglion cell (RGC) injury or severe axonal injury in a subject, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides a method for treating, preventing, or improving peripheral neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. In certain embodiments, the method for treating, preventing, or improving peripheral neuropathy involves improving nerve conduction velocity.

The present invention also provides a method for treating, preventing, or improving diabetic peripheral neuropathy. In certain embodiments, diabetic peripheral neuropathy is diabetic distal symmetrical polyneuropathy, diabetic mononeuropathy, or multiple mononeuropathy. In certain embodiments, the subject exhibits symmetrical symptoms, and symptomatic manifestations in the subject include distal limb sensory abnormalities, sensory abnormalities in both lower limbs, stocking anesthesia, glove anesthesia, median nerve, ulnar nerve, common peroneal nerve involvement, sensory dysfunction, motor dysfunction, insensitivity to trauma, and neuropathic pain. In certain embodiments, diabetic peripheral neuropathy is diabetic painful peripheral neuropathy. In certain embodiments, the subject's sleep is affected by pain caused by neuropathy and nerve conduction abnormalities, and the subject suffers from depression and anxiety caused by pain or neuropathy.

In certain embodiments, the method for treating, preventing, or improving peripheral neuropathy, particularly diabetic peripheral neuropathy, involves: improving peripheral nerve conduction velocity, and significantly improving sensory nerve conduction velocity of sural nerve. In certain embodiments, the effects of the method on the subject include: improving microcirculation, inhibiting neuroinflammation, inhibiting central nervous inflammation, repairing injured nerve tissue, relieving symptoms of peripheral neuropathy for a long term, controlling progression of neuropathy for a long term, and controlling progression of neuropathic pain for a long term.

The present invention also provides use of a pharmaceutical composition in the preparation of a medicament for modulating neuropathy, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for inhibiting neuroinflammation, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for repairing nerve injury, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving cerebrovascular diseases, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving craniocerebral injury, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving neurodegenerative diseases, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving glaucoma, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving ischemic optic neuropathy, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving optic neuritis, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving optic nerve tumors, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving traumatic optic neuropathy, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving retinopathy, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for inhibiting or mitigating retinal ganglion cell (RGC) injury or severe axonal injury in a subject, wherein the use includes administering to the subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving peripheral neuropathy, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The present invention also provides use of a pharmaceutical composition in the preparation of a medicament for treating, preventing, or improving diabetic peripheral neuropathy, wherein the use includes administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

### Brief Description of Drawings

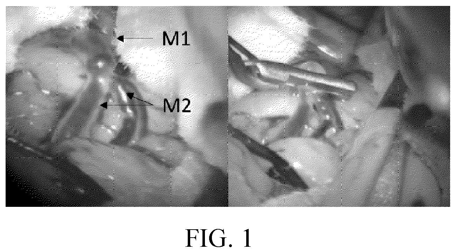

FIG. 1 shows occlusion locations (distal end of M1 segment of the middle cerebral artery) in a middle cerebral artery occlusion (MCAO) model in a rhesus monkey. Left figure of FIG. 1: locations where the middle cerebral artery is clipped by aneurysm clips are the distal end of M1 segment and the proximal end of M2 segment in the middle cerebral artery. Right figure of FIG. 1: the middle cerebral artery is clipped by an aneurysm clip.

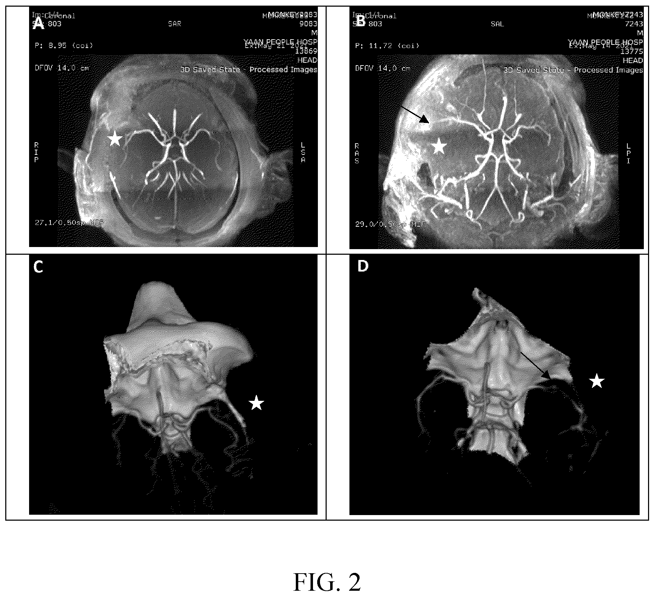

FIG. 2 shows angiography 3-4 hours after MCAO reperfusion in a rhesus monkey. A: MRA image showing that MCAO reperfusion succeeded; B: MRA image showing that MCAO was not reperfused; C: CTA image showing that MCAO reperfusion succeeded; D: CTA image showing that MCAO was not reperfused. The black arrows indicate locations of the middle cerebral artery occlusion, and the white asterisks indicate the surgical side of MCAO.

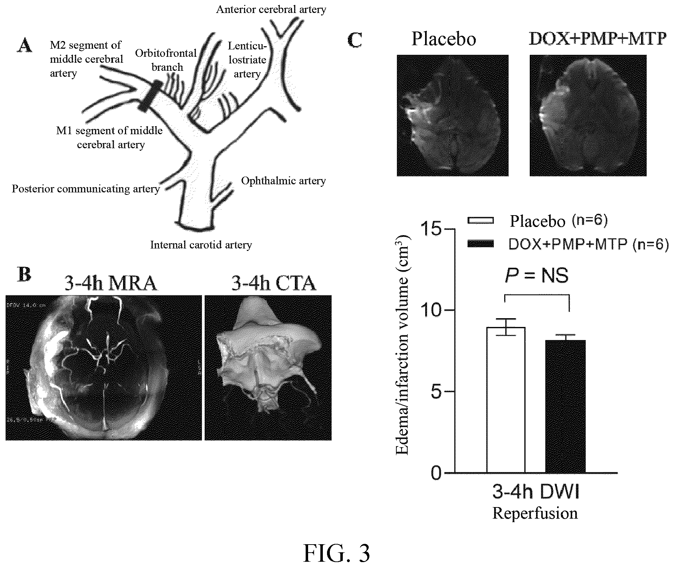

FIG. 3 shows a method of middle cerebral artery occlusion (MCAO) in a rhesus monkey. Craniotomy was performed in a frontotemporal area to open the lateral fissure, expose the middle cerebral artery (MCA) from the origin of the bifurcation of the internal carotid artery, and show the orbitofrontal branch and the M1 and M2 segments of MCA at the distal end. A: a schematic diagram of middle cerebral artery occlusion, located at the distal end of the M1 segment and the proximal end of the M2 segment in the middle cerebral artery. B: magnetic resonance angiography (MRA) and computed tomography angiography (CTA) 3-4 hours after reperfusion after 90-minute middle cerebral artery occlusion showing the completion of revascularization in the distal middle cerebral artery. C: magnetic resonance diffusion weighted imaging (DWI) 3-4 hours (i.e., before drug administration) after reperfusion after 90-minute middle cerebral artery occlusion showing infarction/edema areas with high signals, wherein the infarction/edema volumes in the two groups were similar. NS means "the difference is not statistically significant".

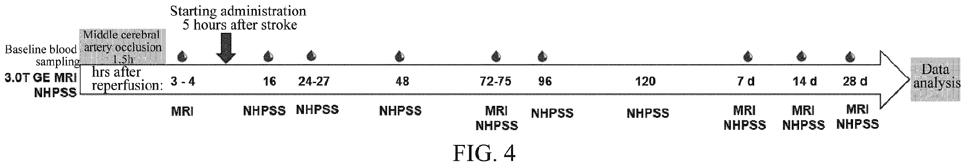

FIG. 4 shows a schedule of the test process, drug administration, and detection of various indexes of the 90-minute middle cerebral artery occlusion (MCAO) reperfusion model in a rhesus monkey.

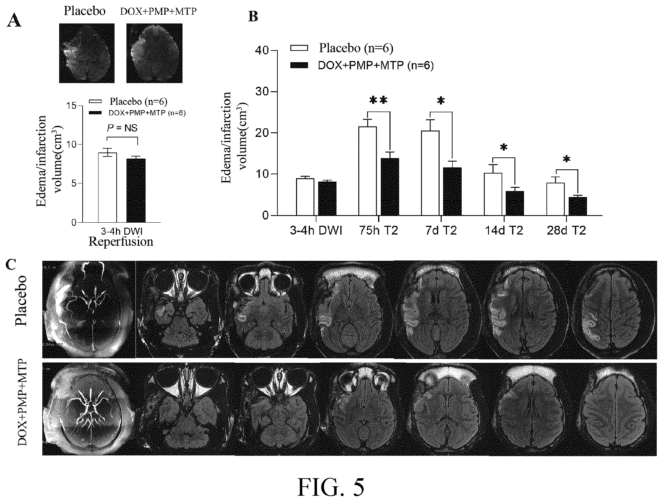

FIG. 5 shows that DOX+PMP+MTP significantly reduced the infarction/edema volume of MCAO reperfusion brain in rhesus monkey, when administered orally starting from 5 hours after stroke after 90-minute middle cerebral artery occlusion in a rhesus monkey for 28 consecutive days. A: magnetic resonance diffusion weighted imaging (DWI) 3-4 hours after reperfusion after 90-minute middle cerebral artery occlusion showing infarction areas, the mean volumes of DWI-quantified cerebral edema/infarction areas in the two groups were 8.98±1.14 cm³ and 8.17±0.85 cm³, respectively, and no statistical difference was seen between the two groups in terms of edema/infarction volume prior to drug administration. NS means "the difference is not statistically significant". B: dynamic changes in cerebral edema/infarction volume measured by magnetic resonance DWI and T2 FLAIR at each time point within 28 days after stroke. DOX+PMP+MTP treatment of MCAO rhesus monkeys showed a significant reduction in the edema/infarction volume of the brain tissue on the surgical side (right side) at 75h, Day 7, Day 14, and Day 28. (Compared with a placebo group, *p<0.05, **p<0.01) C: representative MRA images at 3-4 h and representative T2 FLAIR images at Day 28 after reperfusion for placebo- and DOX+PMP+MTP-treated 90-minute middle cerebral artery occlusion. T2 FLAIR images are images of different levels of a single animal at Day 28, showing lesions in areas of temporal lobe, frontal lobe, parietal lobe and occipital lobe. It can be seen that middle cerebral artery reperfusion was formed in both groups of animals after stroke, and the cerebral edema/infarction area was significantly smaller in DOX+PMP+MTP-treated rhesus monkeys than in placebo-treated rhesus monkeys 28 days after stroke.

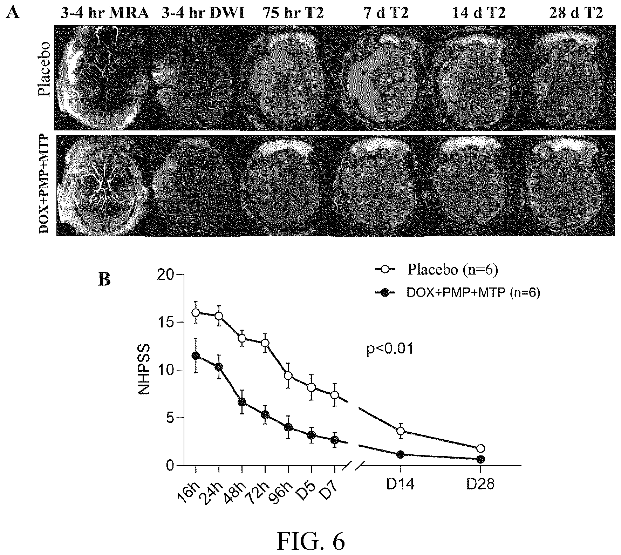

FIG. 6 shows that DOX+PMP+MTP significantly reduced the infarction/edema volume and significantly improved neurobehavioral function, when administered orally starting from 5 hours after stroke after 90-minute middle cerebral artery occlusion in rhesus monkeys for 28 consecutive days. A: representative MRA and DWI images 3-4 hours after reperfusion of 90-minute middle cerebral artery occlusion and T2 FLAIR images at different time points within 28 days of treatment. It can be seen that middle cerebral artery reperfusion was formed in both groups of animals after stroke, and the edema/infarction volumes before drug administration in both groups were similar. The cerebral edema/infarction area was significantly smaller in DOX+PMP+MTP-treated rhesus monkeys than in placebo-treated rhesus monkeys at different times within 28 days of drug administration. B: NHPSS observation results within 28 days of drug administration. DOX+PMP+MTP could significantly reduce the neurobehavioral score of MCAO rhesus monkeys and significantly improve the neurologic deficit of stroke animals (p<0.01).

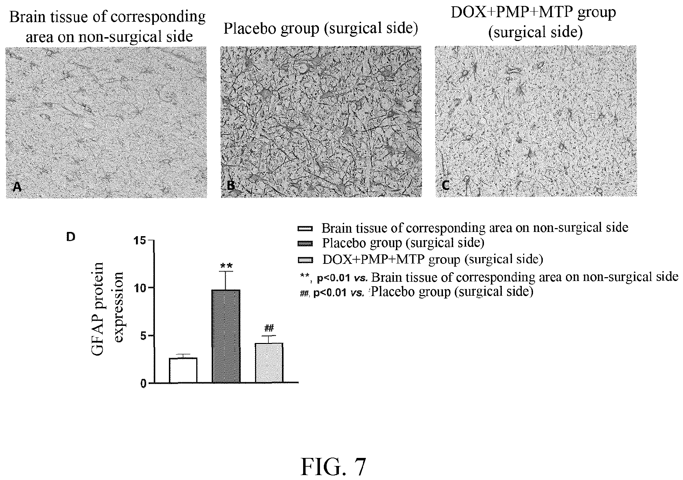

FIG. 7 shows a quantitative analysis of GFAP protein expression in brain tissue in the area around cerebral necrosis at Day 30 after stroke in a rhesus monkey 90-minute middle cerebral artery ischemia reperfusion model. A) Representative image of immunohistochemical staining of GFAP protein in brain tissue of corresponding areas of the temporal lobe and precentral gyrus on the non-surgical side; B) Placebo group; and C): Representative images (×400) of immunohistochemical staining of GFAP protein in brain tissue of the peri-necrosis area in the temporal lobe and precentral gyrus on the surgical side in the DOX+PMP+MTP group; D) GFAP protein expression of brain tissue in the corresponding area on the non-surgical side and in brain tissue in the peri-necrosis area in the temporal lobe and precentral gyrus on the surgical side in the placebo group and the DOX+PMP+MTP group 30 days after stroke. Data was presented as Mean±SD; **, p<0.01 vs. brain tissue in corresponding area on the non-surgical side; ##, p<0.01 vs. placebo group.

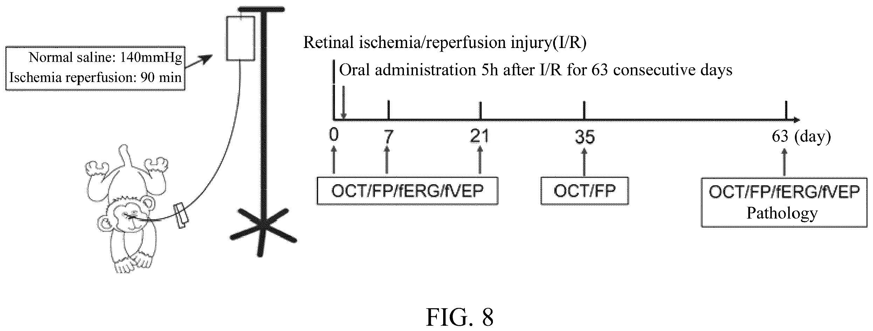

FIG. 8 shows a schematic diagram of preparation of rhesus monkeys with acute retinal ischemia/reperfusion injury (I/R) and a schedule of drug administration and detection of various indexes.

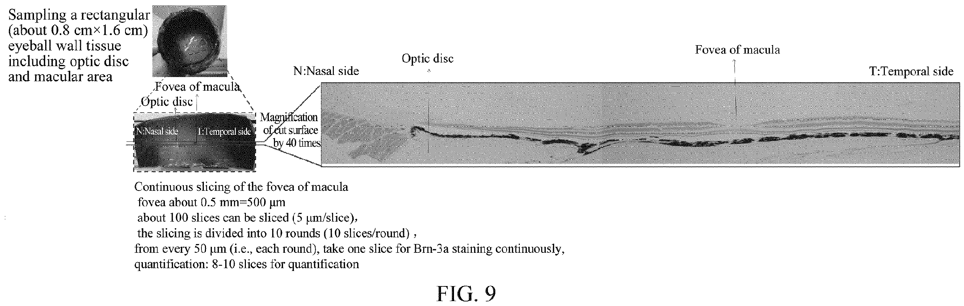

FIG. 9 is a schematic diagram of rhesus monkey retinal tissue sampling, slicing and retinal ganglion cell (RGC) counting.

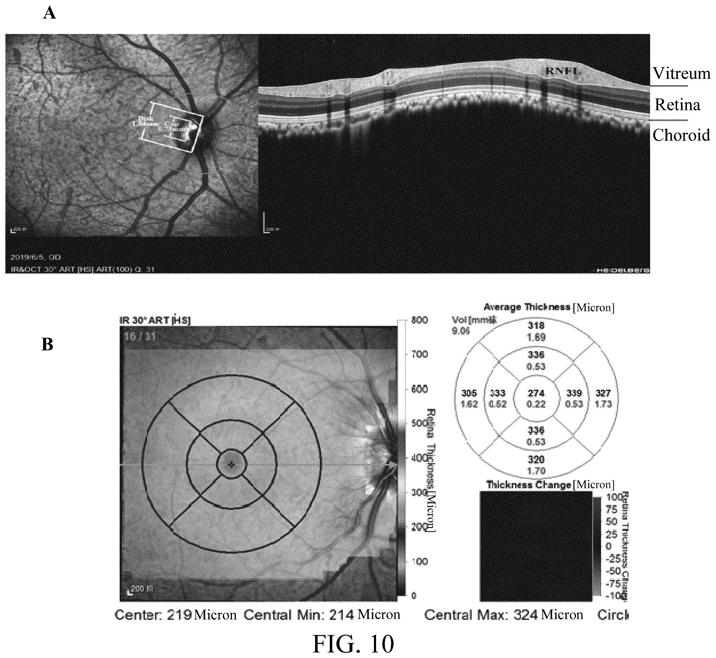

FIG. 10 shows spectral domain-optical coherence tomography (SD-OCT) images of rhesus monkey optic nerve head and retinal layer. (A) Representative SD-OCT image of peri-papilla of a normal monkey, concentric SD-OCT scan shows the retinal cell layer of a normal eye. (B) Schematic diagram of thickness of the 9-part retina EDTRS area of a rhesus monkey measured by optical coherence tomography (OCT).

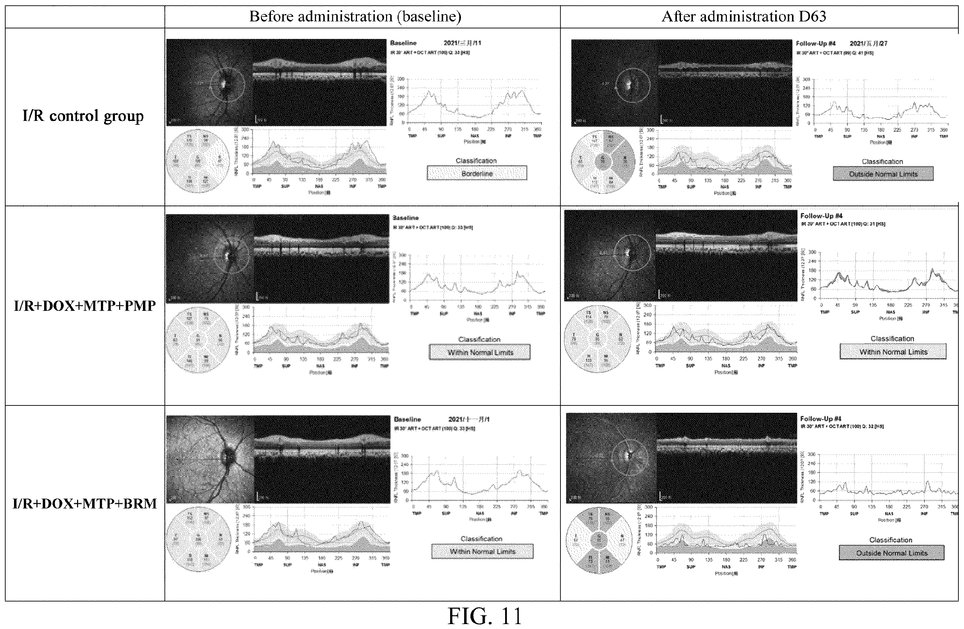

FIG. 11 shows representative images of retinal thickness in the EDTRS area in rhesus monkeys measured by spectral domain-optical coherence tomography (SD-OCT) before drug administration and at D63 after drug administration.

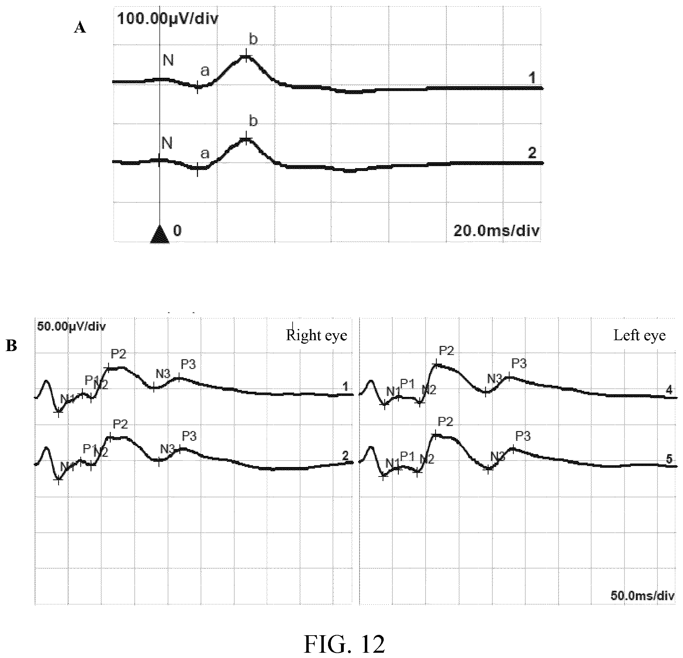

FIG. 12(A) shows a light-adapted 3.0 ERG waveform of a rhesus monkey without ischemic injury, wherein 1 is OD right eye and 2 is OS left eye; (B) shows a flash VEP waveform of a rhesus monkey without ischemic injury.

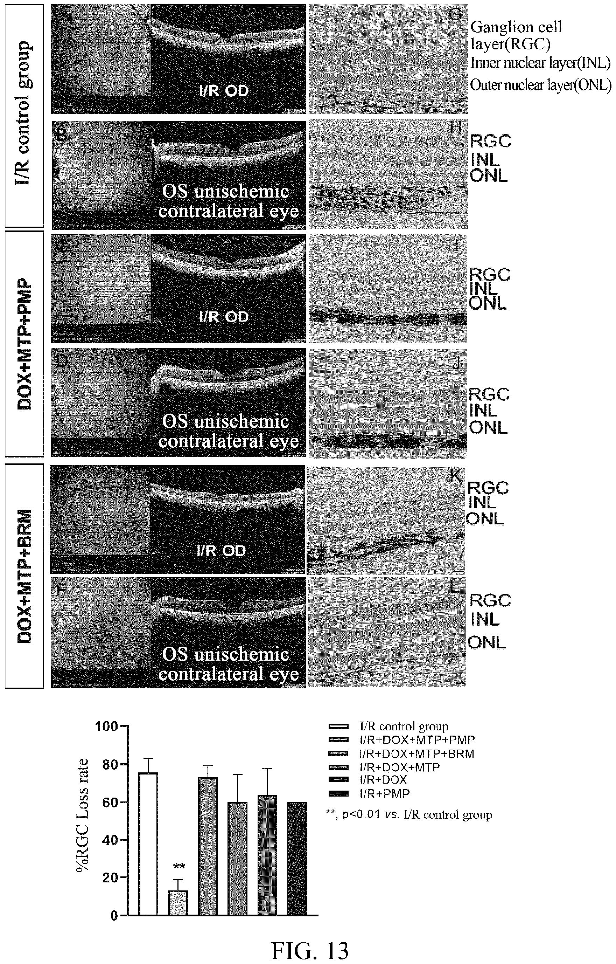

FIG. 13 shows that 63 days of treatment in the DOX+MTP+PMP group significantly improved RGC survival rate after retinal I/R injury in rhesus monkeys; (A-F) OCT images of I/R-injured right eye (OD) and left eye (OS unischemic control eye) of one representative animal from each of the I/R control group, the DOX+MTP+PMP group, and the DOX+MTP+BRM group at D63 after retinal I/R; (G-L) Representative micrographs of Brn3a immunohistochemistry of retinal slices from a same eye, Brn3a: yellow immunolabeled RGC, magnification (×200). (M) RGC survival rate of rhesus monkeys in each group after 63 days of treatment by drug administration (**, p<0.01 compared with I/R eyes in the I/R control group); ##, p<0.01 compared with OS unischemic contralateral eye; wherein I/R control group (N=3), I/R+DOX+MTP+PMP group (N=3), I/R+DOX+MTP+BRM group (N=3), I/R+DOX+MTP group (N=3), I/R+DOX group (N=3) and I/R+PMP group (N=1) (Note: BRM is Bromocriptine).

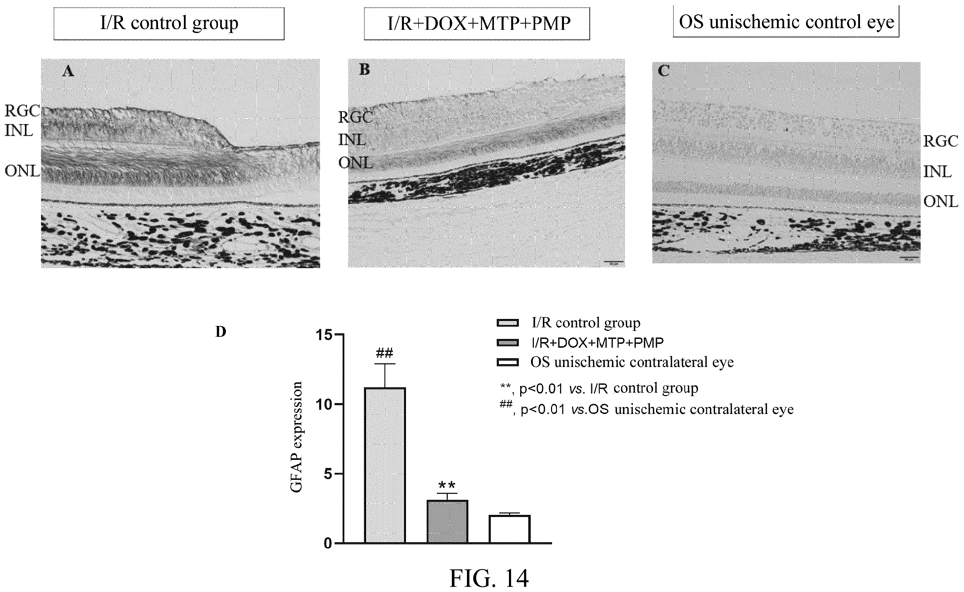

FIG. 14 shows that the expression of GFAP protein in retina of rhesus monkeys treated for 63 days (D63) in the DOX+MTP+PMP group was significantly lower than that in I/R eyes in the I/R control group; (A-C) representative micrographs of GFAP immunohistochemistry of retinal slices from the I/R-injured right eye (OD) of one representative animal from each of the I/R control group and the DOX+MTP+PMP group and from the left eye of one of these animals (unischemic control eye), GFAP: yellow immunolabeled astrocytes, magnification (×200). (D) expression of GFAP protein in rhesus monkeys with retinal ischemia/reperfusion injury at Day 63 (D63) of treatment (**, p<0.01 compared with the I/R control group), wherein n=7 in OS unischemic control eyes.

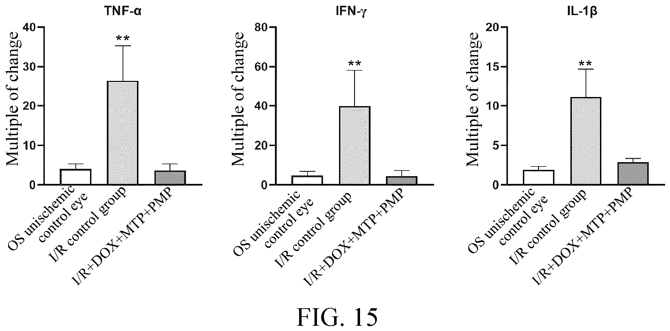

FIG. 15 shows the condition of inflammatory factors (IFN-γ, IL-1β, TNF-α) in each group of rhesus monkeys after drug administration (**, p<0.01 compared with OS unischemic control eyes).

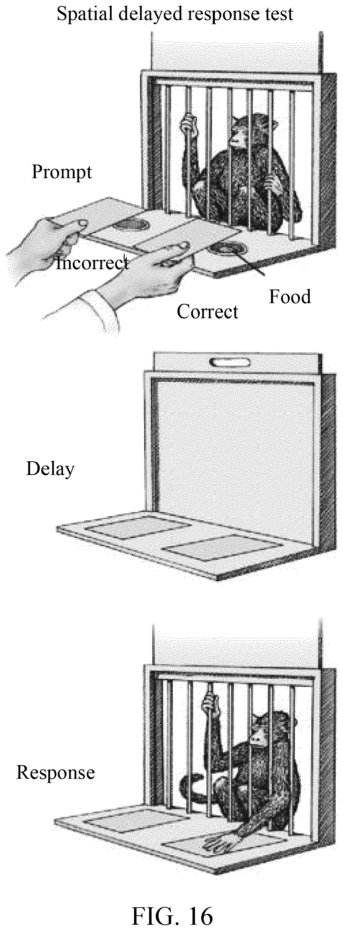

FIG. 16 shows a schematic diagram of a Wisconsin device and experiment.

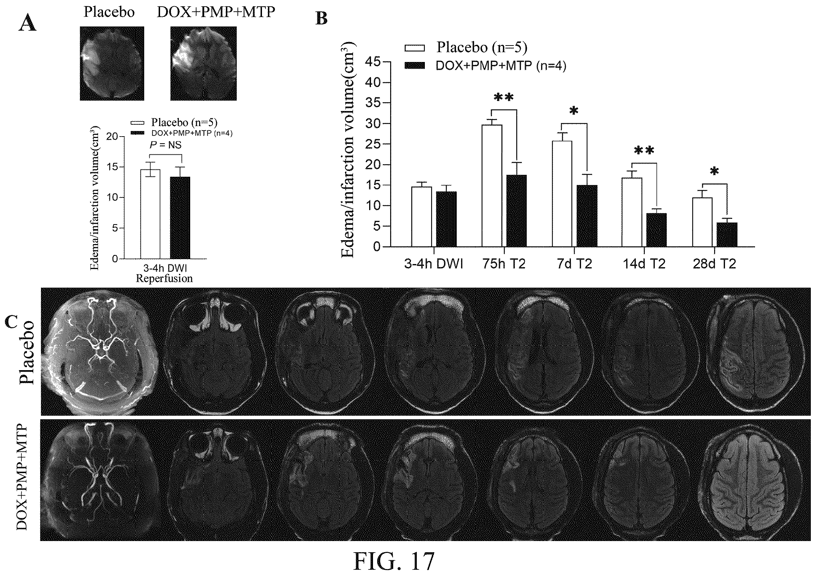

FIG. 17 shows that DOX+PMP+MTP significantly reduced the infarction/edema volume of MCAO reperfusion rhesus monkey brain, when administered orally about 7 hours after stroke in a rhesus monkey 180-minute middle cerebral artery occlusion model for 60 consecutive days. A: magnetic resonance diffusion weighted imaging (DWI) 3-4 hours after reperfusion of 180-minute middle cerebral artery occlusion (i.e. before administration) showed abnormal high signals in the right frontal lobe, parietal lobe and temporal lobe, which was similar to the case of ischemic infarct of middle cerebral artery in clinical patients. The mean volumes of DWI-quantified edema/infarction area of 5 rhesus monkeys in the placebo group and 4 rhesus monkeys in the DOX+PMP+MTP group were 14.61±2.63 cm³ and 13.43±3.14 cm³, respectively, and no statistical difference was seen between the two groups in terms of edema/necrosis volume prior to drug administration. NS means "the difference is not statistically significant". B: compared with the placebo group, edema/necrosis volume in brain issue on the surgical side (right side) in the DOX+PMP+MTP group decreased significantly at 75h, Day 7, Day 14 and Day 28 after reperfusion (p<0.05 or p<0.01). C: representative MRA images at 3-4 h and representative T2 FLAIR images at Day 28 after reperfusion after 180-minute middle cerebral artery occlusion in the placebo group and the DOX+PMP+MTP group. T2 FLAIR images were images of different levels of a single animal at Day 28, showing lesions in areas of temporal lobe, frontal lobe, parietal lobe, and occipital lobe. It could be seen that middle cerebral artery reperfusion was formed in both groups of animals after stroke, and the cerebral edema/infarction area was significantly smaller in DOX+PMP+MTP-treated rhesus monkeys than in placebo-treated rhesus monkeys 28 days after stroke.

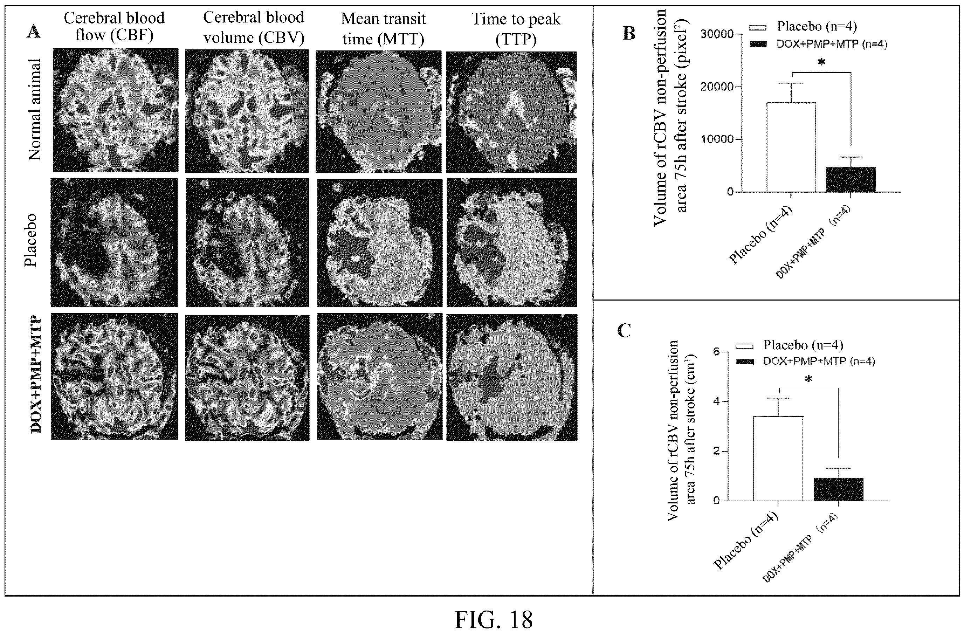

FIG. 18 shows that DOX+PMP+MTP significantly reduced the volume of cerebral tissue perfusion deficit area and non-perfusion area, when administered orally about 7 hours after stroke in a rhesus monkey 180-minute middle cerebral artery occlusion reperfusion model for 60 consecutive days. A: DSC showed no perfusion deficit in normal animals. 75 hours after reperfusion, rhesus monkeys in the placebo group showed significant non-perfusion area, decreased CBF and CBV, and increased MTT and TTP. The perfusion deficit area in the DOX+PMP+MTP group was significantly smaller than that in the placebo group. B: volume analysis of non-perfusion area measured by DSC at 75 h after 180-minute middle cerebral artery occlusion reperfusion. Compared with the placebo group, DOX+PMP+MTP could significantly reduce the volume of non-perfusion area in MCAO rhesus monkeys (p<0.05). The unit of the volume of the non-perfusion area in the figure was expressed as a pixel value (pixel²), which is converted to a volume (cm³) by the formula: volume of rCBV non-perfusion area (cm³)=pixel value (pixel²)÷1000×0.2. The volume of the non-perfusion area (ischemic core area) measured in the CBV image was 17031±6355 pixel² (converted into 3.41±1.27 cm³), and the volume of the non-perfusion area measured in the CBV image of the surgical side (right side) was 4747±3210 pixel² (converted into 0.95±0.64 cm³). C: volume analysis of non-perfusion area measured by DSC at 75 h after 180-minute middle cerebral artery occlusion reperfusion. Compared with the placebo group, DOX+PMP+MTP could significantly reduce the volume of non-perfusion area in MCAO rhesus monkeys (p<0.05). The volume unit of the non-perfusion area in the figure was expressed as cm³.

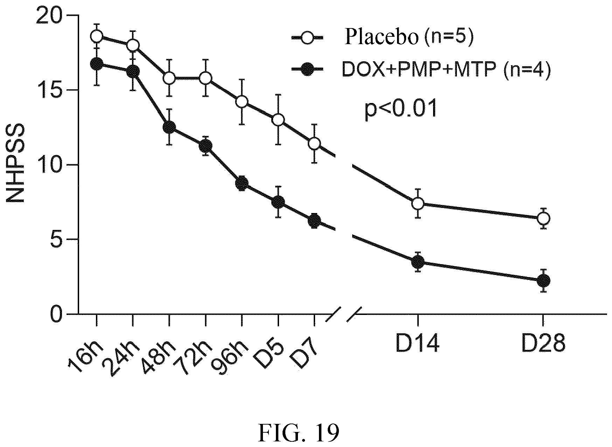

FIG. 19 shows the results of NHPSS observation within 28 days of oral administration of DOX+PMP+MTP in consecutive treatment of 60 days starting at about 7 h after stroke in a rhesus monkey 180-minute middle cerebral artery occlusion reperfusion model. Compared with the placebo group, DOX+PMP+MTP could significantly reduce the neurobehavioral score of MCAO rhesus monkeys and significantly improve the neurologic deficit of stroke animals (p<0.01).

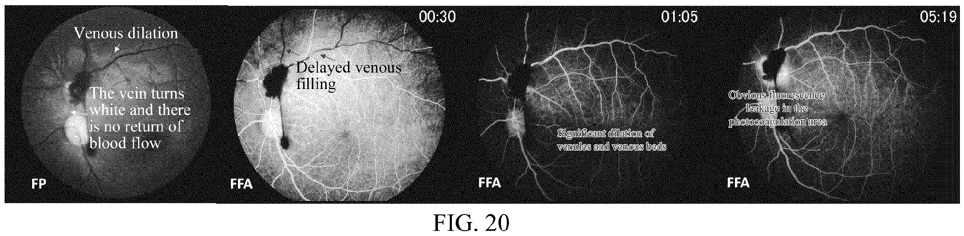

FIG. 20 shows representative FP and FFA images of RVO rhesus monkeys on the day of modeling. FP and FFA images showed successful creation of the RVO rhesus monkey model by laser photocoagulation in the superior branch vein around the optic disc in the left eye. FP showed obvious venous dilatation and tortuosity in the retina, with whitening of the veins and no blood reflux. FFA showed delayed filling and fluorescence leakage in the vicinity of the area of vein occlusion.

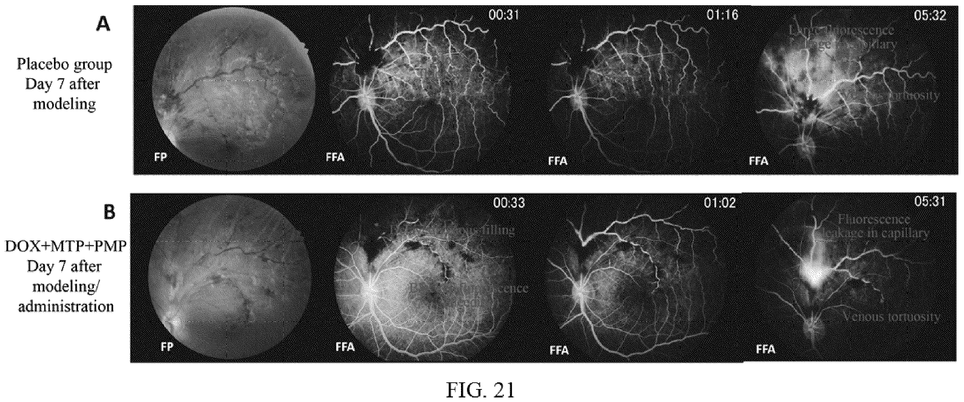

FIG. 21 shows representative FP and FFA images of RVO rhesus monkeys in the placebo group and the DOX+MTP+PMP group at Day 7 of drug administration. A) FP and FFA images of the placebo group showed delayed filling of superior retinal branch veins, flame hemorrhage, venous dilatation, tortuosity and large-area capillary fluorescence leakage around the occlusion site; B) delayed filling of the superior retinal branch veins, flame hemorrhage (slightly milder than in the placebo group), vein dilatation, tortuosity, and capillary fluorescence leakage around the occlusion site were observed in DOX+MTP+PMP group at Day 7 of treatment, which were significantly milder than in the placebo group.

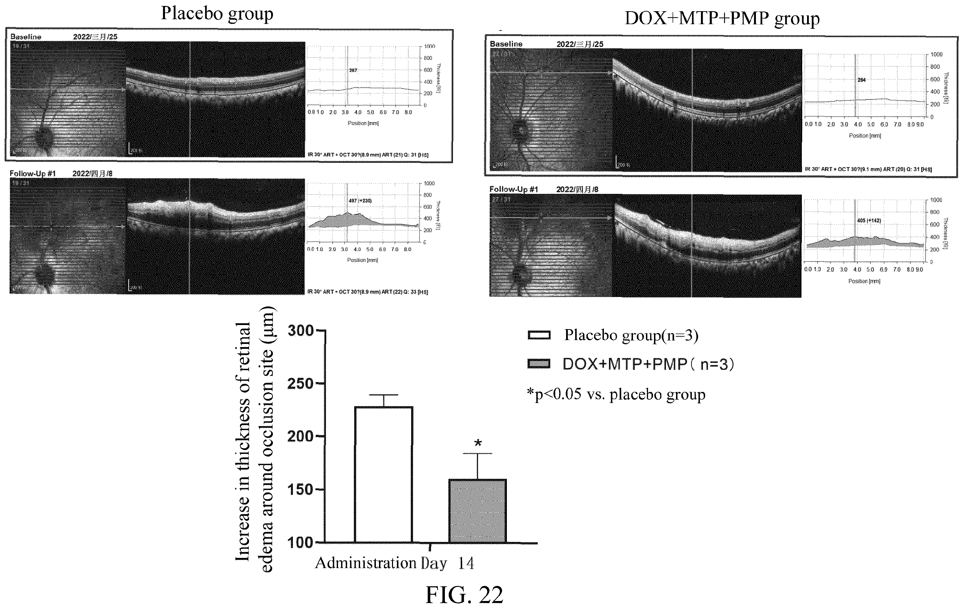

FIG. 22 shows the changes in retinal thickness around the occlusion site of superior retinal branch vein in each group. A) Representative images of changes in retinal thickness around the occlusion site in the placebo group at Day 0 and Day 14 of drug administration; B) representative images of changes in retinal thickness around the occlusion site in the DOX+MTP+PMP group at Day 0 and Day 14 of drug administration; and C) changes in retinal edema thickness around the occlusion site at Day 14 in the placebo group and the DOX+MTP+PMP group. Data was presented as Mean±SD, *p<0.05 vs. placebo group.

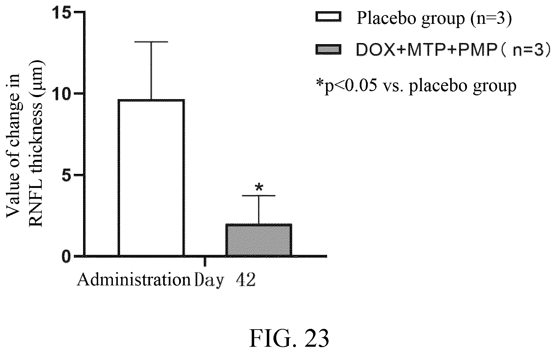

FIG. 23 shows the change in RNFL thickness of RVO rhesus monkeys in the placebo group and the DOX+MTP+PMP group at Day 42 of drug administration. Data was presented as Mean±SD, *p<0.05 vs. placebo group.

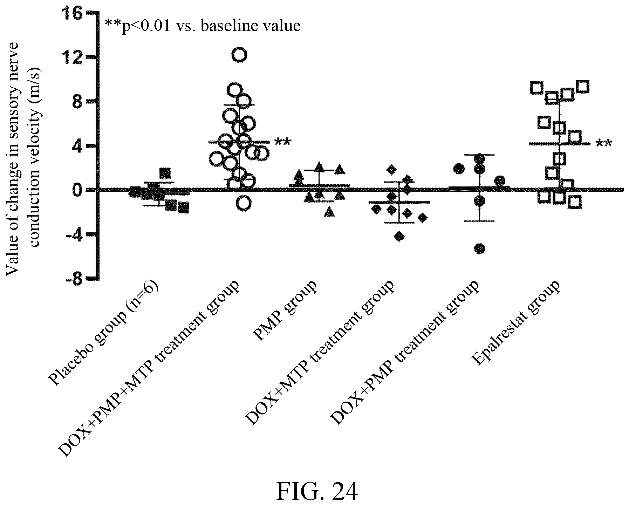

FIG. 24 shows individual data of changes in sensory conduction velocity in rhesus monkeys with diabetic peripheral neuropathy as a result of 58 days of drug administration in each group, including placebo group (n=4, 7 abnormal nerves), DOX+PMP+MTP treatment group (n=4, 17 abnormal nerves), PMP treatment group (n=3, 8 abnormal nerves), DOX+MTP treatment group (n=3, 9 abnormal nerves), DOX+PMP treatment group (n=2, 6 abnormal nerves) and epalrestat group (n=4, 13 abnormal nerves).

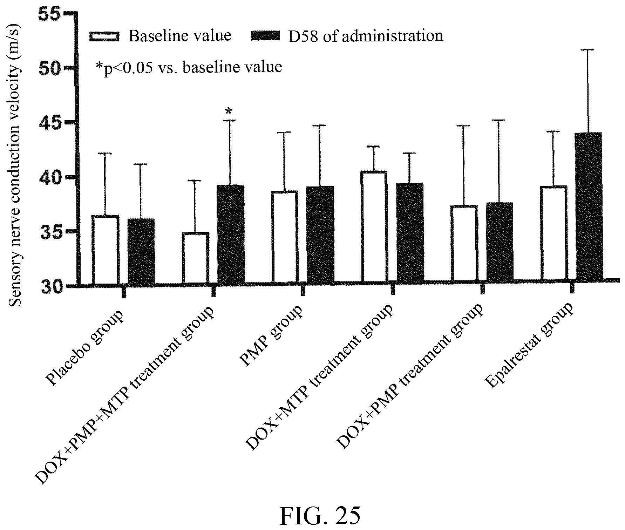

FIG. 25 shows the effect of 58 days of drug administration in each group on sensory nerve conduction velocity in rhesus monkeys with diabetic peripheral neuropathy, including placebo group (n=4, 7 abnormal nerves), DOX+PMP+MTP treatment group (n=4, 17 abnormal nerves), PMP treatment group (n=3, 8 abnormal nerves), DOX+MTP treatment group (n=3, 9 abnormal nerves), DOX+PMP treatment group (n=2, 6 abnormal nerves) and epalrestat group (n=4, 13 abnormal nerves).

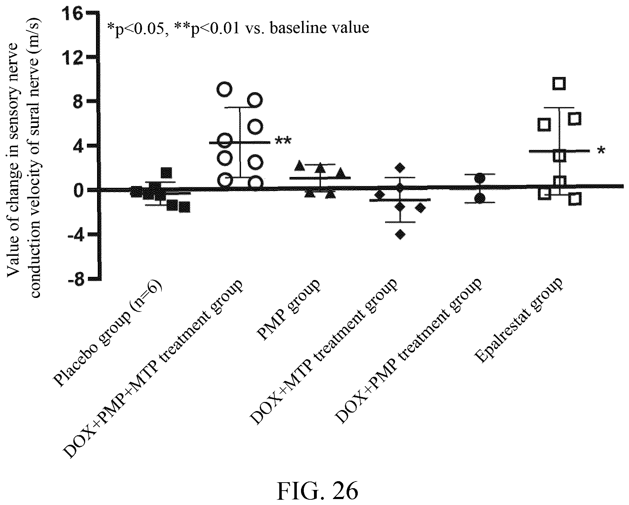

FIG. 26 shows individual data of changes in sural sensory nerve conduction velocity in rhesus monkeys with diabetic peripheral neuropathy as a result of 58 days of drug administration in each group, including placebo group (n=4, 7 abnormal nerves), DOX+PMP+MTP treatment group (n=4, 8 abnormal nerves), PMP treatment group (n=3, 5 abnormal nerves), DOX+MTP treatment group (n=3, 6 abnormal nerves), DOX+PMP treatment group (n=2, 2 abnormal nerves) and epalrestat group (n=4, 7 abnormal nerves).

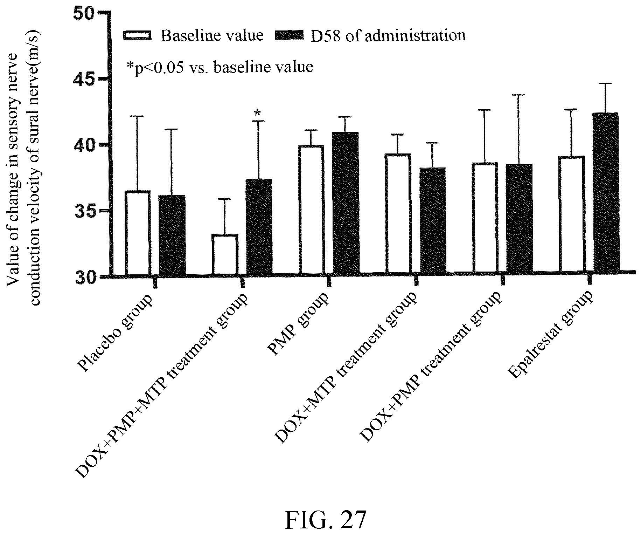

FIG. 27 shows the effect of 58 days of drug administration in each group on sural sensory nerve conduction velocity in rhesus monkeys with diabetic peripheral neuropathy, including placebo group (n=4, 7 abnormal nerves), DOX+PMP+MTP treatment group (n=4, 8 abnormal nerves), PMP treatment group (n=3, 5 abnormal nerves), DOX+MTP treatment group (n=3, 6 abnormal nerves), DOX+PMP treatment group (n=2, 2 abnormal nerves) and epalrestat group (n=4, 7 abnormal nerves).

### Detailed Description

### Definitions

Some terms are used in the description, examples and claims of the present application. All technical and scientific terms used herein have the same meanings as understood by those skilled in the art to which the present application pertains, unless otherwise defined.

As used herein, the term "subject" refers to animals, including, but not limited to, mammals. Mammals include primates or non-primates, such as monkeys, pigs, sheep, dogs, cats, cattle, horses, mice, rats, rabbits, or transgenic species thereof. In certain embodiments, the subject is human.

As used herein, the term "preventing" includes, but is not limited to, delaying and/or impeding a disease, disorder, or condition and/or its accompanying symptoms from occurring; impeding a subject from developing a disease, disorder, or condition; or reducing the subject's risk of developing a disease, disorder, or condition.

As used herein, the term "treating" includes, but is not limited to, relieving or eliminating a disease, disorder, or condition, or one or more symptoms associated therewith; or relieving or eradicating a cause of a disease, disorder, or condition.

As used herein, the term "mitigating", "relieving", or "improving" includes, but is not limited to, relieving one or more symptoms (e.g., cerebral edema) of a disease, disorder, or condition. These terms may also refer to reduction of side effects associated with active ingredients. Sometimes, beneficial effects obtained by a subject from a prophylactic or therapeutic agent will not lead to cure of the symptom, disease, or condition.

As used herein, the term "modulating" refers to prevention, treatment, and/or improvement of a disease, disorder or condition, including, but not limited to, (a) stopping or delaying a disease, disorder, or condition from occurring, or reducing a risk of occurrence of a disease, disorder, or condition, and (b) stopping exacerbation of a disease, disorder, or condition, delaying or mitigating development of a disease, disorder, or condition; and (c) reducing severity of a disease, disorder, or condition, causing regression of a disease, disorder, or condition.

As used herein, the term "administration" refers to an act of delivering, or causing delivery of, a compound or pharmaceutical component/composition to the body of a subject by a method described herein or known in the art. Administering a compound or pharmaceutical component/composition includes prescribing the compound or pharmaceutical component/composition to be delivered to a subject's body. Exemplary forms of administration include oral dosage forms, such as tablets, capsules, syrups, suspensions; injectable dosage forms, such as intravenous (IV) injection, intramuscular (IM) injection, subcutaneous (SC) injection, or intraperitoneal (IP) injection; transepidermal dosage forms, including creams, jellies, powders, or patches; dosage forms of oral cavity; inhalation powders, sprays, suspensions, and rectal suppositories.

As used herein, the term "therapeutically effective amount" is a term recognized in the art. In certain embodiments, the term refers to an amount necessary or sufficient to eliminate, reduce, or maintain a target of a particular therapeutic regimen. The effective amount may vary depending on factors such as the disease or condition being treated, the particular targeted constructs being administered, the size of the subject, or the severity of the disease or condition. One of ordinary skill in the art or a physician may empirically determine the effective amount of a particular compound without necessitating undue experimentation. In certain embodiments, a therapeutically effective amount of a therapeutic agent for in vivo use will likely depend on a number of factors, including: the mode and method of administration; and any other materials incorporated in a drug in addition to the agent. In vitro or in vivo tests may be used to help determine the optimal dosage range.

As used herein, the term "about" or "approximately" refers to an acceptable error of a particular value determined by one of ordinary skill in the art, depending in part on how the value is measured or determined. In certain embodiments, the term "about" or "approximately" means being within 1, 2, 3, or 4 standard deviations. In certain embodiments, the term "about" or "approximately" means 1%, 0.5%, or 0.05% of a given value or range within 50%, 20%, 15%, 10%, 9%, 8%, 7%, 6%, 5%, 4%, 3%, 2%.

When a series of values is listed here, it is intended to cover every value and sub-range within the range. For example, "1-5 mg" or "about 1 mg to about 5 mg" is intended to cover 1 mg, 2 mg, 3 mg, 4 mg, 5 mg, 1-2 mg, 1-3 mg, 1-4 mg, 1-5 mg, 2-3 mg, 2-4 mg, 2-5 mg, 3-4 mg, 3-5 mg and 4-5 mg.

The terms "including", "containing", "having" are used in an inclusive, open sense, meaning that additional elements may be included in addition to those indicated. The terms "such as", "e.g.", as used herein are non-limiting and are for illustrative purposes only. "Including" and "including but not limited to" are used interchangeably.

In this context, the term "and/or" as used in phrases such as "A and/or B" is intended to encompass both A and B; A or B; A (alone); and B (alone). Likewise, the term "and/or" as used in phrases such as "A, B, and/or C" is intended to encompass each of the following embodiments: A, B, and C; A, B or C; A or C; A or B; B or C; A and C; A and B; B and C; A (alone); B (alone); and C (alone).

As used herein, the phrase "at least one" for a list of one or more elements should be construed as meaning at least one element selected from any one or more elements in the list of elements, but does not necessarily include at least one of each element specifically listed in the list of elements, and does not exclude any combination of elements in the list of elements. The definition also allows elements to optionally exist in addition to specifically identified elements in the list of elements referred to by the phrase "at least one", whether related or not to those specifically identified elements. Thus, as a non-limiting example, in one embodiment, "at least one of A and B" (or equivalently, "at least one of A or B", or equivalently, "at least one of A and/or B") may refer to at least one, optionally including more than one, refers to A and B does not exist (and optionally includes elements other than B); in another embodiment, refers to at least one, optionally including more than one, referring to B and A does not exist (and optionally including elements other than A); in yet another embodiment, refers to at least one, optionally including more than one, refers to A, and at least one, optionally including more than one, refers to B (and optionally including other elements); etc.

### 1. Method for modulating neuropathy

The present invention provides a method for modulating neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

Neuropathy refers to an injury, disease, or dysfunction of one or more nerves, including an injury, disease or dysfunction of central nervous system and peripheral nervous system. It is usually manifested as burning or shooting pain, numbness, tingling, muscle weakness or atrophy, which is usually degenerative and usually caused by injuries, infection, diseases, drugs, toxins, or vitamin deficiencies.

The modulation of neuropathy according to the present invention includes prevention, treatment, and/or improvement thereof. The prevention, treatment and/or improvement of neuropathy includes, but is not limited to, (a) impeding or delaying occurrence of neuropathy, or reducing the risk of occurrence of neuropathy, (b) impeding aggravation of neuropathy, delaying or mitigating development of neuropathy; and (c) reducing severity of neuropathy and causing regression of neuropathy.

In certain embodiments, the method for modulating neuropathy of the present invention relates to inhibiting neuroinflammation. Neuroinflammation generally refers to various inflammatory lesions involving nerves. Neuroinflammation is one of the important pathogenesis of dementia, which leads to pathological processes such as white matter lesions, hippocampus injury and Aβ deposition, and then leads to the development of cognitive impairment-related diseases such as vascular dementia, and Alzheimer's disease.

In certain embodiments, the present invention provides a method for inhibiting neuroinflammation, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. In certain embodiments, the method treats, prevents, or improves a disease, disorder, or condition associated with neuroinflammation. Neuroinflammation-related diseases include: ischemic stroke, hemorrhagic stroke, transient ischemic attack, craniocerebral injury, vascular dementia, Alzheimer's disease, Lewy body dementia, frontotemporal dementia, glaucoma, ischemic optic neuropathy, optic neuritis, optic nerve tumor, traumatic optic neuropathy, retinal artery occlusion, retinal vein occlusion, and retinopathy of prematurity.

The inventors found that the expression levels of inflammatory factors TNF-α, IL-1β, and IFN-γ in retinal tissue of subjects having a retinal I/R attack were at least 5 times, at least 7 times, and even 10 times of the normal levels (i.e., the expression levels of inflammatory factors in retinal tissue of subjects without retinal I/R attack). Without theoretical constraints, cytokines play an important role in the process of neuroinflammation. Pro-inflammatory cytokines such as IFN-γ, IL-1β, TNF-α and, IL-6 participate in the process of neuroinflammation and may be regarded as biomarkers of neuroinflammation.

The inventors also found that expression of GFAP in injured areas of retina and brain tissue of subjects with neuroinflammation resulting from retinal I/R injury or ischemic stroke was significantly increased compared with normal expression (i.e., the expression of GFAP in tissue of subjects without neuroinflammation), and overexpressed GFAP may be determined by routine immunohistochemical staining. The increased expression of astrocyte biomarker glial fibrillary acidic protein (GFAP) is also regarded as one of the markers of neuroinflammation (Li M, Li Z, Yao Y, et al. Astrocyte-derived interleukin-15 exacerbates ischemic brain injury via propagation of cellular immunity. Proc Natl Acad Sci U S A, 2017, 114(3): E396-E405.). GFAP is an astrocyte-specific intermediate filament protein, and its expression is a necessity for the normal function of astrocytes, including maintaining the integrity of CNS white matter and blood-brain barrier. It is worth noting that most astrocytes do not express enough GFAP under normal circumstances and cannot be stained by routine immunohistochemical methods.

In addition, neuroinflammation leads to pathological processes such as white matter lesions, hippocampus injury, and Aβ deposition, and then leads to the development of cognitive impairment-related diseases such as vascular dementia, and Alzheimer's disease (Chen Y H, Lin RR, Tao Q Q. The role of P2X7R in neuroflammation and implications in Alzheimer's disease[J]. Life Sciences, 2021, 271: 119187.).

In certain embodiments, the method for modulating neuropathy of the present invention involves repairing nerve injury. Nerve injury generally refers to injuries of nerve tissue. In certain embodiments, the present invention provides a method for repairing nerve injury, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

In certain embodiments, the method of the present invention treats, prevents, or improves a disease, disorder, or condition associated with neuropathy. Neuropathy-related diseases, disorders or conditions include, but are not limited to, diseases, disorders or conditions caused by neuropathy, and/or diseases, disorders, or conditions with neuropathy as one of the symptoms.

In certain embodiments, the disease, disorder, or condition includes peripheral neuropathy. Peripheral nerve refers to cranial nerves and spinal nerves other than olfactory and optic nerves, autonomic nerves and their ganglia. Peripheral nerve diseases refer to diseases that originate from structural or functional damages to the peripheral nervous system. Peripheral nerves are functionally divided into sensory afferent nerves and motor efferent nerves. In certain embodiments, the method for treating, preventing, or improving peripheral neuropathy improves nerve conduction velocity, particularly significantly improves the sensory nerve conduction velocity of the sural nerve, thereby effectively controlling and alleviating diabetic peripheral neuropathy and neuropathic pain progression.

In certain embodiments, the peripheral neuropathy includes ischemic peripheral neuropathy, peripheral neuropathy caused by immune-related inflammation (Bourque PR, Chardon JW, Massie R. Autoimmune peripheral neuropathies. Clin Chim Acta. 2015 Sep 20; 449:37-42.), and peripheral neuropathy caused by trauma (Menorca RM, Fussell TS, Elfar JC. Peripheral Nerve Trauma: Mechanisms of Injury and Recovery. Hand Clin. 2013;29(3):317-330). Ischemic peripheral neuropathy is a common type of polyneuropathy, and the most common causes thereof are arteriosclerosis and vasculitis (Blaes F. Diagnosis and therapeutic options for peripheral vasculitic neuropathy. Ther Adv Musculoskelet Dis. 2015;7(2):45-55).

In certain embodiments, the peripheral neuropathy is diabetic peripheral neuropathy. Diabetic Peripheral Neuropathy (DPN) is a chronic diabetic microvascular complication with high morbidity and high disability rate. DPN may cause neurological dysfunction and low neurosensitivity in the early stage, such as numbness of limbs, hyperalgesia, foot ulcer, gangrene, and other serious lesions. The amputation rate of patients may exceed 80%, and at least about 20% will develop neuropathic pain, i.e., Diabetic Peripheral Neuropathy Pain (DPNP, also known as diabetic painful peripheral neuropathy). In certain embodiments, the present invention provides a method for treating, preventing, or improving diabetic peripheral neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

In certain embodiments, the disease, disorder or condition includes central nervous system disease, disorder, or condition. In certain embodiments, the central nervous system disease, disorder, or condition includes cerebrovascular disease, craniocerebral injury, neurodegenerative disease, optic neuropathy, and/or retinopathy.

### 1.1 Cerebrovascular disease

In certain embodiments, the central nervous system disease, disorder, or condition is a cerebrovascular disease. In certain embodiments, a method for treating, preventing, or improving cerebrovascular diseases is provided herein, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

Cerebrovascular diseases are a general term for cerebrovascular diseases that result from a variety of causes. Stroke is the main clinical type of cerebrovascular diseases, including ischemic stroke and hemorrhagic stroke, and is a group of cerebrovascular diseases caused by organic brain injury with the common clinical features of sudden onset, rapid occurrence of localized or diffuse brain function deficits. In certain embodiments, the cerebrovascular diseases include stroke and/or transient ischemic attack.

### 1.1.1 Stroke

In certain embodiments, the cerebrovascular disease is stroke. Stroke (also known as apoplexy) is a disease caused by impaired blood flow of the central nervous system, with sudden onset, rapid occurrence of localized or diffuse brain function deficits as common clinical features, and is a group of cerebrovascular diseases caused by organic brain injury. Its potential causes include embolism, hemorrhage, and thrombosis. About 82% of stroke patients are ischemic stroke. The primary therapeutic measures for ischemic stroke are to restore cerebral blood flow in a timely manner, re-establish blood circulation in the ischemic area and save ischemic penumbra. At present, penumbra can be saved by intravenous thrombolysis (IVT) within 3 hours after the onset of ischemic stroke. However, due to the low ratio of vascular reperfusion, the injury caused by reperfusion, and the possible symptoms of bleeding after reperfusion, 70% of stroke patients still have different degrees of labor loss.

Without theoretical constraints, neurons in subjects having a stroke attack are difficult to be free from influence of brain injury in the early stage, and depend on the environmental support of various glial cells and immune cells in the later stage of brain tissue reconstruction and repair. Cerebral ischemia-reperfusion (I/R) injury is accompanied by proliferation and activation of glial cells, neuroinflammation and cytokine cascade reaction, which leads to nerve cell death, and this is considered as an important pathological mechanism of stroke. Astrocytes are important component cells of blood-brain barrier, and after stroke, astrocytes swell with increased uptake of glutamic acid and lactic acid, compress blood vessels in ischemic areas and aggravate the condition of inadequate vascular perfusion. The increased expression of astrocyte biomarker glial fibrillary acidic protein (GFAP) is regarded as a marker of neuroinflammation, and the expression of various pro-inflammatory cytokines is increased at the same time, aggravating I/R injury. For patients who have already suffered from stroke, it is extremely important to prevent and delay the death of nerve cells, which may preserve more nerve cells, so as to restore their functions and gain more time for treatment.

In certain embodiments, the stroke includes ischemic stroke and/or hemorrhagic stroke. In certain embodiments, the stroke is an ischemic stroke. Ischemic stroke refers to a type of stroke caused by occlusion of blood flow to the brain. The most common potential conditions for the occlusion is development of fat deposits or blood clots in the lining of blood vessels, called atherosclerosis. These blood clots will cause two types of occlusions. One is to form thrombus in cerebral blood vessels and occlude blood vessels. The other refers to the case that thrombus or plaque fragments formed outside the brain enter cerebral blood vessels until they occlude blood vessels that are too small to pass through. The second important cause of occlusion is arrhythmia, called atrial fibrillation. Atrial fibrillation causes blood clots to form in the heart and move into the brain. Other potential causes of ischemic stroke include cerebral hemorrhage, thrombosis, arterial or venous dissection, cardiac arrest, and iatrogenic causes, such as surgical injury to cerebral vessels or surgery on the brain or heart. Ischemic stroke accounts for about 83% of all stroke cases.

In certain embodiments, the subject having an ischemic stroke attack is a subject within 7 hours after the stroke attack. It should be noted that the current clinical treatments for stroke have a very narrow administration window, with considerable limitations on administration time and conditions. For example, it requires intravenous thrombolytic (IVT) drugs to be administered within 4.5 hours after the onset of acute ischemic stroke symptoms, after proper imaging to rule out intracranial hemorrhage in advance, which brings great inconvenience to clinical use of medication. However, the inventors found that the method for treating, preventing, and/or improving ischemic stroke still produces beneficial effects when applied to subjects within 7 hours after the onset of stroke. In certain embodiments, the subject having an ischemic stroke attack is a subject within 5 hours after the stroke attack.

In certain embodiments, the subjects with ischemic stroke attack have a National Institute of Health Stroke Scale (NIHSS) of 20 or within 20, or 15 or within 15, or between 8 and 20, or between 15 and 20, or between 8 and 15, or 8. In certain embodiments, the subjects are subjects with moderate to severe stroke accompanied by persistent neurologic deficits. The National Institute of Health Stroke Scale (NIHSS) is recommended to evaluate the degree of cerebral infarction. NIHSS baseline evaluation may reflect the severity of stroke at onset, and may also evaluate the effect after treatment regularly. NIHSS ranges from 0 to 42. The higher the score, the more severe the stroke (Adams HP Jr, Davis PH, Leira EC, et al. Baseline NIH Stroke Scale score strongly predicts outcome after stroke: A report of the Trial of Org 10172 in Acute Stroke Treatment (TOAST). Neurology. 1999 Jul;53(1): 126-131.).

For example: 0-1 means being normal or almost normal; 1-4 means mild stroke/minor stroke; 5-15 means moderate stroke; 15-20 means moderate-severe stroke; 20-42 means severe stroke. For every 1 score increase in NIHSS baseline, the probability of good prognosis decreases by 17%. Patients with baseline scores greater than 16 are likely to die. Patients with endovascular therapy (EVT) usually have NIHSS scores ≥15-20, and they have neurologic abnormalities in addition to hemiparesis and are considered to have moderate or severe stroke (Brott TG, Adams HP Jr, Olinger CP, et al. Measurements of acute cerebral infarction: a clinical examination scale. Stroke 1989; 20: 864 - 70.).

In certain embodiments, the subject is a subject with anterior circulation large vessel occlusion. In certain embodiments, the subjects are subjects with acute ischemic stroke caused by anterior circulation large vessel occlusion. In certain embodiments, the subject's NIHSS score is between 8 and 20 (including 8 and 20). In certain embodiments, the subject is accompanied by moderate to severe neurological impairment. Anterior circulation large vessel occlusion refers to middle cerebral artery occlusion or internal carotid artery occlusion, most of which is middle cerebral main artery occlusion, and a few are large cerebral infarction caused by siphon segment occlusion of internal carotid artery. Applying this method to subjects for 75 hours of continuous drug administration can reduce the volume of cerebral edema/infarction, reduce the volume of ischemic core area and significantly improve the ischemic condition, with good neurological prognosis (e.g., reduction in NIHSS scores to 2 or 3) in subjects after 1 or 2 months, and can ameliorate neurological deficits in subjects. Compared with the NIHSS scores of subjects who did not implement this method (for example, the score of 1 month after stroke is about 6), the NIHSS score of subjects who implemented this method could be reduced by about 50% or even about 70%.

In certain embodiments, the subjects are subjects with anterior circulation large vessel occlusion, particularly subjects with symptoms of occlusion in the M1 or M2 segment of the middle cerebral artery. In certain embodiments, the subject has a symptom of occlusion in the M1 segment of the middle cerebral artery. It should be noted that the middle cerebral artery is a site where human ischemic stroke usually occurs. When the M1 segment is occluded, an edema/infarction volume of about 40% of the hemisphere (reperfusion after 1.5 hour occlusion in the M1 segment of the middle cerebral artery) and/or an edema/infarction volume of about 70% of the hemisphere volume (reperfusion after 3 hour occlusion in the M1 segment of the middle cerebral artery) are presented at the peak of edema, with a significant midline shift. The area of infarction 28 days after stroke included cognitive- and memory-related brain areas, such as the temporal lobe and hippocampus, which may lead to long-term neurologic impairment disability and/or long-term cognitive decline in the affected limb. The inventors found that the method for treating, preventing, and/or improving ischemic stroke still produces beneficial effects when used in subjects with this type of severely disabling cerebral edema/infarction, for example, when treatment is given orally about 7 hours after stroke onset.

In certain embodiments, the subject having an ischemic stroke attack is a subject with ischemia-reperfusion injury, wherein reperfusion includes reperfusion by intravascular therapy and/or reperfusion by administration of a thrombolytic agent. Endovascular therapy includes: arterial thrombolysis, mechanical thrombectomy, and emergency angioplasty (Chinese Stroke Association, Chinese Guidelines for Endovascular Treatment of Acute Ischemic Stroke 2018[J]. Chinese Journal of Stroke, 2018, 013(007): 706-729).

In certain embodiments, the method of the present invention may be used in subjects (e.g. within 3 hours after onset) in combination with a method of administration of intravenous rt-PA thrombolysis for acute stroke, which may better improve the neurological prognosis of the subject.

In certain embodiments, where the subject misses the time window for intravenous rt-PA thrombolysis after an acute stroke onset, the method of the present invention may be used, and the method of the present invention may extend the treatment time window, thereby giving the subject who visits beyond the time window for intravenous thrombolysis an opportunity for endovascular reperfusion therapy.

In certain embodiments, subjects misses the time window for intravenous rt-PA thrombolysis after the onset of acute stroke and adopts an endovascular therapy (e.g., performing thrombectomy and reperfusion to a subject with large vessel (carotid/middle cerebral artery M1 segment) 6-16 hours after the onset of stroke) occlusive ischemic stroke, and the method of the present invention may be applied to the subject in combination with this endovascular therapy.

In certain embodiments, the subjects are given intravenous rt-PA thrombolysis after the onset of acute stroke, and at the same time, the method of endovascular treatment (e.g., performing thrombectomy and reperfusion to the subject within 6 hours, or 8 hours, or 6-16 hours after the onset of stroke) is adopted due to large vessel occlusion or severe condition. The method of the present invention may also be applied to the subject in combination with the above method of intravenous thrombolysis and the method of endovascular treatment. The method of the present invention has good safety and does not cause or aggravate the risk of bleeding, so it can be applied to subjects in combination with intravenous thrombolysis and intravascular interventional therapy.

In certain embodiments, the subject of an ischemic stroke attack is an ischemic non-reperfusion subject, such as subjects not given intravenous rt-PA thrombolysis and/or not reperfused with endovascular therapy. In certain embodiments, the subjects having an ischemic stroke attack are subjects with large cerebral infarction, also referred to as malignant middle cerebral artery infarction.

Large hemispheric infarction (LHI), also known as malignant middle cerebral artery infarction, is an important disease leading to death or disability in humans. Such patients have typical clinical symptoms and often die from transtentorial herniation. In certain embodiments, the method of the present invention treats subjects who are reperfused after 3 hours of occlusion in large anterior circulation vessel (e.g., M1 segment of the middle cerebral artery) as confirmed by MRA imaging. In certain embodiments, the method of the present invention treats subjects whose edema/infarct volume accounts for about 70% of the hemisphere volume at the peak of edema (75 hours after stroke) to form a large cerebral infarction. The continuous use of the method of the present invention in subjects for 28 days can significantly reduce the volume of cerebral edema/infarction in the subjects, significantly reduce the volume of the ischemic core area and significantly improve the condition of ischemia at 75 hours, and can improve neurological deficits in the subjects. The NIHSS score of the subjects treated with this method can be reduced by about 50% or even about 70% compared with the subjects not treated with this method.

Advantageously, the inventors of the present application have found that the method has beneficial effects in preventing, treating, and/or improving stroke, especially ischemic stroke. Without theoretical constrains, the pharmaceutical components administered in this method combine multiple mechanisms such as inhibition of neuroinflammation, anti-oxidative stress, and amelioration of ischemia, so as to protect nerves and prevent the expansion of infarct core area. In particular, the method for preventing, treating, and/or improving stroke of the present invention improves the cerebral tissue perfusion of the subject in the acute phase after stroke (i.e., 75 hours after stroke), inhibits the reduction of regional cerebral blood volume (rCBV) around the cerebral tissue infarction, improves the ischemic condition in the acute phase (75 hours after stroke) and rescues the ischemic penumbra; significantly reduce the brain edema/infarct volume in the subjects; inhibit central nervous inflammation, and inhibit overexpression of glial fibrillary acidic protein (GFAP); treat the destructive effect of ischemia on the central nervous system of subjects; reduce neurobehavioral score (e.g., NIHSS) of cerebral stroke, improve neurological deficit in the subject, especially improve and restore finger function and limb strength of an affected limb in the subject, and reduce the disability rate of the subject; and/or prevent, treat, and improve cognitive dysfunction of the subject, including aspects of memory, visuospatial, and comprehension judgment, prevent and impede development of dementia.

In certain embodiments, the method for treating, preventing, or improving stroke improves cerebral tissue perfusion in a subject in the acute phase after stroke (75 hours after stroke), inhibits reduction of regional cerebral blood volume (rCBV) around cerebral tissue infarction, improves acute ischemic condition, and saves penumbra. Local cerebral ischemia consists of central necrotic area and peripheral ischemic penumbra, cerebral cells die in the necrotic area, and there are a large number of surviving neurons in ischemic penumbra due to collateral circulation. If blood flow in the ischemic penumbra can be quickly restored in a short time, the brain injury in this area is reversible, and nerve cells may survive and recover their metabolic function. Advantageously, the volume of the non-perfusion area (i.e., the ischemic core area) of brain tissue in the acute phase (75 hours after stroke) of subjects with stroke attack treated with the method of the present invention is reduced by at least 70% compared with subjects with stroke attack not treated with the method.

In certain embodiments, the method for treating, preventing, or improving stroke reduces brain edema/infarct volume in a subject. The volume of cerebral edema/necrosis refers to the volume of local cerebral ischemia and hypoxic necrosis caused by cerebral blood supply disorder. Ischemic stroke is characterized by significant cell death and obvious inflammatory reaction at infarct site. Cerebral edema volume refers to the volume of excess fluid accumulation in the intracellular or extracellular space of the brain. Advantageously, the inventors used 3.0 T GE MRI to dynamically observe edema/infarct trajectories in subjects at 3-4 h, 72-75 h, Day 7, Day 14 and Day 28 after stroke. MRI imaging sequence includes T1WI, T2 FLAIR, DWI, MRA, CTA. Among them, T1WI may show the anatomical structure of brain, differentiate between gray matter area and white matter area, diagnose hemorrhage and ischemia, etc., T2 FLAIR and DWI may quantify edema/necrosis volume, and T2 FLAIR may suggest increased water content within the tissue, and it is used to show inflammation, ischemia, edema and other lesions. In the present invention, MRA images demonstrate that T2 FLAIR cerebral edema/infarct volume of subjects after a stroke attack who were continuously administered for 28 days treated with the method of the present invention, particularly subjects reperfused after 3-hour and 1.5-hour occlusion of a large anterior circulatory vessel (middle cerebral artery M1 segment) and having more than moderate neurological impairment, was reduced by at least 30% (75 hours after stroke), at least 40% (7 days after stroke) or even close to 50% (28 days after stroke) as compared with T2 FLAIR in subjects with a stroke attack not treated with this method.

In certain embodiments, the method for treating, preventing, or improving stroke inhibits central nervous inflammation, in particular overexpression of glial fibrillary acidic protein (GFAP). Without being constrained by theory, the inventors found that the expression of GFAP in the peri-infarct area of brain tissue of subjects with stroke attack was significantly increased compared with that in contralateral normal brain tissue. Advantageously, the method of the present invention significantly inhibits the increase of GFAP expression in the injured area of brain tissue of subjects with neuroinflammation caused by ischemic stroke attack.

In certain embodiments, the method for treating, preventing, or improving stroke reduces the neurobehavioral score (e.g., NIHSS) of the stroke subjects, improves the neurological impairment of the subjects, especially improve and restore finger function and limb strength of an affected limb in the subject, and reduce the disability rate of the subject. Neurological deficits refer to symptoms after the deficits of nerve structure, and the deficit symptoms refer to weakening or disappearance of normal function after the nerve structure is impaired. Advantageously, the NIHSS score of a subject with a stroke attack treated with the method of the present invention may be reduced by at least 40% (14 days after stroke), at least 50% (28 days after stroke), or even 60% compared to the NIHSS score of a subject with a stroke attack not treated with the method. At the same time, the inventor found that the subjects not treated with the method had long-term neurological impairment and disability 3 months after stroke, and the subjects' contralateral hand function was impaired due to involvement of the anterior cerebral gyrus, e.g., the loss of the opposable finger movement of thumb and index finger, leading to failure in completing the fine task of peeling peanuts with both hands. Advantageously, the subject treated with the method of the present invention has normal hand function on the affected side 3 months after stroke, and the opposite finger movement of the thumb and index finger is not affected, so that the fine task of peeling peanuts with both hands can be completed.

Cognitive impairment and even dementia may develop after a stroke or in cases of certain infections, repetitive physical injuries to the brain, or nutritional deficiencies. In certain embodiments, the method for preventing, treating, and/or improving stroke of the present invention prevents, treats, and improves cognitive dysfunction of a subject, including cognitive dysfunction in memory, visuospatial and comprehension judgment, especially visuospatial, and prevents and prevents the development of dementia. Visual Spatial Dysfunction refers to the dysfunction caused by the patients' inability to accurately judge the position of themselves and their articles, which often manifests itself in the following ways: patients cannot find the parking space when they park their cars; they get lost when they go home due to misjudgment of the direction; when they lay a tablecloth, they cannot make the tablecloth aligned with the table due to failure to correctly judge the position of the tablecloth and the table corners; they cannot accurately place a pot on the stove, and drop the pot to the ground; patients cannot accurately copy stereo images, and in severe cases, they cannot even draw a simple plan; the patient has difficulty in dressing, and cannot tell the upper part from the lower part, and the left part and the right part of the clothes, and wear clothes and pants the other way around. Advantageously, in the spatial delayed response experiment 3 months after stroke, the subjects having a stroke attack treated with the method of the present invention all successfully passed the delayed 1s, 5s, 10s, and 30s task tests, while the subjects having a stroke attack not treated with the method of the present invention only passed the delayed 1s task test, or even failed to complete the delayed 1s task test.

In certain embodiments, the stroke is a hemorrhagic stroke. Hemorrhagic stroke accounts for about 17% of stroke cases. It is caused by fragile blood vessels rupturing and bleeding to the surrounding brain. Blood accumulates and presses the surrounding brain tissue. Two common types of hemorrhagic stroke are intracerebral hemorrhage and subarachnoid hemorrhage. Hemorrhagic stroke is caused by weakened vascular rupture. Potential causes of vascular rupture include hypertensive hemorrhage, in which hypertension leads to vascular rupture, or other potential causes of vascular weakening, such as cerebrovascular malformation rupture, including cerebral aneurysm, arteriovenous malformation (AVM), or cavernous vascular malformation. Hemorrhagic stroke may also be caused by hemorrhagic transformation of ischemic stroke, which leads to weakening of infarcted blood vessels, or hemorrhage of primary or metastatic tumors containing abnormally fragile blood vessels in the central nervous system. Hemorrhagic stroke may also be due to iatrogenic causes, such as direct surgical injury to cerebral vessels. Aneurysm is swelling of a weak area of a blood vessel. If not treated in time, aneurysm may continue to weaken until it ruptures and bleeds into the brain. Arteriovenous malformation (AVM) is a group of abnormally formed blood vessels. Cavernous vascular malformation is a venous abnormality, which may lead to weakening of venous structure and lead to bleeding. Any one of these blood vessels may rupture, which may also lead to cerebral hemorrhage. Hemorrhagic stroke may also be caused by physical trauma.

In certain embodiments, the hemorrhagic stroke includes a condition of cerebral hemorrhage and/or a condition of subarachnoid hemorrhage. Intracerebral hemorrhage refers to non-traumatic bleeding within the brain parenchyma. Intracranial blood vessels rupture and blood flows into subarachnoid space, which is called subarachnoid hemorrhage. It is divided into traumatic and spontaneous cases, and spontaneous cases are further divided into primary and secondary types. Primary subarachnoid hemorrhage is the case where lesions (e.g., congenital aneurysm, cerebrovascular malformation, microaneurysm caused by hypertensive cerebral arteriosclerosis, etc.) of blood vessels of cerebral fundus or cerebral surface rupture, and blood flows to subarachnoid space, which accounts for about 10% of acute stroke; and secondary subarachnoid hemorrhage is the case where intracerebral hematoma penetrates brain tissue, and bloods flow to subarachnoid space.

### 1.1.2 Transient ischemic attack

In certain embodiments, the cerebrovascular disease is a transient ischemic attack. Transient ischemic attack (TIA) is a transient neurologic deficit caused by local cerebral or retinal ischemia. Its clinical symptoms generally last no more than 1 hour and at most 24 hours, with mild or warning stroke. Subjects who undergo heart surgery are particularly prone to transient ischemic attack.

Advantageously, the inventors of the present application have found that the method has beneficial effects in preventing, treating, and/or improving transient ischemic attack. Particularly advantageously, the method for preventing, treating and/or improving transient ischemic attack of the present invention improves the cerebral tissue perfusion of the subject in the acute phase after stroke (75 hours after stroke), inhibits the reduction of regional cerebral blood volume (rCBV) in the injured area of brain tissue, improves the ischemic condition in the acute phase and rescues penumbra; inhibits central nervous inflammation, and inhibits overexpression of glial fibrillary acidic protein (GFAP); treats the destructive effect of ischemia on the central nervous system of subjects; reduces neurobehavioral score (e.g., NIHSS) of cerebral stroke, improves neurological deficit in the subject, especially improves and restores finger function and limb strength of an affected limb in the subject, and reduces the disability rate of the subject; and/or prevents, treats, and improves cognitive dysfunction of the subject, including aspects of memory, visuospatial, and comprehension judgment, and prevents and impedes development of dementia.

In certain embodiments, the method for treating, preventing, or improving transient ischemic attack improves cerebral tissue perfusion in a subject in the acute phase after stroke (75 hours after stroke), inhibits reduction of regional cerebral blood volume (rCBV) in injured areas of brain tissue, improves acute ischemic state, and saves penumbra. Advantageously, the volume of the non-perfusion area (i.e., the ischemic core area) of the brain of subjects with transient ischemic attack treated with the method of the present invention is reduced by at least 70% compared with subjects with transient ischemic attack not treated with the method.

In certain embodiments, the method for treating, preventing, or improving transient ischemic attack inhibits central nervous inflammation, in particular overexpression of glial fibrillary acidic protein (GFAP). Without being bound by theory, the inventors found that the expression of GFAP in brain tissue of subjects with transient ischemic attack was significantly increased compared with that in contralateral normal brain tissue. Advantageously, the method of the present invention significantly inhibits the increase of GFAP expression in the injured area of brain tissue of subjects with neuroinflammation caused by transient ischemic attack.

In certain embodiments, the method for treating, preventing, or improving transient ischemic attack reduces the neurobehavioral score (e.g., NIHSS) of the stroke subjects, improves the neurological impairment of the subjects, especially improve and restore finger function and limb strength of an affected limb in the subject, and reduce the disability rate of the subject. Advantageously, the NIHSS score of a subject with transient ischemic attack treated with the method of the present invention may be reduced by at least 40% (14 days after stroke), at least 50% (28 days after stroke), or even 60% compared to the NIHSS score of a subject with transient ischemic attack not treated with the method.

In certain embodiments, the method for preventing, treating, and/or improving transient ischemic attack of the present invention prevents, treats, and improves cognitive dysfunction of a subject, including cognitive dysfunction in memory, visuospatial and comprehension judgment, especially visuospatial, and prevents and prevents the development of dementia. Advantageously, in the spatial delayed response experiment, the subjects having transient ischemic attack treated with the method of the present invention all successfully passed the delayed 1s, 5s, 10s, and 30s task tests, while the subjects having transient ischemic attack not treated with the method of the present invention only passed the delayed 1s task test, or even failed to complete the delayed 1s task test.

### 1.2 Craniocerebral injury

In certain embodiments, the central nervous system disease, disorder or condition is craniocerebral injury. In certain embodiments, the present invention provides a method for treating, preventing or improving craniocerebral injury, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

Craniocerebral injury refers to any trauma to the skull and brain. In certain embodiments, the craniocerebral injury is traumatic brain injury. Traumatic brain injury (TBI) refers to injury to the brain exerted by a non-degenerative, non-reproductive external mechanical force, which may lead to permanent or temporary impairment to cognitive, physiological and psychosocial functions, accompanied by diminished or altered consciousness. Traumatic brain injury is an injury that affects the way the brain works. It may be caused by bump, blow, or shake to the head, or a penetrating head injury (such as a gunshot). Traumatic brain injury is mainly divided into three types: mild TBI or concussion; moderate TBI; and severe TBI. TBI usually originates from primary injury, and then gradually develops into secondary injury. Secondary pathogenesis includes ischemic changes, inflammatory cascade reaction, oxidative stress, etc., which may lead to temporary or permanent neurologic deficit. Improving ischemia, inhibiting neuroinflammation and resisting oxidative stress are key mechanisms of neuroprotection and repair of TBI (Galgano M, Toshkezi G, Qiu X, et al. Traumatic Brain Injury: Current Treatment Strategies and Future Endeavors [J]. Cell Transplantation, 2017, 26(7): 1118-1130.).

Without being bound by theory, neurons in subjects having traumatic brain injury are difficult to be free from influence of brain injury in the early stage, and depend on the environmental support of various glial cells and immune cells in the later stage of brain tissue reconstruction and repair. Cerebral ischemia-reperfusion (I/R) injury is accompanied by proliferation and activation of glial cells, neuroinflammation and cytokine cascade reaction, which leads to nerve cell death. The increased expression of astrocyte biomarker glial fibrillary acidic protein (GFAP) is regarded as a marker of neuroinflammation, and the expression of various pro-inflammatory cytokines is increased at the same time, aggravating I/R injury. For patients who have already suffered from traumatic brain injury, it is also extremely important to prevent and delay the death of nerve cells, which may preserve more nerve cells, so as to restore their functions and gain more time for treatment.

Without being bound by theory, the inventors of the present application found that the pharmaceutical components administered in this method combine multiple mechanisms such as inhibition of neuroinflammation, anti-oxidative stress, and amelioration of ischemia, so as to protect nerves and prevent the expansion of infarct core area. Advantageously, the inventors of the present application have found that the method has beneficial effects in preventing, treating, and/or improving traumatic brain injury. Particularly advantageously, the method for preventing, treating, and/or improving traumatic brain injury of the present invention inhibits central nervous inflammation, in particular GFAP overexpression.

### 1.3 Neurodegenerative diseases

In certain embodiments, the central nervous system disease, disorder, or condition is a neurodegenerative disease. In certain embodiments, the present invention provides a method for treating, preventing, or improving neurodegenerative diseases, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

Neurodegenerative disease is a group of diseases with unknown causes that chronically and progressively damage nerves and other tissues. Many degenerative diseases may be the result of a series of complex molecular biological disorders in tissues such as nerves during derivation, development, maturation and aging, thus exhibiting structural and functional changes. The degeneration process may involve the whole nerve cell (cell body, nucleus, axon tip) and may also affect other components such as myelin sheath.

In certain embodiments, the neurodegenerative disease includes dementia, wherein dementia includes vascular dementia, Alzheimer's disease, Lewy body dementia, and/or frontotemporal dementia. Cognitive impairment and even dementia may develop after stroke or in cases of certain infections, repetitive physical injuries to the brain, or nutritional deficiencies. The boundaries between different forms of dementia are blurred, but cerebrovascular injury is often present in various dementia. In certain embodiments, the dementia includes vascular dementia.

In certain embodiments, the dementia includes Alzheimer's disease. Alzheimer's disease (AD) is a degenerative disease of the central nervous system, which occurs in old age and pre-old age and is characterized by progressive cognitive dysfunction and behavioral impairment. Its clinical manifestations are memory impairment, aphasia, apraxia, agnosia, impairment of visuospatial abilities, impairment of abstract thinking and calculation, personality and behavioral changes, etc. AD is the most common type of senile dementia, accounting for about 50%-70% of senile dementia.

In certain embodiments, the dementia includes Lewy body dementia. Dementia with Lewy bodies (DLB) is a neurodegenerative disease. Its clinical manifestations are fluctuating cognitive impairment, Parkinson's syndrome, and psychiatric symptoms with visual hallucinations as prominent manifestations. A change in pathological feature is visible presence of Lewy bodies in the cerebral cortex.

In certain embodiments, the dementia includes frontotemporal dementia. Frontotemporal dementia refers to slow onset of dementia syndromes of personality changes, speech disorders, and abnormal behaviors in middle-aged and elderly patients. Neuroimaging shows atrophy of frontotemporal lobe. This disease is a common cause of neurodegenerative dementia, accounting for about 1/4 of all dementia patients.

Without theoretical constraints, cytokines play an important role in the process of neuroinflammation. Pro-inflammatory cytokines such as IFN-γ, IL-1β, TNF-α, and IL-6 participate in the process of neuroinflammation. In addition, IFN-γ and TNF-α jointly increase NO level and participate in the death of dopaminergic neurons, thus promoting the development of Alzheimer's disease. Increased expression of astrocyte biomarker glial fibrillary acidic protein (GFAP) is regarded as a marker of neuroinflammation. Neuroinflammation leads to pathological processes such as white matter lesions, hippocampus injury, and Aβ deposition, and then leads to the development of cognitive impairment-related diseases such as vascular dementia, and Alzheimer's disease. Therefore, neuroinflammation plays a key role in the pathogenesis of various dementia and neurodegenerative changes.

Advantageously, the inventors of the present application have found that the method has beneficial effects in preventing, treating, and/or improving dementia. Without being bound by theory, the pharmaceutical components administered in this method combine multiple mechanisms such as inhibition of neuroinflammation, anti-oxidative stress, and amelioration of ischemia, and break the vicious circle between nerve injury and neuroinflammation to protect nerves.

Particularly advantageously, the method for preventing, treating, and/or improving dementia according to the present invention prevents, treats, and improves cognitive dysfunction of a subject, including cognitive dysfunction in memory, visuospatial and comprehension judgment, especially the visuospatial aspect, and prevents and impedes the development of dementia. Advantageously, in the spatial delayed response experiment, the subjects with cognitive impairment treated with the method of the present invention all successfully passed the delayed 1s, 5s, 10s, and 30s task tests, while the subjects with cognitive impairment not treated with the method of the present invention only passed the delayed 1s task test, or even failed to complete the delayed 1s task test.

More advantageously, the method for preventing, treating, and/or improving dementia of the present invention also improves cerebral tissue perfusion of the subjects, inhibits reduction of regional cerebral blood volume (rCBV) around cerebral tissue infarction, improves ischemia and saves penumbra; significantly reduces the brain edema/infarction volume of the subjects; inhibits central nervous inflammation, especially inhibits GFAP overexpression; significantly reduces neurobehavioral scores (e.g., NIHSS) and improves neurologic deficit, and/or reduces the overall disability rate of the subjects.

In certain embodiments, the method for treating, preventing, or improving dementia improves cerebral tissue perfusion in the subject, inhibits reduction in regional cerebral blood volume (rCBV) around cerebral tissue infarction, improves ischemia and save penumbra. Advantageously, the volume of the non-perfusion area (i.e., the ischemic core area) of the brain of subjects with cognitive impairment treated with the method of the present invention is reduced by at least 70% compared with subjects with cognitive impairment not treated with the method.

In certain embodiments, the method for treating, preventing, or improving dementia reduces brain edema/infarction volume in a subject. 3.0 T GE MRI is used to dynamically observe edema/infarction trajectories in subjects at 3-4 h, 72-75 h, Day 7, Day 14, and Day 28 after stroke. Advantageously, the inventors confirmed by MRA imaging that T2 FLAIR cerebral edema/infarction volume of subjects who were continuously administered for 60 days using the method of the present invention, particularly subjects reperfused after 3-hour occlusion of a large anterior circulatory vessel (middle cerebral artery M1 segment) and having more than moderate neurological impairment, was reduced by at least 30% (72 hours after stroke), at least 40% (7 days after stroke) or even close to 50% (28 days after stroke) as compared with T2 FLAIR in subjects with cognitive impairment not treated with this method.

In certain embodiments, the method for treating, preventing, or improving dementia inhibits central nervous inflammation, in particular overexpression of glial fibrillary acidic protein (GFAP). Without being constrained by theory, the inventors found that the expression of GFAP in the peri-infarct area of brain tissue of subjects with cognitive impairment caused by stroke was significantly increased compared with that in contralateral normal brain tissue. Advantageously, the method of the present invention significantly inhibits the increase of GFAP expression in the brain tissue impaired area of subjects with cognitive impairment due to neuroinflammation.

In certain embodiments, the method for treating, preventing, or improving dementia significantly reduces neurobehavioral scores (e.g., NIHSS) and improves neurologic deficit, especially improves and restores finger function and limb strength of an affected limb in the subject, and reduces the overall disability rate of the subject. Advantageously, the NIHSS scores of subjects treated with the method of the present invention for continuous drug administration for 60 days, especially those who could be confirmed by MRA imaging to have reperfusion after 3-hour anterior circulation large vessel (M1 segment of middle cerebral artery) occlusion with moderate or more serious neurological impairment, could be reduced by at least 30%, at least 40%, at least 50%, at least 60%, or even close to 70% compared with the NIHSS scores of subjects with cognitive impairment not treated with the method. The inventors found that subjects with cognitive impairment not treated with the method developed long-term neurological impairment and disability 3 months after the onset of stroke, and the subjects' anterior cerebral gyrus was involved, resulting in impaired contralateral hand function, e.g., the loss of the opposable finger movement of thumb and index finger, leading to a failure in completing fine tasks. Advantageously, the dementia subject treated with the method of the present invention has normal hand function on the affected side 3 months after the onset of stroke, and the opposite finger movement of thumb and index finger is not affected, and can complete fine tasks with both hands.

### 1.4 Optic neuropathy

In certain embodiments, the central nervous system disease, disorder, or condition is optic neuropathy, which includes glaucoma, ischemic optic neuropathy, optic neuritis, optic nerve tumor, and/or traumatic optic neuropathy. Optic neuropathy refers to optic nerve injury or disease, which affects the survival and function of retinal neurons and affects the function of transmission of visual signals from eye retina to brain.

In certain embodiments, the method for preventing, treating, and/or improving optic neuropathy involves inhibiting or mitigating retinal ganglion cell (RGC) injury or severe axonal injury in a subject.

In certain embodiments, the present invention provides a method for inhibiting or mitigating retinal ganglion cell (RGC) injury or severe axonal injury in a subject, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof. In certain embodiments, the method treats, prevents, or improves a disease, disorder, or condition associated with retinal ganglion cell (RGC) injury or severe axonal injury. Diseases associated with retinal ganglion cell (RGC) injury or severe axonal injury include: glaucoma, ischemic optic neuropathy, optic neuritis, optic nerve tumor, traumatic optic neuropathy, retinal artery occlusion, retinal vein occlusion, and retinopathy of prematurity.

### 1.4.1 Glaucoma

In certain embodiments, the optic neuropathy is glaucoma. The present invention provides a method for treating, preventing, or improving glaucoma, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

Glaucoma is a group of eye diseases characterized by progressive retinal ganglion cell (RGC) death and typical visual field defect, which has become a main cause of irreversible blindness in the world. The main risk factors associated with this disease include ischemia, gliosis, inflammation, excitotoxicity, and oxidative stress. At present, lowering intraocular pressure is only effective partially in protecting the visual function of glaucoma patients. Therefore, it is very important to develop new therapeutic methods to provide safe and effective RGC neuroprotection. Subjects are diagnosed with glaucoma, including primary open-angle glaucoma, angle-closure glaucoma, secondary glaucoma, normal tension glaucoma, or congenital glaucoma. Glaucoma may have normal intraocular pressure or high intraocular pressure.

In certain embodiments, the glaucoma includes primary glaucoma, secondary glaucoma, and/or developmental glaucoma. Secondary glaucoma refers to a type of glaucoma with clear causes such as other diseases of the eye or systemic diseases. Developmental glaucoma is a type of glaucoma caused by dysplasia or abnormal development in the outcome of eyeball angle during embryonic and developmental stages, which develops before and after birth and in infancy and early childhood, as well as in adolescence and childhood.

Primary glaucoma is a main type of glaucoma seen in people aged 18 years and above, it is usually bilateral, but the two eyes may develop at different times and often have different degrees of severity. Primary glaucoma is divided into two categories: angle-closure glaucoma and open-angle glaucoma. The etiologic mechanism of this type of glaucoma has been gradually understood but not yet clarified after a long period of research. In order to distinguish it from secondary glaucoma, it is customarily referred to as primary glaucoma.

In certain embodiments, the primary glaucoma includes open-angle glaucoma, angle-closure glaucoma, and/or special types of glaucoma. Primary open-angle glaucoma refers to a group of syndromes with optic nerve injury and open anterior chamber angle, in which intraocular pressure can be elevated or sometimes in a normal range. Primary angle-closure glaucoma refers to a type of glaucoma in which the anterior chamber angle is mechanically blocked by the peripheral iris tissue due to the existing iris configuration, which leads to the obstruction of aqueous humor outflow and the increase of intraocular pressure.

The term "special types of glaucoma" refers to a unique type of glaucoma, which is still primary and different from angle-closure and open-angle glaucoma, including high-pleated iris glaucoma, malignant glaucoma, normal intraocular pressure glaucoma, pigmentary glaucoma, and exfoliative glaucoma.

Advantageously, the inventors of the present application have found that the method has beneficial effects in preventing, treating, and/or improving glaucoma. Particularly advantageously, the method for preventing, treating, and/or improving glaucoma of the present invention mitigates retinal ganglion cell (RGC) loss, protects visual function, and inhibits or reduces severity of secondary neuronal injury; mitigates internal retinal injury, reduces retinal edema and mitigates retinal thickness reduction; reduces edema of optic nerve fiber layer, maintains thickness of retinal nerve fiber layer and prevents thinning of optic nerve fiber layer; significantly inhibits optic nerve inflammation and GFAP overexpression, inhibits the expression level of inflammatory factors such as IFN-γ, IL-1β, and TNF-α that are associated with nerve cell injury; and mitigates optic nerve function decline, including improving functional indexes of full-field electroretinogram (FERG) and flash visual evoked potential (FVEP), and/or protecting overall retinal function.

In certain embodiments, the method for treating, preventing, or improving glaucoma mitigates retinal ganglion cell (RGC) loss, protects visual function, and inhibits or reduces the severity of secondary neuronal injury. Advantageously, the average loss rate of the number of retinal ganglion cells in glaucoma subjects treated with the method of the present invention is reduced by at least 80% compared with the placebo group (i.e., subjects not treated with the method of the present invention), while the one-component control group (i.e., subjects administered with only one pharmaceutical component), the two-component control group (i.e., subjects administered with only two components of DOX+MTP) and the three-component control group (i.e., subjects administered with a three-component drug DOX+MTP+BRM different from the present invention) only reduce the average loss rate of the number of retinal ganglion cells by 20%, or even only 15%.

In certain embodiments, the method for treating, preventing, or improving glaucoma significantly inhibits optic nerve inflammation and GFAP overexpression. Astrocytes are the main glial cells in normal retina, which play a vital role in maintaining blood-retinal barrier and RGC health. The increased expression of astrocyte biomarker GFAP is regarded as a sign of neuroinflammation and a marker of retinal degeneration. Without being bound by theory, the inventors found that the expression of GFAP in retinal tissue of glaucoma subjects increased by about 5 times of normal expression (i.e., the expression of GFAP in retinal tissue of subjects without glaucoma), which is considered as overexpression. Advantageously, the expression of GFAP in retinal tissue of glaucoma subjects treated with the method of the present invention is reduced to 50% or less, 40% or less, or even close to 30% of the overexpression.

In certain embodiments, the method for treating, preventing, or improving glaucoma inhibits an increased level of expression of at least one inflammatory factor, wherein the inflammatory factor includes IFN-γ, IL-1β, and/or TNF-α. Without being bound by theory, the inventors found that the expression level of inflammatory factors in retinal tissue of subjects with glaucoma was significantly increased compared with normal levels (i.e., the expression level of inflammatory factors in retinal tissue of subjects without glaucoma). Advantageously, the expression level of inflammatory factors in retinal tissue of glaucoma subjects treated with the method of the present invention is reduced to be close to normal levels.

In certain embodiments, the method for treating, preventing, or improving glaucoma mitigates internal retinal injury, reduces retinal edema, and mitigates retinal thickness reduction. Advantageously, the reduction in the average thickness of the retinal EDTRS area in glaucoma subjects treated with the method of the present invention is reduced by at least 70%, or even by at least 80%, compared with the placebo group (i.e., glaucoma subjects not treated with the method of the present invention), while the one-component control group (i.e., glaucoma subjects administered with only one pharmaceutical component), the two-component control group (i.e., glaucoma subjects administered with only two components of DOX+MTP) and the three-component control group (i.e., glaucoma subjects administered with a three-component drug DOX+MTP+BRM different from the present invention) only reduce the reduction in the average thickness of the retinal EDTRS area by about 30%, or 20%, or 10%, or 5%, or even increase the reduction instead of reducing the reduction. More advantageously, the reduction in the average thickness of the retinal EDTRS area of non-glaucoma subjects treated with the method of the present invention is reduced by at least 30%, at least 40%, at least 50%, at least 60%, or even close to 70% compared to the control group (i.e., non-glaucoma subjects not treated with the method of the present invention).

In certain embodiments, the method for treating, preventing, or improving glaucoma reduces retinal nerve fiber layer edema, maintains retinal nerve fiber layer thickness, and prevents thinning of the optic nerve fiber layer. The inventors observed a rhesus monkey eye phenotype of spontaneous glaucoma, which exhibits important ophthalmic features of human primary open-angle glaucoma, including thinning of the retinal peripapillary nerve fiber layer (RNFL). (Louis R Pasquale et al. Development of Primary Open Angle Glaucoma-Like Features in a Rhesus Macaque Colony From Southern China. [J]. Transl Vis Sci Technol. 2021 Aug 2; 10 (9): 20) Advantageously, the reduction in the average thickness of the retinal nerve fiber layer (RNFL) in glaucoma subjects treated with the method of the present invention is reduced by at least 80%, compared with the placebo group (i.e., glaucoma subjects not treated with the method of the present invention), while the one-component control group (i.e., glaucoma subjects administered with only one pharmaceutical component), the two-component control group (i.e., glaucoma subjects administered with only two components of DOX+MTP) and the three-component control group (i.e., glaucoma subjects administered with a three-component drug DOX+MTP+BRM different from the present invention) only reduce the reduction in the average thickness of the retinal nerve fiber layer (RNFL) by about 30%, or even increase the reduction instead of reducing the reduction.

In certain embodiments, the method for treating, preventing, or improving glaucoma mitigating optic nerve function decline includes improving functional indexes of full-field electroretinogram (FERG) and flash visual evoked potential (FVEP), and protecting overall retinal function. Advantageously, amplitudes of the a-wave and b-wave of the light-adapted 3.0 ERG response of the full-field electroretinogram (FERG) of glaucoma subjects treated with the method of the present invention are increased by about 30%, or 50%, or 100%, or 150%, or even close to 200% compared with the control group (i.e., glaucoma subjects not treated with the method of the present invention). More advantageously, the N2-P2 amplitude of the flash visual evoked potential (FVEP) of glaucoma subjects treated with the method of the present invention is increased by about 100%, or 200%, or 300%, or 400%, or even close to 500% compared with the control group (i.e., glaucoma subjects not treated with the method of the present invention).

### 1.4.2 Ischemic optic neuropathy

In certain embodiments, the optic neuropathy is ischemic optic neuropathy. The present invention provides a method for treating, preventing, or improving ischemic optic neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

Ischemic optic neuropathy is caused by acute circulation of nutrient vessels of optic nerve. Clinically, it can be divided into anterior ischemic optic neuropathy and posterior ischemic optic neuropathy. The clinical manifestation is a sudden, isolated, and usually painless visual impairment with optic disc swelling. Anterior ischemic optic neuropathy is a disease with acute ischemia and edema of optic disc caused by insufficient blood supply to optic disc caused by posterior ciliary artery circulation disorder. Posterior ischemic optic neuropathy is a disease in which acute circulation disorder occurs in optic nerve vessels from posterior lamina cribrosa to optic chiasma, and optic nerve function is damaged due to ischemic insufficiency.

Advantageously, the inventors of the present application have found that the method has beneficial effects in preventing, treating, and/or improving ischemic optic neuropathy. Particularly advantageously, the method for preventing, treating, and/or improving ischemic optic neuropathy of the present invention mitigates retinal ganglion cell (RGC) loss, protects visual function, and reduces severity of secondary neuronal injury associated with optic neuropathy; mitigates internal retinal injury, reduces retinal edema and mitigates retinal thickness reduction; reduces edema of optic nerve fiber layer, maintains thickness of retinal nerve fiber layer and prevents thinning of optic nerve fiber layer; significantly inhibits optic nerve inflammation and GFAP overexpression, inhibits an expression level of inflammatory factors such as IFN-γ, IL-1β, and/or TNF-α that are associated with nerve cell injury; and mitigates optic nerve function decline, including improving functional indexes of full-field electroretinogram (FERG) and flash visual evoked potential (FVEP), and/or protecting overall retinal function.

In certain embodiments, the method for treating, preventing, or improving ischemic optic neuropathy mitigates retinal ganglion cell (RGC) loss, protects visual function, and inhibits or reduces the severity of secondary neuronal injury associated with optic neuropathy. Advantageously, the average loss rate of the number of retinal ganglion cells in subjects with ischemic optic neuropathy treated with the method of the present invention is reduced by at least 80% compared with the placebo group (i.e., subjects with ischemic optic neuropathy not treated with the method of the present invention), while the one-component control group (i.e., subjects with ischemic optic neuropathy administered with only one pharmaceutical component), the two-component control group (i.e., subjects with ischemic optic neuropathy administered with only two components of DOX+MTP) and the three-component control group (i.e., subjects with ischemic optic neuropathy administered with a three-component drug DOX+MTP+BRM different from the present invention) only reduce the average loss rate of the number of retinal ganglion cells by 20%, or even only 15%.

In certain embodiments, the method for treating, preventing, or improving ischemic optic neuropathy significantly inhibits optic neuroinflammation and GFAP overexpression. Without being bound by theory, the inventors found that the expression of GFAP in retinal tissue of subjects with ischemic optic neuropathy increased by about 5 times of normal expression (i.e., the expression of GFAP in retinal tissue of subjects without ischemic optic neuropathy), which is considered as overexpression. Advantageously, the expression of GFAP in retinal tissue of subjects with ischemic optic neuropathy treated with the method of the present invention is reduced to 50% or less, 40% or less, or even close to 30% of the overexpression.

In certain embodiments, the method for treating, preventing, or improving ischemic optic neuropathy inhibits an increased level of expression of at least one inflammatory factor, wherein the inflammatory factor includes IFN-γ, IL-1β, and/or TNF-α. Without being bound by theory, the inventors found that the expression level of inflammatory factors in retinal tissue of subjects with ischemic optic neuropathy was significantly increased compared with normal levels (i.e., the expression level of inflammatory factors in retinal tissue of subjects without ischemic optic neuropathy). Advantageously, the expression level of inflammatory factors in retinal tissue of subjects with ischemic optic neuropathy treated with the method of the present invention is reduced to be close to normal levels.

In certain embodiments, the method for treating, preventing, or improving ischemic optic neuropathy mitigates internal retinal injury, reduces retinal edema, and mitigates retinal thickness reduction. Advantageously, the reduction in the average thickness of the retinal EDTRS area in subjects with ischemic optic neuropathy treated with the method of the present invention is reduced by at least 70%, or even by at least 80%, compared with the placebo group (i.e., subjects with ischemic optic neuropathy not treated with the method of the present invention), while the one-component control group (i.e., subjects with ischemic optic neuropathy administered with only one pharmaceutical component), the two-component control group (i.e., subjects with ischemic optic neuropathy administered with only two components of DOX+MTP) and the three-component control group (i.e., subjects with ischemic optic neuropathy administered with a three-component drug DOX+MTP+BRM different from the present invention) only reduce the reduction in the average thickness of the retinal EDTRS area by about 30%, or 20%, or 10%, or 5%, or even increase the reduction instead of reducing the reduction. More advantageously, the reduction in the average thickness of the retinal EDTRS area of subjects without ischemic optic neuropathy treated with the method of the present invention is reduced by at least 30%, at least 40%, at least 50%, at least 60%, or even close to 70% compared to the control group (i.e., subjects without ischemic optic neuropathy not treated with the method of the present invention).

In certain embodiments, the method for treating, preventing, or improving ischemic optic neuropathy reduces retinal nerve fiber layer edema, maintains retinal nerve fiber layer thickness, and prevents thinning of the optic nerve fiber layer. Advantageously, the reduction in the average thickness of the retinal nerve fiber layer (RNFL) in subjects with ischemic optic neuropathy treated with the method of the present invention is reduced by at least 80%, compared with the placebo group (i.e., subjects with ischemic optic neuropathy not treated with the method of the present invention), while the one-component control group (i.e., subjects with ischemic optic neuropathy administered with only one pharmaceutical component), the two-component control group (i.e., subjects with ischemic optic neuropathy administered with only two components of DOX+MTP) and the three-component control group (i.e., subjects with ischemic optic neuropathy administered with a three-component drug DOX+MTP+BRM different from the present invention) only reduce the reduction in the average thickness of the retinal nerve fiber layer (RNFL) by about 30%, or even increase the reduction instead of reducing the reduction.

In certain embodiments, the method for treating, preventing, or improving ischemic optic neuropathy mitigating optic nerve function decline includes improving functional indexes of full-field electroretinogram (FERG) and flash visual evoked potential (FVEP), and protecting overall retinal function. Advantageously, amplitudes of the a-wave and b-wave of the light-adapted 3.0 ERG response of the full-field electroretinogram (FERG) of subjects with ischemic optic neuropathy treated with the method of the present invention are increased by about 30%, or 50%, or 100%, or 150%, or even close to 200% compared with the control group (i.e., subjects with ischemic optic neuropathy not treated with the method of the present invention). More advantageously, the N2-P2 amplitude of the flash visual evoked potential (FVEP) of subjects with ischemic optic neuropathy treated with the method of the present invention is increased by about 100%, or 200%, or 300%, or 400%, or even close to 500% compared with the control group (i.e., subjects with ischemic optic neuropathy not treated with the method of the present invention).

### 1.4.3 Optic neuritis, optic nerve tumor, and traumatic optic neuropathy

In certain embodiments, the optic neuropathy is optic neuritis. Optic neuritis generally refers to various inflammatory lesions involving optic nerve. In certain embodiments, the optic neuropathy is optic nerve tumor. Optic nerve tumors are tumors occurring in the optic nerve, and the two common types are optic glioma and optic meningioma. In certain embodiments, the optic neuropathy is traumatic optic neuropathy. Traumatic optic neuropathy refers to impact injury of optic nerve by an external force, which may lead to partial or total loss of vision.

Advantageously, the inventors of the present application have found that the method has beneficial effects in preventing, treating, and/or improving optic neuritis, optic nerve tumors, and/or traumatic optic neuropathy. Particularly advantageously, the method for preventing, treating and/or improving optic neuritis, optic nerve tumor and/or traumatic optic neuropathy of the present invention inhibits or alleviates retinal ganglion cell (RGC) injury or severe axonal injury in a subject; significantly inhibits the overexpression of glial fibrillary acidic protein (GFAP, neuroinflammatory marker), wherein GFAP may be induced by I/R; and inhibits the expression of inflammatory factors such as IFN-γ, IL-1β, and/or TNF-α that are associated with nerve cell injury.

In certain embodiments, the method for treating, preventing, or improving optic neuritis, optic nerve tumor, and/or traumatic optic neuropathy inhibits or mitigates retinal ganglion cell (RGC) injury or severe axonal injury in a subject. Advantageously, the average loss rate of the number of retinal ganglion cells in subjects with optic neuritis, optic nerve tumor and/or traumatic optic neuropathy treated with the method of the present invention is reduced by at least 80% compared with the placebo group (i.e., subjects with optic neuritis, optic nerve tumor, and/or traumatic optic neuropathy not treated with the method of the present invention), while the one-component control group (i.e., subjects with optic neuritis, optic nerve tumor, and/or traumatic optic neuropathy administered with only one pharmaceutical component), the two-component control group (i.e., subjects with optic neuritis, optic nerve tumor and/or traumatic optic neuropathy administered with only two components of DOX+MTP) and the three-component control group (i.e., subjects with optic neuritis, optic nerve tumor and/or traumatic optic neuropathy administered with a three-component drug DOX+MTP+BRM different from the present invention) only reduce the average loss rate of the number of retinal ganglion cells by 20%, or even only 15%.

In certain embodiments, the method for treating, preventing, or improving optic neuritis, optic nerve tumor and/or traumatic optic neuropathy inhibits overexpression of glial fibrillary acidic protein (GFAP), wherein GFAP may be I/R-induced. Without being bound by theory, the inventors found that the expression of GFAP in retinal tissue of subjects with optic neuritis, optic nerve tumor and/or traumatic optic neuropathy increased by about 5 times of normal expression (i.e., the expression of GFAP in retinal tissue of subjects without optic neuritis, optic nerve tumor, and/or traumatic optic neuropathy), which is considered as overexpression. Advantageously, the expression of GFAP in retinal tissue of subjects with optic neuritis, optic nerve tumor, and/or traumatic optic neuropathy treated with the method of the present invention is reduced to 50% or less, 40% or less, or even close to 30% of the overexpression.

In certain embodiments, the method for treating, preventing, or improving optic neuritis, optic nerve tumor, and/or traumatic optic neuropathy inhibits an increase of the expression level of at least one inflammatory factor. Inflammatory factor mainly refers to various cytokines involved in inflammatory reaction. In certain embodiments, the inflammatory factors include IFN-γ, IL-1β, and/or TNF-α. Without being bound by theory, the inventors found that the expression level of inflammatory factors in retinal tissue of subjects with optic neuritis, optic nerve tumor and/or traumatic optic neuropathy was significantly increased compared with normal levels (i.e., the expression level of inflammatory factors in retinal tissue of subjects without optic neuritis, optic nerve tumor, and/or traumatic optic neuropathy). Advantageously, the expression level of inflammatory factors in retinal tissue of subjects with optic neuritis, optic nerve tumor and/or traumatic optic neuropathy treated with the method of the present invention is reduced to be close to normal levels.

### 1.5 Retinopathy

In certain embodiments, the central nervous system disease, disorder, or condition includes retinopathy. Retinopathy is a disease occurring on retina. Retina is the initial site of vision formation and the pathological site of many blinding eye diseases. The present invention provides a method for treating, preventing, or improving retinopathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

In certain embodiments, the method for preventing, treating, and/or improving retinopathy involves inhibiting or mitigating retinal ganglion cell (RGC) injury or severe axonal injury in a subject. In certain embodiments, the method treats, prevents, or improves a disease, disorder, or condition associated with retinal ganglion cell (RGC) injury or severe axonal injury. Diseases associated with retinal ganglion cell (RGC) injury or severe axonal injury include: glaucoma, ischemic optic neuropathy, optic neuritis, optic nerve tumor, traumatic optic neuropathy, retinal artery occlusion, retinal vein occlusion, and retinopathy of prematurity.

### 1.5.1 Retinal vascular occlusion disease

In certain embodiments, the retinopathy includes retinal vascular occlusion disease. The present invention provides a method for treating, preventing, or improving retinal vascular occlusion disease, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

Retinal vascular occlusion is divided into retinal artery or vein occlusion, which may cause the condition of high retinal ischemia or hemorrhage, and is a common fundus vascular disease. Occlusion in retinal artery or vein may cause the condition of high degree of ischemia, edema, or hemorrhage of retina. The main symptoms are decreased central vision or deficit of a certain part of visual field. It has complex causes, and is closely related to retinal inflammation, retinal hypoperfusion, hypertension, arteriosclerosis, high blood viscosity, abnormal hemodynamics, etc.

Without theoretical constraints, retinal ischemia/reperfusion injury is accompanied by glial activation, neuroinflammation, and cytokine cascade reaction, leads to neurodegeneration and retinal cell death, and is considered as the main early factors of neuronal death. Astrocytes are the main glial cells in normal retina, which play an important role in maintaining the blood-retinal barrier and RGC health. The increased expression of glial fibrillary acidic protein (GFAP), a biomarker of astrocytes, is regarded as a sign of neuroinflammation and a marker of retinal degeneration. At the same time, the increased expression of various pro-inflammatory cytokines such as IFN-γ, IL-1β, and/or TNF-α aggravates I/R injury. Without theoretical constraints, targeted inhibition of GFAP proliferation and RGC apoptosis caused by I/R injury, inhibition of increase of various pro-inflammatory cytokines and improvement of retinal degeneration after I/R play a very important role in disease treatment.

In certain embodiments, the retinal vascular occlusion disease includes retinal vein occlusion. Retinal vein occlusion (RVO) is caused by interruption of venous reflux in normal retinal tissue, and is a common retinal vascular disease which is second only to diabetic retinopathy. The affected eye is susceptible to impaired vision and even blindness due to complications. According to the location of vein occlusion, it can be divided into central retinal vein occlusion, hemi-central retinal vein occlusion, and branch retinal vein occlusion. The occlusion part of central retinal vein occlusion is mostly located in the main trunk of central retinal vein in the lamina cribrosa area or posterior lamina cribrosa of optic disc, which is characterized by high degree retinal vein expansion and extensive hemorrhage. Branch retinal vein occlusion is more common in patients with arteriosclerosis. Occlusion is common in temporal branches, especially superior temporal branch. The arteries at the site of occlusion are mostly located in front of veins. It occurs at the intersection of arteries and veins from the first branch to the third branch of veins.

Retinal vein occlusion is a retinal vascular disease that leads to severe vision loss. Its causes include atherosclerosis of the central retinal artery resulting in compression of the central retinal vein at or behind the lamina cribrosa of the optic disc, venous blood flow stasis, and venous vascular endothelial injury leading to thrombosis. Depending on the location of occlusion, it can be divided into central retinal vein occlusion (CRVO) and branch retinal vein occlusion (BRVO). Most patients show massive fundus hemorrhage, venous tortuosity, cotton wool spots, macular edema and optic disc edema. At the same time, patients with central retinal vein occlusion may develop neovascular glaucoma (NVG), which leads to complete blindness.

In certain embodiments, the retinal vascular occlusion disease includes retinal artery occlusion. Retinal artery occlusion is a fundus disease with acute attack and serious damage to vision. Depending on the source and grade of blood vessels involved, it can be divided into central retinal artery occlusion, branch retinal artery occlusion, retinal cilioretinal artery occlusion, and retinal precapillary arteriolar occlusion. Central retinal artery occlusion refers to an acute ophthalmic disease in which the central retinal artery is partially or completely occluded, resulting in sudden blindness or sudden visual loss in patients. Branch retinal artery occlusion refers to a disease in which occlusion occurs in branch retinal artery. It is more common in temporal side, especially in superior temporal branch artery occlusion. The prognoses of vision and fundus changes depend on the location and degree of arterial occlusion. Cilioretinal artery occlusion is a rare critical illness in ophthalmology. If it is not rescued in time, it will seriously threaten vision and even leads to blindness. Cilioretinal artery is composed of short posterior ciliary artery, long posterior ciliary artery and anterior ciliary artery, which serves to nourish the choroid, the outer layer of the retina, and the whole eyeball except part of the optic nerve. Retinal precapillary arteriolar occlusion is manifested by small patches of grayish white beads, i.e., cotton wool spots, and occurs in the presence of systemic diseases such as diabetes, hypertension, and arteriosclerosis.

Advantageously, the inventors of the present application have found that the method has beneficial effects in preventing, treating, and/or improving retinal vascular occlusion diseases, in particular retinal artery occlusion diseases and retinal vein occlusion diseases. Particularly advantageously, the method for preventing, treating, and/or improving retinal vascular occlusion diseases of the present invention mitigates retinal ganglion cell (RGC) loss, protects visual function, and inhibits or reduces severity of secondary neuronal injury; mitigates internal retinal injury, reduces retinal edema and mitigates retinal thickness reduction; reduces edema of optic nerve fiber layer, maintains thickness of retinal nerve fiber layer and prevents thinning of optic nerve fiber layer; significantly inhibits optic nerve inflammation and GFAP overexpression, inhibits an expression level of inflammatory factors such as IFN-γ, IL-1β, and/or TNF-α that are associated with nerve cell injury; and mitigates optic nerve function decline, including improving functional indexes of full-field electroretinogram (FERG) and flash visual evoked potential (FVEP), and protecting overall retinal function.

In certain embodiments, the method for treating, preventing, or improving retinal vascular occlusion diseases mitigates retinal ganglion cell (RGC) loss, protects visual function, and inhibits or reduces the severity of secondary neuronal injury. Advantageously, the average loss rate of the number of retinal ganglion cells in subjects with retinal vascular occlusion diseases treated with the method of the present invention is reduced by at least 80% compared with the placebo group (i.e., subjects with retinal vascular occlusion diseases not treated with the method of the present invention), while the one-component control group (i.e., subjects with retinal vascular occlusion diseases administered with only one pharmaceutical component), the two-component control group (i.e., subjects with retinal vascular occlusion diseases administered with only two components of DOX+MTP) and the three-component control group (i.e., subjects with retinal vascular occlusion diseases administered with a three-component drug DOX+MTP+BRM different from the present invention) only reduce the average loss rate of the number of retinal ganglion cells by 20%, or even only 15%.

In certain embodiments, the method for treating, preventing, or improving retinal vascular occlusion diseases significantly inhibits optic neuroinflammation and GFAP overexpression. Without being bound by theory, the inventors found that the expression of GFAP in retinal tissue of subjects with retinal vascular occlusion diseases increased by about 5 times of normal expression (i.e., the expression of GFAP in retinal tissue of subjects without retinal vascular occlusion disease), which is considered as overexpression. Advantageously, the expression of GFAP in retinal tissue of subj ects with retinal vascular occlusion diseases treated with the method of the present invention is reduced to 50% or less, 40% or less, or even close to 30% of the overexpression.

In certain embodiments, the method for treating, preventing, or improving retinal vascular occlusion diseases inhibits an increased level of expression of at least one inflammatory factor, wherein the inflammatory factor includes IFN-γ, IL-1β, and/or TNF-α. Without being bound by theory, the inventors found that the expression level of inflammatory factors in retinal tissue of subjects with retinal vascular occlusion diseases was significantly increased compared with normal levels (i.e., the expression level of inflammatory factors in retinal tissue of subjects without retinal vascular occlusion disease). Advantageously, the expression level of inflammatory factors in retinal tissue of subjects with retinal vascular occlusion diseases treated with the method of the present invention is reduced to be close to normal levels.

In certain embodiments, the method for treating, preventing, or improving retinal vascular occlusion diseases mitigates internal retinal injury, reduces retinal edema, and mitigates retinal thickness reduction. Advantageously, the reduction in the average thickness of the retinal EDTRS area in subjects with retinal vascular occlusion diseases treated with the method of the present invention is reduced by at least 70%, or even by at least 80%, compared with the placebo group (i.e., subjects with retinal vascular occlusion diseases not treated with the method of the present invention), while the one-component control group (i.e., subjects with retinal vascular occlusion diseases administered with only one pharmaceutical component), the two-component control group (i.e., subjects with retinal vascular occlusion diseases administered with only two components of DOX+MTP) and the three-component control group (i.e., subjects with retinal vascular occlusion diseases administered with a three-component drug DOX+MTP+BRM different from the present invention) only reduce the reduction in the average thickness of the retinal EDTRS area by about 30%, or 20%, or 10%, or 5%, or even increase the reduction instead of reducing the reduction. More advantageously, the reduction in the average thickness of the retinal EDTRS area of subjects without retinal vascular occlusion diseases treated with the method of the present invention is reduced by at least 30%, at least 40%, at least 50%, at least 60%, or even close to 70% compared to the control group (i.e., subjects without retinal vascular occlusion diseases not treated with the method of the present invention).

In certain embodiments, the method for treating, preventing, or improving retinal vascular occlusion diseases reduces retinal nerve fiber layer edema, maintains retinal nerve fiber layer thickness, and prevents thinning of the optic nerve fiber layer. Advantageously, the reduction in the average thickness of the retinal nerve fiber layer (RNFL) in subjects with retinal vascular occlusion diseases treated with the method of the present invention is reduced by at least 80%, compared with the placebo group (i.e., subjects with retinal vascular occlusion diseases not treated with the method of the present invention), while the one-component control group (i.e., subjects with retinal vascular occlusion diseases administered with only one pharmaceutical component), the two-component control group (i.e., subjects with retinal vascular occlusion diseases administered with only two components of DOX+MTP) and the three-component control group (i.e., subjects with retinal vascular occlusion diseases administered with a three-component drug DOX+MTP+BRM different from the present invention) only reduce the reduction in the average thickness of the retinal nerve fiber layer (RNFL) by about 30%, or even increase the reduction instead of reducing the reduction.

In certain embodiments, the method for treating, preventing, or improving retinal vascular occlusion diseases mitigating optic nerve function decline includes improving functional indexes of full-field electroretinogram (FERG) and flash visual evoked potential (FVEP), and protecting overall retinal function. Advantageously, amplitudes of the a-wave and b-wave of the light-adapted 3.0 ERG response of the full-field electroretinogram (FERG) of subjects with retinal vascular occlusion diseases treated with the method of the present invention are increased by about 30%, or 50%, or 100%, or 150%, or even close to 200% compared with the control group (i.e., subjects with retinal vascular occlusion diseases not treated with the method of the present invention). More advantageously, the N2-P2 amplitude of the flash visual evoked potential (FVEP) of subjects with retinal vascular occlusion diseases treated with the method of the present invention is increased by about 100%, or 200%, or 300%, or 400%, or even close to 500% compared with the control group (i.e., subjects with retinal vascular occlusion diseases not treated with the method of the present invention).

### 1.5.2 Retinopathy of prematurity

In certain embodiments, the retinopathy includes retinopathy of prematurity. Most children with retinopathy of prematurity (ROP) are premature infants with gestational age below 32 weeks and a birth weight of less than 1500 g and with a history of inhaling high concentration of oxygen, or low-birth-weight infants with growth retardation. ROP is an important cause of blindness in infants, and it is also one of the important eye diseases leading to white pupil sign. Preterm birth with low birth weight and inhalation of high concentration oxygen are known pathogenic factors, and it is caused by vasoconstriction and vascular proliferation in response to oxygen in the incompletely vascularized retina.

Advantageously, the inventors of the present application have found that the method has beneficial effects in preventing, treating, and/or improving retinopathy of prematurity. Particularly advantageously, the method for preventing, treating, and/or improving retinopathy of prematurity of the present invention mitigates retinal ganglion cell (RGC) loss, protects visual function, and inhibits or reduces severity of secondary neuronal injury; mitigates internal retinal injury, reduces retinal edema, and mitigates retinal thickness reduction; reduces edema of optic nerve fiber layer, maintains thickness of retinal nerve fiber layer and prevents thinning of optic nerve fiber layer; significantly inhibits optic nerve inflammation and GFAP overexpression, inhibits the expression level of inflammatory factors such as IFN-γ, IL-1β, and/or TNF-α that are associated with nerve cell injury; and mitigates optic nerve function decline, including improving functional indexes of full-field electroretinogram (FERG) and flash visual evoked potential (FVEP), and protecting overall retinal function.

In certain embodiments, the method for treating, preventing, or improving retinopathy of prematurity mitigates retinal ganglion cell (RGC) loss, protects visual function, and inhibits or reduces the severity of secondary neuronal injury. Advantageously, the average loss rate of the number of retinal ganglion cells in subjects with treated with the method of the present invention is reduced by at least 80% compared with the placebo group (i.e., subjects with retinopathy of prematurity not treated with the method of the present invention), while the one-component control group (i.e., subjects with retinopathy of prematurity administered with only one pharmaceutical component), the two-component control group (i.e., subjects with retinopathy of prematurity administered with only two components of DOX+MTP) and the three-component control group (i.e., subjects with retinopathy of prematurity administered with a three-component drug DOX+MTP+BRM different from the present invention) only reduce the average loss rate of the number of retinal ganglion cells by 20%, or even only 15%.

In certain embodiments, the method for treating, preventing, or improving retinopathy of prematurity significantly inhibits optic neuroinflammation and GFAP overexpression. Without being bound by theory, the inventors found that the expression of GFAP in retinal tissue of subjects with retinopathy of prematurity increased by about 5 times of normal expression (i.e., the expression of GFAP in retinal tissue of subjects without retinopathy of prematurity), which is considered as overexpression. Advantageously, the expression of GFAP in retinal tissue of subjects with retinopathy of prematurity treated with the method of the present invention is reduced to 50% or less, 40% or less, or even close to 30% of the overexpression.

In certain embodiments, the method for treating, preventing or improving retinopathy of prematurity inhibits an increased level of expression of at least one inflammatory factor, wherein the inflammatory factor includes IFN-γ, IL-1β, and/or TNF-α. Without being bound by theory, the inventors found that the expression level of inflammatory factors in retinal tissue of subjects with retinopathy of prematurity was significantly increased compared with normal levels (i.e., the expression level of inflammatory factors in retinal tissue of subjects without retinopathy of prematurity). Advantageously, the expression level of inflammatory factors in retinal tissue of subjects with retinopathy of prematurity treated with the method of the present invention is reduced to be close to normal levels.

In certain embodiments, the method for treating, preventing, or improving retinopathy of prematurity mitigates internal retinal injury, reduces retinal edema, and mitigates retinal thickness reduction. Advantageously, the reduction in the average thickness of the retinal EDTRS area in subjects with retinopathy of prematurity treated with the method of the present invention is reduced by at least 70%, or even by at least 80%, compared with the placebo group (i.e., subjects with retinopathy of prematurity not treated with the method of the present invention), while the one-component control group (i.e., subjects with retinopathy of prematurity administered with only one pharmaceutical component), the two-component control group (i.e., subjects with retinopathy of prematurity administered with only two components of DOX+MTP) and the three-component control group (i.e., subjects with retinopathy of prematurity administered with a three-component drug DOX+MTP+BRM different from the present invention) only reduce the reduction in the average thickness of the retinal EDTRS area by about 30%, or 20%, or 10%, or 5%, or even increase the reduction instead of reducing the reduction. More advantageously, the reduction in the average thickness of the retinal EDTRS area of subjects without retinopathy of prematurity treated with the method of the present invention is reduced by at least 30%, at least 40%, at least 50%, at least 60%, or even close to 70% compared to the control group (i.e., subjects without retinopathy of prematurity not treated with the method of the present invention).

In certain embodiments, the method for treating, preventing, or improving retinopathy of prematurity reduces retinal nerve fiber layer edema, maintains retinal nerve fiber layer thickness, and prevents thinning of the optic nerve fiber layer. Advantageously, the reduction in the average thickness of the retinal nerve fiber layer (RNFL) in subjects with retinopathy of prematurity treated with the method of the present invention is reduced by at least 80%, compared with the placebo group (i.e., subjects with retinopathy of prematurity not treated with the method of the present invention), while the one-component control group (i.e., subjects with retinopathy of prematurity administered with only one pharmaceutical component), the two-component control group (i.e., subjects with retinopathy of prematurity administered with only two components of DOX+MTP) and the three-component control group (i.e., subjects with retinopathy of prematurity administered with a three-component drug DOX+MTP+BRM different from the present invention) only reduce the reduction in the average thickness of the retinal nerve fiber layer (RNFL) by about 30%, or even increase the reduction instead of reducing the reduction.

In certain embodiments, the method for treating, preventing, or improving retinopathy of prematurity mitigating optic nerve function decline includes improving functional indexes of full-field electroretinogram (FERG) and flash visual evoked potential (FVEP), and protecting overall retinal function. Advantageously, amplitudes of the a-wave and b-wave of the light-adapted 3.0 ERG response of the full-field electroretinogram (FERG) of subjects with retinopathy of prematurity treated with the method of the present invention are increased by about 30%, or 50%, or 100%, or 150%, or even close to 200% compared with the control group (i.e., subjects with retinopathy of prematurity not treated with the method of the present invention). More advantageously, the N2-P2 amplitude of the flash visual evoked potential (FVEP) of subjects with retinopathy of prematurity treated with the method of the present invention is increased by about 100%, or 200%, or 300%, or 400%, or even close to 500% compared with the control group (i.e., subjects with retinopathy of prematurity not treated with the method of the present invention).

### 1.6 Diabetic peripheral neuropathy

In certain embodiments, the peripheral neuropathy is Diabetic Peripheral Neuropathy (DPN). In certain embodiments, there is provided a method for treating, preventing, or improving diabetic peripheral neuropathy, including administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

In certain embodiments, diabetic peripheral neuropathy is diabetic distal symmetrical polyneuropathy (DSPN) or diabetic mononeuropathy or multiple mononeuropathy (Zakin E, Abrams R, Simpson DM. Diabetic Neuropathy. Semin Neurol. 2019 Oct; 39(5):560-569.). Patients with diabetic mononeuropathy or multiple mononeuropathy are often involved in median nerve, ulnar nerve and common peroneal nerve, often with insidious attack, but also with acute onset, mainly showing sensory and motor dysfunction in innervated area.

Diabetic distal symmetrical polyneuropathy (DSPN) is the most common type of DPN, accounting for about 50% of diabetic patients. The Toronto Diabetic Neuropathy Consensus Group defines DSPN as "a symmetrical, length-dependent sensorimotor polyneuropathy attributable to metabolic and microvascular alterations resulting from chronic hyperglycemic exposure and cardiovascular risk covariates". Patients with DSPN have two main clinical manifestations, i.e., insensitivity to trauma leading to foot ulcer and extremely painful neuropathic pain. In certain embodiments, DSPN patients exhibit symmetrical symptoms, and symptomatic manifestations in the patients include distal limb sensory abnormalities, both lower limb sensory abnormalities, stocking anesthesia, glove anesthesia, median nerve, ulnar nerve, common peroneal nerve involvement, sensory dysfunction, motor dysfunction, insensitivity to trauma, and neuropathic pain.

Electromyography (EMG) is widely used to evaluate various types of nerve dysfunction in patients with diabetic peripheral neuropathy, and EMG may provide an objective and quantitative diagnosis of nerve function. Neuroelectrophysiological studies have proved that sensory nerve conduction slows in patients with diabetes for 5-10 years, i.e., these diabetic patients are accompanied by diabetic peripheral neuropathy. In patients with diabetic peripheral neuropathy, the degree and rate of sensory nerve conduction velocity (SCV) abnormalities are usually more severe than motor nerve conduction velocity (MCV) abnormalities, and the patients' neurological abnormalities are more severe in the lower limbs than in the upper limbs (Neurocomplications Group of Chinese Diabetes Society, Expert consensus on diagnosis and treatment of diabetic neuropathy (2021 Edition). Chinese Journal of Diabetes, 2021, 13(06):540-557).

Advantageously, the inventors of the present application have found that the method has beneficial effects in preventing, treating, and/or improving peripheral neuropathy, in particular diabetic peripheral neuropathy. In certain embodiments, the method improves peripheral nerve conduction velocity, significantly improves the sensory nerve conduction velocity of the sural nerve; improves microcirculation, inhibits neuroinflammation, inhibits central nervous inflammation, repairs damaged nerve tissue, relieves symptoms of peripheral neuropathy for a long time, controls the progression of neuropathy for a long time, and controls the progression of neuropathic pain for a long time.

Particularly advantageously, the method of preventing, treating, and/or improving diabetic peripheral neuropathy of the present invention improves the peripheral nerve conduction velocity, in particular significantly improves the sensory nerve conduction velocity of the sural nerve. After drug administration, the sensory nerve conduction velocity of DSPN subjects treated with the method of the present invention is improved by at least 4.3 m/s or even 4.6 m/s on average. In contrast, the sensory nerve conduction velocity of the placebo group (i.e., DSPN subjects not treated with the method of the present invention), the one-component control group (i.e., subjects administered with only one pharmaceutical component), and the two-component control group (i.e., subjects administered with only two components of DOX+MTP or with only two components of DOX+PMP) does not change significantly before and after drug administration, and even decreases after drug administration. Advantageously, in the abnormal sural sensory nerves of DSPN subjects, up to 75% of the nerves are effectively improved after treatment with the method of the present invention, and the effective improvement rate is higher than that of the positive drug control group.

Advantageously, the method of preventing, treating, and/or improving diabetic peripheral neuropathy of the present invention can improve microcirculation, inhibit the reduction of regional cerebral blood volume (rCBV) around cerebral tissue infarction, improve cerebral tissue perfusion of the subjects, improve cerebral edema and brain tissue lesions, and reduce brain tissue necrosis. Without theoretical constraints, this method improves microcirculation in patients with diabetic peripheral neuropathy by inhibiting the reduction of cerebral volume in the patients, relieves ischemia and hypoxia of neural tissues and injuries to neural structure and function caused by microcirculatory disorders. The results of DSC-PWI sequence of MRI scan may be used to judge the cerebral microcirculation. Among them, cerebral blood flow (CBF) refers to the flow of blood through a given amount of brain tissue vascular structures per unit of time, and the smaller the value, the lower the cerebral blood flow; cerebral blood volume (CBV) refers to the volume of blood present within the vascular structures of a certain amount of brain tissue, and the smaller the value, the less the blood volume of brain tissue; and mean transit time (MTT) refers to the average time for a contrast agent to pass through brain tissue, and time to peak (TTP) refers to the time for a contrast agent to reach its peak in brain area. When there is a significant decrease in relative cerebral blood flow (rCBF), a significant decrease in relative cerebral blood volume (rCBV), a prolongation of relative mean transit time (rMTT), and a prolongation of relative time to peak (rTTP), it is indicative of microcirculatory disorders in the patient and inadequate perfusion. Recovery or increase of relative cerebral blood volume (rCBV) indicates improvement in the microcirculation of patients. DSC-PWI results can provide sensitive and exact brain microcirculation information for clinical patients (LI Yongli, YAN Fengshan, DOU Shewei, et al., Clinical comparative application of arterial proton spin labeling and dynamic magnetic susceptibility contrast-enhanced perfusion imaging in the evaluation of cerebral microcirculation in patients with cerebral ischemia, Journal of Zhengzhou University: Medical Edition, 2013 (4): 4; JING Yanping, LUO Bin, GAO Zhengrong, et al., Application value of DSC-PWI in ischemic cerebrovascular diseases, Journal of Chinese Physician, 2020, 22(3): 6). Advantageously, the volume of the non-perfusion area (i.e., the ischemic core area) of the brain of subjects with microcirculatory disorder treated with the method of the present invention is reduced by at least 70% compared with subjects with microcirculatory disorder not treated with the method.

Advantageously, the method for preventing, treating, and/or improving diabetic peripheral neuropathy of the present invention can also inhibit neuroinflammation, in particular central nervous system inflammation, manifested as inhibiting overexpression of GFAP protein in brain tissue. The inventors found that the expression of GFAP in the tissue surrounding cerebral infarction of subjects with cerebral microcirculatory disorder increased by about 3-4 times of the normal expression level (i.e., the expression of GFAP in the brain tissue of subjects without microcirculatory disorder), which is considered as overexpression. Without theoretical constraints, this method inhibits neuroinflammation by inhibiting the overexpression of GFAP in brain tissue, inhibits the increase of oxidative stress level caused by abnormal glucose metabolism, repairs inflammation and injuries to vascular endothelium and blood-brain barrier, and relieves diabetic neuropathy. Advantageously, the GFAP expression in the brain tissue of subjects with brain microcirculatory disorder treated with the method of the present invention is reduced to 50% or less, or even 40% or less of overexpression.

Advantageously, the method for preventing, treating, and/or improving diabetic peripheral neuropathy of the present invention can also repair injured nerve tissue. Particularly advantageously, the method of the present invention can reduce the edema of the optic nerve fiber layer, maintain the thickness of the retinal nerve fiber layer and prevent thinning of the optic nerve fiber layer. Without theoretical constraints, this method mitigates edema, degeneration and necrosis of nerve fibers by maintaining the thickness of the optic nerve fiber layer, maintains the structure and function of nerve cells, protects injured neurons and repairs injured nerve tissue. The reduction in the average thickness of the retinal nerve fiber layer (RNFL) in subjects treated with the method of the present invention can be reduced by at least 80%, compared with the placebo group (i.e., subjects not treated with the method of the present invention), while the one-component control group (i.e., glaucoma subjects with ischemic optic neuropathy administered with only one pharmaceutical component), the two-component control group (i.e., glaucoma subjects with ischemic optic neuropathy administered with only two components of DOX+MTP) and the three-component control group (i.e., glaucoma subjects with ischemic optic neuropathy administered with a three-component drug DOX+MTP+BRM different from the present invention) can only reduce the reduction in the average thickness of the retinal nerve fiber layer (RNFL) by about 30%, or even increase the reduction instead of reducing the reduction.

Advantageously, the method for preventing, treating and/or improving diabetic peripheral neuropathy of the present invention relieves the symptoms of peripheral neuropathy for a long term, controls the progression of neuropathy for a long term, and shows good safety during drug administration. Without being bound by theory, the method for preventing, treating and/or improving diabetic peripheral neuropathy of the present invention relieves and controls the progression of peripheral neuropathy for a long time by improving cerebral microcirculation, inhibiting central nervous inflammation and repairing injured nerve tissue. Advantageously, the method of the present invention does not cause elevation of liver enzyme level and gastrointestinal related toxic and side effects, and can have the effect of relieving and controlling the progress of neuropathy for a long time.

In certain embodiments, diabetic peripheral neuropathy is diabetic peripheral neuropathic pain (DPNP). About 20% of DSPN patients will have neuropathic pain (painful-DSPN), i.e., DPNP, which is called painful DSPN patients. The most common neurological symptoms in these patients are burning sensation, "electric shock" type pain and severe pain. In certain embodiments, the patient's sleep is affected by pain and suffers from pain-induced depression and anxiety. More than half of patients rate their pain as "severe", and patients often report that their symptoms are worse at night. It is predicted that as many as three quarters of patients will suffer from sleep interference, and patients with diabetic painful peripheral neuropathy are also more likely to have symptoms of depression and anxiety.

Advantageously, the method for preventing, treating, and/or improving diabetic peripheral neuropathy of the present invention can control the progression of neuropathic pain for a long term and relieve neuropathic pain symptoms. Without being bound by theory, the method for preventing, treating, and/or improving diabetic peripheral neuropathy of the present invention protects against both central and peripheral neuropathy by improving brain microcirculation, inhibiting central nervous inflammation and repairing injured nerve tissue, and can treat DPNP and relieve neuropathic pain of the subjects based on the above mechanisms. Without theoretical constraints, this method can inhibit the overexpression of GFAP protein, inhibit neuroinflammation, repair injured nerve cells, and relieve neuropathic pain and other symptoms of patients. Without theoretical constraints, the protection of the structure and function of peripheral central nervous system and central nervous system (CNS) suggests the relief of pain symptoms in DPNP patients.

### 2. Pharmaceutical composition

In certain embodiments, the present invention relates to a pharmaceutical composition for modulating neuropathy, including a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

The term "pharmaceutical composition" refers to a combination of a variety of substances having a particular pharmaceutical or biological use, typically expected to have a therapeutic or preventive effect on a particular disease after administration to a subject. Pharmaceutical composition may either contain only specified active ingredients (biologically active substances) or may be provided with conventional pharmaceutically acceptable carriers for various purposes. The term "composition" in the present application should be interpreted according to its broad meaning. As one implementation of the pharmaceutical composition of the present invention, for example, it may be provided, for example, in the form of an indistinguishable mixture of individual components by mixing together specified active ingredients (and optionally pharmaceutically acceptable carriers). As another implementation of the pharmaceutical composition of the present invention, the "pharmaceutical composition" of the present invention may also be provided by individually packaging each prescribed active ingredient into small portions and then combining and containing these small independent packages together in a larger container.

In certain embodiments, the therapeutically effective amount of doxazosin, a pharmaceutically acceptable salt or an acceptable form thereof, the therapeutically effective amount of pramipexole, a pharmaceutically acceptable salt or an acceptable form thereof, and the therapeutically effective amount of metoprolol, a pharmaceutically acceptable salt or an acceptable form thereof are formulated into a single pharmaceutical composition.

The terms "active ingredient" and "biologically active substance" refer to molecules and other agents that function biologically, physiologically, or pharmaceutically, acting in a subject for the treatment of diseases or conditions (e.g., diabetic retinopathy). The terms are used in relation to terms such as "pharmaceutically acceptable carrier", "excipient", "additive", etc. "Active ingredients" (or "biologically active substances") include, but are not limited to, pharmaceutically acceptable salts and prodrugs thereof. These reagents may be acids, bases or salts; they may be neutral molecules, polar molecules or molecular complexes capable of hydrogen bonding; and they may be prodrugs in the form of ethers, esters, amides, etc., which are bioactivated when given to a subject.

The term "acceptable form thereof' includes, but is not limited to, pharmaceutically acceptable hydrates, solvates, isotopically labeled compounds, optical isomers, geometric isomers, tautomers, mixtures of isomers, and/or prodrugs of an active ingredient (e.g., doxazosin molecules). The solvates include hydrates, ethanol solvates, acetone solvates, etc.

The "acceptable form thereof' of a certain active ingredient of the present invention further includes pharmaceutically acceptable salts formed from an isotope labeled compound of the active ingredient, or an optical isomer, a geometric isomer, a tautomer isomer or a mixture of isomers thereof, or a prodrug thereof.

It should be understood that all references to various active ingredients or pharmaceutically acceptable salts include various crystalline forms (e.g., amorphous, polymorphic substances, etc.) of the same active ingredient or salt.

The term "doxazosin" used herein refers to the following molecule with the English name Doxazosin (abbreviated as DOX) and the IUPAC name (RS)-2-[4-(2,3-Dihydro-1,4-benzodioxine-2-carbonyl)piperazin-1-yl]-6,7-dimethoxyquinazolin-4-amine (molecular formula: C₂₃H₂₅N₅O₅, molecular weight: 451.475 g/mol):

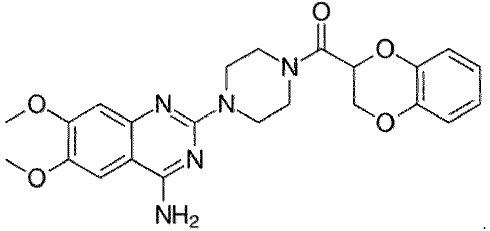

The present invention also includes isotope labeled compounds of doxazosin, or optical isomers, geometric isomers, tautomers, or mixtures of isomers thereof, or prodrugs thereof (i.e., compounds from which the above molecule is obtained by in vivo reactions).

Doxazosin is a selective α1 receptor antagonist, which inhibits the binding of norepinephrine (released from sympathetic nerve endings) to α1 receptor on vascular smooth muscle cell membrane, and is often used to treat essential hypertension.

The pharmaceutical composition of the present invention may contain doxazosin or its pharmaceutically acceptable salt as active ingredients. In certain embodiments, the salt form of doxazosin is methanesulfonate.

The term "pramipexole" used herein refers to the following molecule with the English name Pramipexole (abbreviated as PMP) and the IUPAC name (S)-N6-propyl-4,5,6,7-tetrahydro-1,3-benzothiazole-2,6-diamine (molecular formula: C₁₀H₁₇N₃S, molecular weight: 211.324 g/mol):

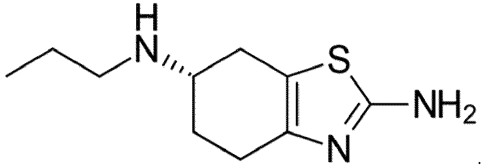

The present invention also includes isotope labeled compounds of pramipexole, or optical isomers, geometric isomers, tautomers or mixtures of isomers thereof, or prodrugs thereof (i.e., compounds from which the above molecule is obtained by in vivo reactions).

Pramipexole is an antihistamine that acts as a dopamine receptor D2/D3 agonist and is mainly clinically used for the treatment of Parkinson's disease, alone (without levodopa) or in combination with levodopa. Pramipexole is sometimes called "Mirapex", "Mirapexin", "Sifrol", etc.

The pharmaceutical composition of the present invention may contain pramipexole or its pharmaceutically acceptable salt as active ingredients. In certain embodiments, the salt form of pramipexole is a hydrochloride. In certain embodiments, the present invention includes dihydrochloride of pramipexole. In certain embodiments, the present invention includes the form of pramipexole hydrate. In certain embodiments, the present invention includes a monohydrate of pramipexole.

The term "metoprolol' used herein refers to the following molecule with the English name Metoprolol (abbreviated as MTP) and the IUPAC name (RS)-1-[4-(2-methoxyethyl)phenoxy]-3-[(prop-2-yl)amino]prop-2-ol (molecular formula: C₁₅H₂₅NO₃, molecular weight: 267.37 g/mol):

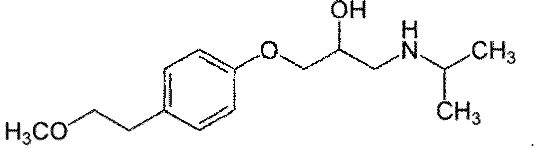

The present invention also includes isotope labeled compounds of metoprolol, or optical isomers, geometric isomers, tautomers or mixtures of isomers thereof, or prodrugs thereof (i.e., compounds from which the above molecule is obtained by in vivo reactions).

Metoprolol is a selective adrenergic β1 receptor blocker commonly used in the treatment of hypertension and angina pectoris. The pharmaceutical composition of the present invention may contain metoprolol or its pharmaceutically acceptable salt as active ingredients. In certain embodiments, the salt form of metoprolol is tartrate.

The active ingredients (doxazosin, pramipexole, and metoprolol) contained in the composition of the present invention may be replaced by their pharmaceutically acceptable salts. The term "pharmaceutically acceptable salt" of a compound means a salt that is pharmaceutically acceptable and has the desired pharmacological activity of the parent compound. Pharmaceutically acceptable salts used herein are salts formed with pharmaceutically acceptable acids or bases. Pharmaceutically acceptable acids include, but are not limited to, inorganic acids, such as hydrochloric acid, sulfuric acid, phosphoric acid, pyrophosphoric acid, hydrobromic acid, or nitric acid, and organic acids, such as citric acid, fumaric acid, maleic acid, malic acid, ascorbic acid, succinic acid, tartaric acid, benzoic acid, acetic acid, methanesulfonic acid, ethanesulfonic acid, salicylic acid, stearic acid, benzenesulfonic acid, or p-toluenesulfonic acid. Pharmaceutically acceptable bases include hydroxides of alkali metals (e.g., sodium or potassium) and alkaline earth metals (e.g., calcium or magnesium), as well as organic bases such as alkylamines, arylamines, or heterocyclic amines.

Pharmaceutically acceptable salts can be synthesized by conventional chemical methods from parent compounds containing basic or acidic moieties. Typically, these salts may be prepared by reacting the free acid or base forms of these compounds with a stoichiometric amount of suitable bases or acids in water or in an organic solvent or in a mixture of the two. Generally, a non-aqueous medium such as ether, ethyl acetate, ethanol, isopropanol, or acetonitrile is preferred. A list of salts may be found in Remington's Pharmaceutical Sciences, 18th edition (Mack Publishing Company, 1990). For example, the salts may include, but are not limited to, hydrochloride, tartrate, methanesulfonate, etc. of the compounds of the present invention.

The contents of doxazosin (or its pharmaceutically acceptable salt), pramipexole (or its pharmaceutically acceptable salt), and metoprolol (or its pharmaceutically acceptable salt) in the pharmaceutical composition of the invention may be adjusted according to actual needs. For example, the content or proportion of each drug in the pharmaceutical composition may be varied depending on the mode of administration (orally or injected, etc.) of the pharmaceutical composition.

In the pharmaceutical composition of the present application, if the weight of doxazosin or a pharmaceutically acceptable salt thereof is used as a reference (=1), the weight ratio of metoprolol or a pharmaceutically acceptable salt thereof to doxazosin or a pharmaceutically acceptable salt thereof is generally in the range of 0.3:1 to 400:1, and may be, for example, greater than or equal to 0.4:1, 0.5:1, 0.6:1, 0.7:1, 0.8:1, 0.9:1, 1:1, 1.5:1, 2:1, 3:1, 4:1, 5:1, 10:1, 12.5:1, or 15:1, preferably greater than or equal to 1:1, 1.5:1, 2:1, 3:1, 4:1, 5:1, 10:1, 12.5:1, or 15:1, and may be less than or equal to 350:1, 300:1, 250:1, 200:1, 150:1, 100:1, 90:1, 80:1, 70:1, 60:1, 50:1, 40:1, 30:1, or 20:1, preferably less than or equal to 100:1, 90:1, 80:1, 70:1, 60:1, 50:1, 40:1, 30:1, or 20:1; likewise, if the weight of doxazosin or a pharmaceutically acceptable salt thereof is used as a reference (=1), the weight ratio of pramipexole or a pharmaceutically acceptable salt thereof to doxazosin or a pharmaceutically acceptable salt thereof is generally in the range of 0.003:1 to 10:1, for example, may be greater than or equal to 0.004:1, 0.005:1, 0.006:1, 0.007:1, 0.008:1, 0.009:1, 0.01:1, 0.015:1, 0.02:1, 0.03:1, 0.04:1, 0.05:1, 0.06:1, 0.07:1, 0.08:1, 0.1:1, or 0.2:1, preferably greater than or equal to 0.005:1, 0.006:1, 0.007:1, 0.008:1, 0.009:1, 0.01:1, 0.015:1, 0.02:1, 0.03:1, 0.04:1, 0.05:1, 0.06:1, 0.07:1, 0.08:1, or 0.1:1, and may be less than or equal to 9:1, 6:1, 5:1, 4:1, 3:1, 2:1, 1:1, 0.9:1, 0.6:1, 0.5:1, 0.4:1, or 0.3:1, preferably less than or equal to 1:1, 0.9:1, 0.6:1, 0.5:1, or 0.4:1.

### 3. Pharmaceutical dosage form

In some embodiments, the pharmaceutical composition of the present invention may be provided in the form of an bulk drug (e.g., a homogeneous mixture or a separate package of each ingredient). In other embodiments, the pharmaceutical composition of the present invention may be formulated into various pharmaceutical dosage forms (formulations) by adding pharmaceutically acceptable carriers as desired. For this purpose, various liquid or solid fillers, diluents, excipients, solvents or encapsulating materials may be used as "pharmaceutically acceptable carriers". In the present application, the phrase "pharmaceutically acceptable" refers to compounds, compositions, polymers and other materials that, within the range of reasonable medical judgment, are compatible with other components of the composition herein and suitable for contact with human and animal tissues without excessive toxicity, irritation, allergic reactions, or other problems or complications. In certain preferred embodiments, the pharmaceutically acceptable carrier is pyrogen-free. Some examples of materials that may be used as pharmaceutically acceptable carriers include: (1) sugars, such as lactose, glucose, and sucrose; (2) starch, such as corn starch and potato starch; (3) cellulose and its derivatives, such as sodium carboxymethyl cellulose, ethyl cellulose, and cellulose acetate; (4) powdered tragacanth; (5) maltodextrin; (6) gelatin; (7) talc powder; (8) auxiliary materials, such as cocoa butter and suppository wax; (9) oils, such as peanut oil, cottonseed oil, sunflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminum hydroxide; (15) alginic acid; (16) non-pyrogenic water; (17) isotonic saline; (18) Ringer's solution; (19) ethanol; (20) phosphate buffer solution; and (21) other non-toxic compatible substances used in pharmaceutical preparations.

The pharmaceutical composition of the present invention may be formulated into dosage forms suitable for oral administration, parenteral administration (including subcutaneous, muscular, cortical, and intravenous administration), bronchial administration, nasal administration and the like as required. Preferably, the pharmaceutical composition of the present invention is formulated into a dosage form (preparation) suitable for oral administration.

If a solid carrier is used, the preparation may be in the form of tablets, in the form of powders or granules in hard gel capsules, or in the form of sugar lozenges or ingots. The solid carrier may include conventional excipients, such as adhesives, fillers, tablet lubricants, disintegrants, wetting agents and the like. If necessary, the tablets may be coated by conventional techniques. If a liquid carrier is used, the preparation may be in the form of syrup, emulsion, soft gel capsule, sterile carrier for injection, aqueous or non-aqueous liquid suspension, or may be a dry product restored with water or other suitable carrier before use. Liquid preparations may include conventional additives, such as suspending agents, emulsifiers, wetting agents, non-aqueous carriers (including edible oils), preservatives, and flavoring agents and/or colorants. For parenteral administration, the carrier usually includes at least most sterile water, but saline solution, glucose solution, etc. may also be used. An injectable suspension may also be used, and in such a case, a conventional suspending agent may be used. Conventional preservatives, buffer reagents, etc. may also be added to parenteral dosage forms.

Dosage forms suitable for parenteral injection may include physiologically acceptable sterile aqueous or non-aqueous solutions, dispersions, suspensions, or emulsions and sterile powders for sterile injectable solutions or dispersions. Examples of suitable aqueous and non-aqueous carriers, diluents and solvents include water, ethanol, polyols (propylene glycol, polyethylene glycol, glycerol, etc.), suitable mixtures thereof, vegetable oils (e.g., olive oil), and injectable organic esters (e.g., ethyl oleate).

These pharmaceutical dosage forms may also contain various excipients, such as preservatives, wetting agents, emulsifiers, and dispersants. Inhibition of the effect of microorganisms may be ensured by a variety of antimicrobial and antifungal agents (e.g., p-hydroxybenzoate, chlorobutanol, phenol, sorbic acid, etc.). Isotonic agents, such as sugar, sodium chloride, etc. may also be included. Absorption of injectable pharmaceutical dosage forms may be prolonged by using absorption delaying reagents (e.g., aluminum monostearate and gels).

The present invention also provides a pharmaceutical composition for administration to humans and animals in unit dosage forms, e.g., tablets, capsules, pills, powders, granules, sterile parenteral solutions or suspensions, as well as oral solutions or suspensions, and oil-water emulsions containing suitable amounts of compounds or pharmaceutically acceptable salts thereof. Pharmaceutically therapeutically active compounds and salts thereof are formulated and administered in unit dose forms or in multiple dose forms. Unit dose as used in the present invention refer to physically independent units suitable for human and animal subjects and packaged independently, which is known in the art. Each unit dose contains a predetermined number of therapeutically active compounds sufficient to produce desired therapeutic effect, associated with a desired drug carrier, carrier, or diluent. Examples of unit dose form include ampoules and syringes and individually packaged tablets or capsules. The unit dose form may be administered in portions or in plurality thereof. A multi-dose form is a plurality of identical unit dose forms packaged in a single container to be administered in separate unit dose forms. Examples of multi-dose forms include vials, tablets or capsules, or pint or gallon bottles. Therefore, the multi-dose form is a plurality of unit doses that are not separated in the package.

In certain embodiments, the pharmaceutical composition is a long-acting preparation. In certain embodiments, the long-acting preparation may be a sustained-release product. Suitable examples of sustained-release products include semi-permeable matrix of solid hydrophobic polymers including the compounds provided in the present invention, the matrix being in the form of tangible objects, such as films or microcapsules. Examples of sustained-release matrix include ionic electroosmotic patches, polyesters, hydrogels (e.g., poly(2-hydroxyethylmethacrylate), or poly(vinyl-ethyl alcohol)), polylactides, copolymers of L-glutamic acid and ethyl-L-glutamic acid, non-degradable ethylene-vinyl acetate, degradable lactic acid-glycolic acid copolymers, e.g., LUPRON DEPOTTM (injectable microspheres consisting of lactic acid-glycolic acid copolymers and leuprorelin acetate), and poly-D-(-)-3-hydroxybutyric acid. Polymers such as ethylene-vinyl acetate and lactic acid-hydroxyacetic acid allow release of molecules for more than 100 days, and some hydrogels release proteins for a relatively short period of time. When encapsulated compounds remain in vivo for a long time, they may denature or aggregate as a result of exposure to humid environment at 37 °C, resulting in loss of biological activity and possible changes in their structures. Depending on the mechanism involved, reasonable strategies may be designed for stability. For example, if it is found that the aggregation mechanism is formed by intermolecular S-S bonds of thio-disulfide interchange, stabilization may be achieved by modifying the sulfhydryl residues, lyophilizing from acidic solutions, controlling moisture content, utilizing appropriate additives, and developing special polymer matrix compositions. In certain embodiments, the pharmaceutical composition is a long-acting oral preparation.

Solid dosage forms for oral administration include capsules, tablets, pills, powders and granules. In such a solid dosage form, the active compound is mixed with at least one inert excipient (or carrier) (e.g., sodium citrate or dicalcium phosphate), and the followings may also be included: (a) fillers or blenders (e.g., starch, lactose, sucrose, glucose, mannitol, and silicic acid); (b) binders (e.g., carboxymethylcellulose, alginate, gels, polyvinylpyrrolidone, sucrose, and gum Arabic); (c) moisturizing agents (e.g., glycerine); (d) disintegrants (e.g., agar-agar, calcium carbonate, potato or cassava starch, alginic acid, certain synthetic silicates, sodium carbonate); (e) solution blockers (e.g., paraffin wax); (f) absorption enhancers (e.g., quaternary ammonium compounds); (g) wetting agents (e.g., hexadecanol and glycerol monostearate); (h) adsorbents (e.g., kaolin and bentonite) and (i) lubricants (e.g., talc, calcium stearate, magnesium stearate, solid polyethylene glycol, sodium lauryl sulfate) or mixtures thereof.

Similar types of solid compositions may also be used as fillers in soft-filled and hard-filled gel capsules using, for example, lactose, high molecular weight polyethylene glycol and the like as excipients.

Solid dosage forms (e.g., tablets, sugar-coated pills, capsules, pills, and granules) may be prepared using coatings and shells (e.g. intestinal coatings and others known in the art). They may contain light shielding agents, and they may also be active compounds or compositions of various active compounds released in a delayed manner in a part of the intestinal tract. Examples of usable embedding compositions are polymeric substances and waxes. The active ingredient may also be in microencapsulated form and may have one or more of the above excipients if appropriate.

Liquid dosage forms for oral administration include pharmaceutically acceptable emulsions, solutions, dispersions, syrups and elixirs. In addition to the active compounds, liquid dosage forms may include inert diluents (e.g., water or other solvents), solubilizers and emulsifiers (e.g., ethanol, isopropanol, ethyl carbonate, ethyl acetate, benzyl alcohol, benzyl benzoate, propylene glycol, 1,3-butanediol, dimethylformamide), oils (specifically cotton seed oil, peanut oil, corn oil, olive oil, castor oil, sesame oil), glycerol, tetrahydrofuran alcohol, polyethylene glycol, fatty acid esters of sorbitan, or mixtures of these substances, as commonly used in the art.

In addition to these inert diluents, pharmaceutical dosage forms may also include, for example, wetting agents, emulsifying and suspending agents, fragrance agents, flavoring agents, and aromatizing agents.

In addition to the active compounds, the suspension may contain suspending agents, such as ethoxylated isooctadecanol, polyethylene oxide sorbitol, sorbitan ester, microcrystalline fibers, meta-aluminum hydroxide, bentonite, agar-agar, astragalus gum, or mixtures of these substances, and the like.

The pharmaceutical dosage forms of the present invention also include ointments, powders, sprays and inhalants. The active component is mixed under sterile conditions with a physiologically acceptable carrier and any desired preservative, buffer or propellant.

The amount of the active ingredient in the pharmaceutical composition and pharmaceutical dosage form may be appropriately determined by a person skilled in the art as desired, e.g., each active ingredient is typically present in the pharmaceutical composition or dosage form in a therapeutically effective amount.

For example, the pharmaceutical composition of the present invention may be formulated in an oral dosage form (preferably an oral dosage form once, twice, three, or four times a day), an intravenous dosage form, a subcutaneous dosage form, or an intramuscular dosage form. In certain embodiments, the pharmaceutical composition of the present invention is formulated as a long-acting oral preparation.

In a preferred embodiment of the present invention, the pharmaceutical composition of the present invention is an orally administered dosage form. In a further preferred embodiment of the present invention, the pharmaceutical composition of the present invention is a dosage form for oral administration once, twice, three, or four times a day. In certain embodiments, the pharmaceutical composition of the present invention is a long-acting sustained-release oral preparation.

In a preferred embodiment of the present invention, the pharmaceutical composition of the present invention is an orally administered dosage form. In a further preferred embodiment of the present invention, the pharmaceutical composition of the present invention is a daily oral dosage form.

### 4. Mode of administration

The therapeutic method of the present invention involves combined administration of multiple active ingredients, also known as "combination therapy" or "combination treatment". The "combined administration" or "combination therapy" means administration of the various active ingredients of the present invention so that they work together to provide beneficial effects. The beneficial effects of the combinations include, but are not limited to, the combined effects in the pharmacokinetic aspect or pharmacodynamic aspects produced by the combinations of the active ingredients. The combined administration of these active ingredients is usually completed over a specified period of time (usually minutes, hours, days or weeks, depending on the doctor's judgment). "Combined administration" or "combination therapy" is intended to include administering the active ingredients in a sequential manner, i.e. each of the active ingredients is administered at a different time, and also include administering the active ingredients in a substantially simultaneous manner, or administering at least two of the active ingredients. Substantially simultaneous administration may be accomplished by, for example, administering to the host a single capsule containing a fixed proportion of each active ingredient, or administering to the host a plurality of capsules each containing one of the active ingredients. Sequential or substantially simultaneous administration of each active ingredient may be affected by any suitable route including, but not limited to, the oral route, the intravenous route, the intramuscular route, the intraocular route, and direct absorption via mucosal tissues. The active ingredients may be administered by a same route or by different routes. For example, a first active ingredient in a selected combination may be administered by intravenous injection, while the other active ingredients in the combination may be administered orally. Alternatively, for example, all active ingredients may be administered orally, or all active ingredients may be administered intravenously. The order of administration of these active ingredients is not strictly limited.

"Combined administration" or "combination therapy" also includes further administration of the active ingredients described above in combination with other biologically active ingredients and non-pharmaceutical therapy (e.g., surgical or mechanical therapy). When the combination therapy further includes a non-pharmaceutical therapy, the non-pharmaceutical therapy may be performed at any appropriate time as long as the beneficial effect produced by the combined action of the combination of the active ingredients and the non-pharmaceutical therapy can be achieved. For example, in appropriate cases, the beneficial effect can still be achieved after the non-pharmaceutical treatment is temporarily discontinued from the administration of the active ingredients, wherein the discontinuation may be for a few days or even a few weeks.

In order to achieve the desired effect, it is generally necessary to administer therapeutically effective amounts of the pharmaceutical composition or dosage form of the present invention or individual active ingredients to the subject.

In certain embodiments, the method of the present invention includes administering to a subject a therapeutically effective amount of doxazosin, a pharmaceutically acceptable salt or an acceptable form thereof, in combination with a therapeutically effective amount of pramipexole, a pharmaceutically acceptable salt or an acceptable form thereof, and further in combination with a therapeutically effective amount of metoprolol, a pharmaceutically acceptable salt or an acceptable form thereof.

In certain embodiments, the method of the present invention involves administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof simultaneously.

In certain embodiments, the method of the present invention involves administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof separately.

In some embodiments of the present invention, for a normal adult with a body weight of about 60 kg, when the pharmaceutical dosage form or pharmaceutical composition of the present invention is administered orally, the daily dose range of doxazosin may be 0.4 mg to 16 mg, for example, 0.4 mg to 15 mg, 0.4 mg to 13 mg, 0.4 mg to 12 mg, 0.4 mg to 10 mg, 0.4 mg to 8 mg, 0.4 mg to 6 mg, 0.4 mg to 5 mg, 0.4 mg to 4 mg, 0.5 mg to 16 mg, 0.5 mg to 15 mg, 0.5 mg to 13 mg, 0.5 mg to 12 mg, 0.5 mg to 10 mg, 0.5 mg to 8 mg, 0.5 mg to 6 mg, 0.5 mg to 5 mg, 0.5 mg to 4 mg, 1 mg to 15 mg, 1 mg to 13 mg, 1 mg to 12 mg, 1 mg to 10 mg, 1 mg to 8 mg, 1 mg to 6 mg, 1 mg to 5 mg, 1 mg to 4 mg, 1.5 mg to 16 mg, 1.5 mg to 15 mg, 1.5 mg to 13 mg, 1.5 mg to 12 mg, 1.5 mg to 10 mg, 1.5 mg to 8 mg, 1.5 mg to 6 mg, 1.5 mg to 5 mg, 1.5 mg to 4 mg, 2 mg to 16 mg, 2 mg to 15 mg, 2 mg to 13 mg, 2 mg to 12 mg, 2 mg to 10 mg, 2 mg to 8 mg, 2 mg to 6 mg, 2 mg to 5 mg, 2 mg to 4 mg, etc. In a preferred embodiment of the present invention, the daily dose of doxazosin may be about 2 mg. In another preferred embodiment of the present invention, the daily dose of doxazosin is about 8 mg.

In some embodiments of the present invention, for a normal adult with a body weight of about 60 kg, when the pharmaceutical dosage form or pharmaceutical composition of the present invention is administered orally, the daily dose range of pramipexole may be 0.03 mg to 4.5 mg, for example, 0.03 mg to 4.2 mg, 0.03 mg to 4 mg, 0.03 mg to 3.5 mg, 0.03 mg to 3 mg, 0.03 mg to 2.5 mg, 0.03 mg to 2 mg, 0.03 mg to 1.5 mg, 0.03 mg to 1 mg, 0.05 mg to 4.5 mg, 0.05 mg to 4.2 mg, 0.05 mg to 4 mg, 0.05 mg to 3.5 mg, 0.05 mg to 3 mg, 0.05 mg to 2.5 mg, 0.05 mg to 2 mg, 0.05 mg to 1.5 mg, 0.05 mg to 1 mg, 0.1 mg to 4.5 mg, 0.1 mg to 4.2 mg, 0.1 mg to 4 mg, 0.1 mg to 3.5 mg, 0.1 mg to 3 mg, 0.1 mg to 2.5 mg, 0.1 mg to 2 mg, 0.1 mg to 1.5 mg, 0.1 mg to 1 mg, 0.2 mg to 4.5 mg, 0.2 mg to 4.2 mg, 0.2 mg to 4 mg, 0.2 mg to 3.5 mg, 0.2 mg to 3 mg, 0.2 mg to 2.5 mg, 0.2 mg to 2 mg, 0.2 mg to 1.5 mg, 0.2 mg to 1.0 mg, 0.5 mg to 4.5 mg, 0.5 mg to 4.2 mg, 0.5 mg to 4 mg, 0.5 mg to 3.5 mg, 0.5 mg to 3 mg, 0.5 mg to 2.5 mg, 0.5 mg to 2 mg, 0.5 mg to 1.5 mg, 0.5 mg to 1 mg, 1 mg to 4 mg, 1 mg to 3 mg, 1 mg to 2.5 mg, etc. In a preferred embodiment of the present invention, the daily dose of pramipexole is about 0.0625 mg. In another preferred embodiment of the present invention, the daily dose of pramipexole is about 0.125 mg.

The daily dose range of metoprolol may be 2 mg to 200 mg, for example, 2.5 mg to 200 mg, for example, 5 mg to 200 mg, preferably 5 mg to 100 mg. In some embodiments of the present invention, for a normal adult with a body weight of about 60 kg, when the pharmaceutical dosage form or pharmaceutical composition of the present invention is administered orally, the daily dose range of metoprolol may be 2 mg to 180 mg, 2 mg to 160 mg, 2 mg to 150 mg, 2 mg to 130 mg, 2 mg to 120 mg, 2 mg to 100 mg, 2 mg to 80 mg, 2 mg to 60 mg, 2 mg to 50 mg, 2 mg to 40 mg, 5 mg to 180 mg, 5 mg to 160 mg, 5 mg to 150 mg, 5 mg to 130 mg, 5 mg to 120 mg, 5 mg to 100 mg, 5 mg to 80 mg, 5 mg to 60 mg, 5 mg to 50 mg, 5 mg to 40 mg, 10 mg to 180 mg, 10 mg to 160 mg, 10 mg to 150 mg, 10 mg to 130 mg, 10 mg to 120 mg, 10 mg to 100 mg, 10 mg to 80 mg, 10 mg to 60 mg, 10 mg to 50 mg, 10 mg to 40 mg, 15 mg to 200 mg, 15 mg to 180 mg, 15 mg to 160 mg, 15 mg to 150 mg, 15 mg to 130 mg, 15 mg to 120 mg, 15 mg to 100 mg, 15 mg to 80 mg, 15 mg to 60 mg, 20 mg to 200 mg, 20 mg to 180 mg, 20 mg to 160 mg, 20 mg to 150 mg, 20 mg to 130 mg, 20 mg to 120 mg, 20 mg to 100 mg, 20 mg to 80 mg, and 20 mg to 60 mg, etc. In a preferred embodiment of the present invention, the daily dose range of metoprolol is 10 mg to 100 mg. In another preferred embodiment of the present invention, the daily dose of metoprolol is about 20 mg. In another preferred embodiment of the present invention, the daily dose of metoprolol is about 100 mg.

The daily doses described above may be administered periodically and continuously, e.g., once every 2 hours, every 6 hours, every 8 hours, every 12 hours, or approximately every 24 hours. Preferably, the daily dose may be administered to the subject once, twice, three, or four times a day, or as sustained-release tablets. The daily oral doses of the above three active ingredients are quite different, which is determined by the pharmacokinetics of each active ingredient in vivo.

In certain embodiments, the therapeutically effective amount of doxazosin, a pharmaceutically acceptable salt or an acceptable form thereof, the therapeutically effective amount of pramipexole, a pharmaceutically acceptable salt or an acceptable form thereof, and the therapeutically effective amount of metoprolol, a pharmaceutically acceptable salt or an acceptable form thereof are administered to a subject at a frequency of once, twice, three, or four times per day.

It will be understood by those skilled in the art that when the pharmaceutical composition of the present invention is formulated into other dosage forms suitable for intravenous drip, intramuscular injection or the like, the dose range of each active ingredient may differ from the oral dose range given above, which may be reasonably determined by those skilled in the art or physician in conjunction with in vivo and in vitro experiments and taking into account the different pharmacokinetic characteristics of various routes of administration.

### 5. Subjects

In a preferred embodiment of the present invention, the pharmaceutical composition of the present invention is used in mammals, primate subjects and, in particular, human subjects.

Over the past decades, neuroprotection has been demonstrated to be effective in more than 1,000 cell experiments and rodents such as ischemia-reperfusion rat/mouse models, but has been ineffective and unsuccessful in clinical trials, mainly because of the differences in anatomical structure between small animal brains and human brains (Cook DJ, Teves L, Tymianski M. Treatment of stroke with a PSD-95 inhibitor in the gyrencephalic primate brain. Nature. 2012 Feb 29; 483(7388):213-7.).

In the examples of the present invention, 90-minute middle cerebral artery occlusion (MCAO) reperfusion model in rhesus monkeys is used to evaluate the neuropathic modulation and protective effects of the composition, in particular the therapeutic effect on stroke. As primate models, MCAO rhesus monkey models have the following advantages:
(1) Rhesus monkeys have obvious brain sulci, and their brain volume and structure are similar to human cortex, subcortex and white matter anatomy, and they can be evaluated for cognitive, motor, and sensory deficits through standardized tests.
(2) Rhesus monkeys can survive for a long time and undergo multiple dynamic MRI examinations. The trajectories of brain degeneration and necrosis such as ischemia, edema and necrosis after stroke are dynamically observed at 3-4 h, 72-75 h, Day 7, Day 14 and Day 28 after reperfusion. MRI imaging sequences include T1WI, T2FLAIR, DWI, MRA, and DSC. Among them, T1WI may show the anatomical structure of brain, differentiate between gray matter area and white matter area, diagnose hemorrhage and ischemia, etc., T2 FLAIR and DWI may quantify edema/necrosis volume, and T2 FLAIR may suggest increased water content within the tissue, and it is used to show inflammation, ischemia, edema and other lesions. After cerebral infarction, inflammatory reaction occurs immediately, accompanied by an increase in water content. Therefore, the abnormal high signal of T2 FLAIR indicates the existence of cerebral infarction and inflammation. DSC can show the cerebral tissue perfusion condition and sensitive response to cerebral ischemia area after acute ischemic stroke, and can quantitatively evaluate the volume of ischemic core area of brain tissue. MRI can quantify the ischemic penumbra range and edema/infarction volume, and the results thereof can be applied to humans with high consistency.
(3) The middle cerebral artery of anterior circulation large vessel is a site where human ischemic stroke occurs frequently. In the present invention, rhesus monkey 90-minute-MCAO reperfusion model is used, and M1 segment is selected as the occlusion site. The model animals exhibit an edema/infarction volume of about 40% of the hemisphere at the peak of edema, showing a significant midline shift. The practice of evaluating the modulation effect on neuropathy using such serious brain edema/infarction models can be transformed to be used for more clinical patients. MRA scanning 3-4 hours after reperfusion can accurately show whether blood vessels are reperfused and whether collateral circulation is formed. Moreover, DWI images show ischemic infarction lesions in middle cerebral artery and quantitative edema volume similar to those in clinical patients. They are used as the criteria for model success, which may reduce variability of the models.
(4) In this study, Non-Human Primate Stroke Scale (NHPSS) is used to quantify the neurological deficits in rhesus monkeys. NHPSS is a combination of scores similar to the National Institute of Health Stroke Scale (NIHSS).

In the examples of the present invention, rhesus monkey 180-minute middle cerebral artery occlusion (MCAO) reperfusion model is used to evaluate the neuropathic modulation effect of the composition. The 180-minute MCAO reperfusion model may be used as cognitive dysfunction models to evaluate the neuroprotective effect of the composition and prevent and improve cognitive function. In addition to the advantages of the 90-minute MCAO reperfusion model mentioned above, the 180-minute MCAO reperfusion model can form a wider range of edema/infarction than the 90-minute MCAO reperfusion model at baseline (i.e., before grouping and drug administration). The model animals show an edema/infarction volume accounting for about 70% of the hemisphere at the peak of edema, which may lead to long-term obvious cognitive decline, and may be used to evaluate the efficacy of neuroprotective agents in preventing and improving cognitive function. In the examples of the 180-minute MCAO reperfusion models of the present invention described below, cognitive functions of the monkeys, including learning and memory ability, visuospatial ability and ability to understand and judge, are also evaluated by Spatial Delayed Response testing.

The rhesus monkey panretinal ischemia/reperfusion injury models provide important insights for the mechanism and treatment strategy of human I/R injury, especially in the neurodegenerative injury of retinal neurovascular unit. In the examples of the present invention, rhesus monkey panretinal ischemia/reperfusion injury models are used. At present, the commonly used models are rat/mouse acute elevated intraocular pressure (IOP) reperfusion models, while the advantages of primate models lie in: (1) the presence of macula in rhesus monkeys, which is an important site of injury; (2) retinal vascular pattern: the morphology of retinal blood vessels in rhesus monkeys is the same as that in humans; and (3) rhesus monkeys have larger eyes, with the volume of the anterior chamber and the vitreous body being more similar to that of human eyes, which provides more opportunities for new interventions to be applied to humans (Pasquale, L. R. et al. The Prevalence of Primary Open-Angle Glaucoma-Like Features in a Rhesus Macaque Colony from Southern China. Invest. Ophthalmol. Vis. Sci. 61, 1436-1436 (2020); Morgan, J. E. Optic nerve head structure in glaucoma: Astrocytes as mediators of axonal damage. Eye 14, 437-444 (2000); Tang, Y. et al. Therapeutic Targeting of Retinal Immune Microenvironment With CSF-1 Receptor Antibody Promotes Visual Function Recovery After Ischemic Optic Neuropathy. Front. Immunol. 11, 585918 (2020)).

Therefore, in the present invention, rhesus monkey panretinal ischemia/reperfusion injury models are used to study retinal nerve cell apoptosis and retinal degeneration trajectory after I/R injury, fundus photography (FP) and optical coherence tomography (OCT) quantitatively and dynamically evaluate the effects on retinal structure at Day 7 (D7), Day 21 (D21), Day 35 (D35) and Day 63 (D63) after I/R injury in rhesus monkeys, including changes in retinal thickness and retinal nerve fiber layer (RNFL), flash visual evoked potential (FVEP) and full-field electroretinogram (FERG) dynamically evaluate optic nerve conduction function and overall retinal function, while evaluating whether the composition can inhibit RGC cell apoptosis, retinal gliosis and inflammation to prevent the loss of retinal function after I/R.

In the examples of the present invention, spontaneous diabetic DPN rhesus monkey models are also used to evaluate the efficacy and safe tolerance of the composition to peripheral neuropathy. The primate models have the advantages that rhesus monkeys are very similar to humans in physiology, biochemistry, and systems biology, and disease characteristics of spontaneous DPN rhesus monkeys have been found to be very similar to those of clinical human DPN patients. In 1989, Cornblath et al. (Cornblath D R, Hillman M A , Striffler J S, et al. Peripheral neuropathy in diabetic monkeys[J]. Diabetes, 1989, 38(11):1365-1370.; Cornblath D R, Dellon A L, Mackinnon S E. Spontaneous diabetes mellitus in a rhesus monkey: Neurophysiological studies[J]. Muscle & Nerve, 1989, 12(3):233-235.) reported that spontaneous diabetic DPN rhesus monkey models exhibit significantly reduced motor and sensory nerve conduction velocities compared with non-diabetic rhesus monkeys, and the pathogenesis and clinical features are similar to those of human patients. In addition, most of the DPN animal models commonly used in current studies are rodents, for example, a variety of rat and mouse models are created using ischemia-reperfusion, neurotomy, STZ (streptozotocin) induction alone, transgenic techniques, etc. However, because the pathogenesis of DPN is complex, which involves many steps, it is difficult for the impairment of neurologic function in these animal models to accurately reflect the course of DPN in patients. Therefore, the spontaneous diabetic DPN rhesus monkey models can provide important research means for DPN research and new drug development, and provide more accurate conclusions than other animal models.

Those skilled in the art will appreciate that the various aspects of the present invention described herein may be combined separately in various manners as will be apparent to those skilled in the art without departing from the subject matter and idea of the present application. The combination is also included in the scope of the present application. For example, the range of amount of certain components involved in the present invention includes any combination of any lower limit and any upper limit mentioned in the specification, and also includes any range formed by the specific contents of the component in each specific example as upper or lower limit combinations; and all these ranges are covered within the scope of the present invention. Further, each of the features of the present invention listed in the specification may be combined with any other features of the invention, and such combinations are also within the scope of the disclosure of the present invention. Specific examples of the present application are described in detail below with reference to the accompanying drawings.

### Examples

### 1. Example 1

The inventors evaluated the prevention, treatment, and/or improvement effects of doxazosin (DOX), pramipexole (PMP) and metoprolol (MTP) in combination on central neuropathy, especially ischemic stroke, including the effects on reducing edema/infarction volume and improving neurological deficit after stroke, using rhesus monkey 90-minute middle cerebral artery ischemia reperfusion model. Evaluation indexes include:
(1) 3.0 T GE MRI is used to dynamically observe edema/infarction trajectories at 3-4 h, 72-75 h, Day 7, Day 14, and Day 28 after reperfusion; MRI imaging sequence including T1WI, T2 FLAIR, DWI, MRA, CTA. Among them, T1WI may show the anatomical structure of brain, differentiate between gray matter area and white matter area, diagnose hemorrhage and ischemia, etc., T2 FLAIR and DWI may quantify edema/necrosis volume, and T2 FLAIR may suggest increased water content within the tissue, and it is used to show inflammation, ischemia, edema, and other lesions.
(2) MRA and CTA scanning 3-4 hours after stroke can accurately show whether blood vessels are reperfused and whether collateral circulation is formed. Moreover, DWI images show ischemic infarction lesions in middle cerebral artery and quantitative edema volume similar to those in clinical patients. They are used as the criteria for model success, which may reduce variability of the models. The design in which the composition is administered 5 hours after stroke can be transformed to clinical practice better than prophylactic administration.

### 1.1 Experimental materials

### Test product 1:

Name or abbreviation (English name): Doxazosin mesylate (DOX)
Purity: 99.6% HPLC
Produced by Zhejiang Xinsaike Pharmaceutical Co., Ltd.

### Test product 2:

Name or abbreviation (English name): Pramipexole 2HCL Monohydrate (PMP)
Purity: 99.55% HPLC
Produced by Shanghai Ziqi Biotechnology Co., Ltd.

### Test product 3

Name or abbreviation (English name): Metoprolol tartrate salt (MTP)
Purity: 100% HPLC
Produced by Sun Pharmaceutical Industries Ltd., India

### 1.2 Specific experimental scheme

### 1.2.1 Laboratory animals

12 healthy rhesus monkeys aged 5-7 years were selected, Macaca mulatta (Rhesus Macaque), general grade, weight: 6-9 kg, provided by Primed Primate Research Center.

The feeding environment grade in the test period: ordinary grade. Temperature: 18-26 °C. Relative humidity: 40% to 70%. Ventilation: no less than 8 air changes per hour, using 100% fresh air (no air circulation). Illumination time: automatic illumination, alternating light and dark every 12 hours. Animal cage: 850*900*2365 mm double-layer stainless steel cage. Feeding density: 1 per cage. All operations had been reviewed and approved by the IACUC Committee of the Primed Primate Research Center.

### 1.2.2 Preparation of rhesus monkey 90-minute middle cerebral artery occlusion (MCAO) reperfusion model

Rhesus monkey MCAO model was prepared by craniotomy (FIG. 1 and FIG. 2). The monkeys were fasted but could drink water for at least 8 hours before surgery, and after being anesthetized with 10 mg/kg propofol and 2% isoflurane, their heads were shaved and the skin of the surgical area was sterilized. The animals were placed supine on a thermostatic surgical bed. Skin was cut from the right top of the head to the ear to expose the skull. The skull was drilled with an electric drill, and a bone flap with a size of 4×5 cm was removed with bone forceps to expose the dura mater. After cutting the dura mater, the middle cerebral artery was located along the lateral sulcus of the brain under a surgical microscope, and the M1 distal end and M2 branch of the middle cerebral artery were determined, where the middle cerebral artery was clipped with an aneurysm clip for 90 minutes, after that, the aneurysm clip was removed, and blood supply was restored for reperfusion. The dura mater, muscles, and skin were sutured and sterilized.

Magnetic resonance angiography (MRA) and CT angiography (CTA) of the brain were performed in rhesus monkeys 3h-4h after MCAO (after reperfusion). Whether the middle cerebral artery of rhesus monkeys was reperfused was determined through angiographic images. As shown in FIGS. 2A and 2C, bilateral middle cerebral arteries were obviously visible, and it was determined that reperfusion succeeded (see FIG. 3). As shown in FIGS. 2B and 2D, compared with the contralateral side, the middle cerebral artery on the surgical side was invisible. And it was determined that reperfusion was not formed. In addition, DWI images 3-4 hours after reperfusion were shown in FIG. 3C, showing abnormal high signal in the right frontal, parietal, and temporal lobes, similar to the ischemic infarction lesions of the middle cerebral artery in clinical patients. The 12 animals selected in this example were formed by reperfusion after 90-minute MCAO occlusion in rhesus monkeys.

### 1.2.3 Grouping and drug administration

12 rhesus monkey model animals formed by reperfusion after 90-minute occlusion were quantified by DWI for edema volume 3-4 hours after reperfusion, and grouped. DWI is the only imaging method that can reflect the diffusion characteristics of water molecules. Its high signal reflects infarction areas in which diffusion movement of water molecules is limited, changes may occur just 2 hours after ischemia, and early brain edema which cannot be found by T2 imaging can be found. Therefore, quantifying edema/infarction area volume by DWI is the basis for grouping. 12 MCAO rhesus monkeys were divided into model control group (6) and DOX+PMP+MTP group (6). The mean volumes of edema/infarction areas quantified by DWI in the two groups were 8.98±1.14 cm³ and 8.17±0.85 cm³. There was no statistical difference between the two groups. The results were shown in FIG. 3.

Animals in the administration group were administered orally about 5 hours after stroke (i.e., the time point of implementing aneurysm clipping artery was the time point of stroke attack), and were administered 2-4 times a day at intervals for 28 consecutive days. The following was the dosage and mode of each administration:
Model control group: administered with empty capsules.
DOX+PMP+MTP treatment group: DOX: 0.266 mg/kg to 0.532 mg/kg; PMP: 0.002 to 0.004 mg/kg; MTP: 1.667 mg/kg.

### 1.2.4 Test procedure

MRI scans were performed at 3-4h, 72-75h, Day 7, Day 14, and Day 28 after MCAO reperfusion to measure brain edema/infarction volume, and neurobehavioral scoring (NHPSS) was performed. The specific experimental design is shown in FIG. 4.

### 1.3 Observation index and monitoring method

### 1.3.1 Main efficacy indexes

### 1.3.1.1 Brain tissue scanning and brain edema/infarction volume quantification by 3.0T GE 750w MRI

After the rhesus monkeys were anesthetized with 5-10 mg/kg propofol and 2% isoflurane, and they were subjected to head scanning on a 3.0T GE 750w MRI instrument using customized head coils for non-human primates. Scanning sequences included: T1WI, T2FLAIR, DWI and 3D-TOF. The scanning covers the whole brain tissue, including cerebral cortex, cerebellum and temporal lobe. The scanning parameters of T2 FLAIR were: TE 120 ms, TR 8000 ms, matrix 288×288, layer thickness: 1.5 mm, FOV 14 cm, resolution: 273 µm×273 µm×1500 µm; the scanning parameters of DWI were: TE 85 ms, TR 7000 ms, b=1000s, matrix 140×140, layer thickness: 2.0 mm, FOV 14 cm, resolution: 1094 µm×1094 µm×2000 µm. The two sequences were used to measure infarction volume.

Advantage Workstation 4.7 of GE MRI was used for image processing. In each scanning layer image, the boundary of the high signal region of the brain tissue on the surgical side was manually delineated as the region of interest (ROI). The software automatically calculated the area of ROI, and this area was the area of cerebral infarction at this layer. Calculation method of cerebral infarction volume: infarction volume V=t×(A1+A2+A3... An). t was the thickness of the scanning layer, and A was the cerebral infarction area of each layer.

### 1.3.1.2 NHPSS neurobehavioral score

Non-Human Primate Stroke Scale (NHPSS) was used for quantitative evaluation of neurological deficits of the animals. The score of 41 indicated severe bilateral nerve injury and the score of 0 indicated being normal. The detection time points were: 16 h, 24 h, 48 h, 72 h, 96 h, Day 5, Day 7, Day 14, and Day 28 after MCAO.

Neurobehavioral evaluation of monkeys after cerebral ischemia surgery was carried out by single blind method, that is, the animals were marked by group by experimental designers, and the testers who scored the neurobehavior were unaware of the grouping of the animals. After scoring, the scorers submitted the scoring results to the designers who would unblind to obtain the score of each animal in each test group.

The National Institute of Health Stroke Scale (NIHSS) is recommended to evaluate the degree of cerebral infarction. NIHSS baseline evaluation may reflect the severity of stroke at onset, and may also evaluate the effect after treatment regularly. NIHSS ranges from 0 to 42. The higher the score, the more severe the stroke.

The Non-Human Primate Stroke Scale (NHPSS) is a combination of scores similar to the National Institute of Health Stroke Scale (NIHSS). Some literatures already show that the scoring indexes of NHPSS and NIHSS are substantially consistent (Brott TG, Adams HP Jr, Olinger CP, et al. Measurements of acute cerebral infarction: a clinical examination scale. Stroke 1989; 20: 864-70.; A S E K. Clinical interpretation and use of stroke scales.[J]. The Lancet Neurology, 2006, 5(7):603-612.).

According to NHPSS, improvement of neurologic deficits was evaluated dynamically at 16 h, 24 h, 48 h, 72 h, 96 h, Day 5, Day 7, Day 14, and Day 28 after stroke. NHPSS scores consists of comprehensive scores of state of consciousness, defense response, grasping reflex, limb movements, gait, rotational movements, motor delays, balance, response to the outside world, and facial muscle strength. The total score is 41, 0 represents normal behavior, and 41 represents severe bilateral nerve injury. Specific NHPSS scoring items are shown in Table 1 (Roitberg B , Khan N , Tuccar E , et al. Chronic ischemic stroke model in cynomolgus monkeys: Behavioral, neuroimaging and anatomical study[J]. Neurological Research, 2003, 25(1):68-78.).

**Table 1 Neurobehavioral score table (NHPSS) of monkeys with cerebral ischemia**

| Item (score) (Healthy side-right side, affected side-left side) | | Scoring standard |
|---|---|---|
| State of consciousness (0-2) | | 0-Normal |
| | | 1 -Befuddled/apathetic |
| | | 2-Unconscious |
| Defensive response (0-2) | | 0-Normal |
| | | 1-weakened |
| | | 2-None |
| Upper limb grasping reflex (0-1×2) (right/left) | | 0-Yes |
| | | 1-None |
| Moveme nt of limbs | (Score of affected upper limb) | 0-Normal |
| | | 1-Asymmetric use or strength |
| | (Score of affected lower limb) | 2-Significantly weak |
| | | 3-Small movements, severely weak |
| | | 4-Involuntary use, no response to stimulation (no active use, no use under stimulation) |
| Gait (0-3) | | 0-Normal |
| | | 1-Limp (because the upper limbs could not move, the sense of balance of movement was poor, and the speed and flexibility of movement were reduced) |
| | | 2-Seriously damaged |
| | | 3-Unable to walk (could crawl) |
| Circling movement (0-2) | | 0-Normal behavior |
| | | 1-Clear tendency to turn to one side |
| | | 2-Rotation at constant velocity |
| Bradykinesia (0-2) (Affected side) | | 0-None |
| | | 1-Slight |
| | | 2-Serious |
| Balance (0-2) | 0-Normal | |
| | 1-Mildly damaged | |
| | 2-Seriously damaged and unable to stand on both feet | |
| Ignoring (0-2×2) (right/left) | 0-No ignoring | |
| | 1-When both sides were stimulated at the same time, the affected side had no stimulation response | |
| | 2-Completely ignore all stimuli on the affected side, including visual, auditory, and tactile stimuli | |
| Loss of visual field/hemianopia (0-1×2) (right/left) | 0-None | |
| | 1-No response to visual stimuli | |
| Facial paralysis (0-2×2) (right/left) | 0-None | |
| | 1-Mildly damaged | |
| | 2-Deeply damaged (salivation, hanging corners of the mouth) (upturned corners of the mouth, drooling, hanging corners of the mouth) | |
| Total score: 41 | | |

The following is the scoring indexes of the U.S. National Institute of Health Stroke Scale (NIHSS).

### 1. Level of Consciousness (LOC)

### 1a: LOC-response

| **Score** | **Test results** |
|---|---|
| **0** | Alert, keenly responsive |
| **1** | Not alert; but arousable by minor stimulation to obey, answer, or respond |
| **2** | Not alert; requires repeated stimulation to attend, or is obtunded and requires strong or painful stimulation to make movements (not stereotyped) |
| **3** | Responds only with reflex motor or autonomic effects, or totally unresponsive, flaccid, and areflexic |

### 1b: LOC-questions

| **Score** | **Test results** |
|---|---|
| **0** | Answers both questions correctly |
| **1** | Answers one question correctly, or patients who fail because of orotracheal trauma, or any other problem not secondary to aphasia |
| **2** | Answers neither question correctly, aphasic and stuporous patients who do not comprehend the questions, default coma scale (DCS) |

### 1c: LOC-instructions

| **Score** | **Test results** |
|---|---|
| **0** | Performs both tasks correctly |
| **1** | Performs one task correctly |
| **2** | Performs neither task correctly |

### 2. Gaze

| **Score** | **Test results** |
|---|---|
| **0** | Normal |
| **1** | Partial gaze palsy; gaze is abnormal in one or both eyes, but forced deviation or total gaze paresis is not present; isolated peripheral ophthalmoplegia |
| **2** | Forced gaze, or total gaze paresis (not overcome by the oculocephalic reflex) |

### 3. Visual field

| **Score** | **Test results** |
|---|---|
| **0** | No visual loss |
| **1** | A clear-cut asymmetry, including quadrantanopia, or partial hemianopia or on the verge of death |
| **2** | Complete hemianopia |
| **3** | Bilateral hemianopia (including cortical blindness) or total blindness of any cause |

### 4. Facial palsy

| **Score** | **Test results** |
|---|---|
| **0** | Normal |
| **1** | Minor paralysis (flattened nasolabial fold, asymmetry on smiling) |
| **2** | Partial paralysis (total or near-total paralysis of lower face) |
| **3** | Complete paralysis of one or both sides (absence of facial movement in the upper and lower face) |

### 5. Upper limb movement

| **Score** | **Test results** |
|---|---|
| **0** | In the required position; limb holds for full 10 seconds |
| **1** | Drifts down before full 10 seconds; does not touch bed or other support, although not kept in the required position |
| **2** | Some effort against gravity; drifts down to bed or other support within 10 seconds |
| **3** | No effort against gravity; limbs fall immediately, but can still do some movements (such as shrugging shoulders) |
| **4** | No movement, unable to cause voluntary movement of upper limbs |
| **8** | Default coma scale (DCS) |
| **UN** | Amputation or joint fusion (records: 5a. left upper limb; 5b. right upper limb) |

### 6. Movement of lower limbs

| **Score** | **Test results** |
|---|---|
| **0** | In the required position, leg holds for full 5 seconds |
| **1** | Drifts down before full 5 seconds; does not touch bed or other support, although not kept in the required position |
| **2** | Some effort against gravity; drifts down to bed or other support within 5 seconds |
| **3** | No effort against gravity; leg falls to bed immediately, but can still do some movements (such as hip flexion) |
| **4** | No movement, unable to cause voluntary movement of lower limbs |
| **8** | Default coma scale (DCS) |
| **UN** | Amputation or joint fusion (records: 6a. left lower limb; 6b. right lower limb) |

### 7. Limb ataxia

| **Score** | **Test results** |
|---|---|
| **0** | Absent: smooth and accurate movements |
| **1** | Present in one limb: stiff or inaccurate movements |
| **2** | Present in two or more limbs: stiff or inaccurate movement of limb on one side |
| **UN** | Amputation or joint fusion (record) |

### 8. Sensory

| **Score** | **Test results** |
|---|---|
| **0** | No sensory loss |
| **1** | Mild-to-moderate sensory loss; patient feels pinprick is less sharp or is dull on the affected side; or there is a loss of superficial pain with pinprick, but patient is aware of being touched |
| **2** | Severe or total sensory loss on one side; patient is not aware of being touched in the limbs on one side, default coma scale (DCS) |

### 9. Language

| **Score** | **Test results** |
|---|---|
| **0** | Normal: no aphasia |
| **1** | Mild-to-moderate aphasia: some loss of fluency or facility of comprehension, without significant limitation on ideas expressed or form of expression |
| **2** | Severe aphasia: all communication is through fragmentary expression; great need for inference, questioning, and guessing by the listener, ask, guess, with difficulties in communication |
| **3** | Mute, complete aphasia; no usable speech or auditory comprehension, default coma scale (DCS) |

### 10. Dysarthria

| **Score** | **Test results** |
|---|---|
| **0** | Normal: clear and fluent pronunciation |
| **1** | Mild-to-moderate dysarthria; patient slurs at least some words and, at worst, can be understood |
| **2** | Severe dysarthria; patient's speech is so slurred as to be unintelligible, or is mute, default coma scale (DCS) |
| **UN** | Intubated or other physical barrier (record) |

### 11. Neglect

| **Score** | **Test results** |
|---|---|
| **0** | Normal: answer all questions correctly |
| **1** | Visual, tactile, auditory, or spatial awareness: neglect of one side under a certain stimulus mode: |
| **2** | Hemi-inattention: neglect of the same side in more than one stimulation mode, default coma scale (DCS) |

### 1.3.1.3 Immunohistochemical quantitation of expression of GFAP in peri-infarction area

30 days after stroke in rhesus monkey 90-minute middle cerebral artery ischemia reperfusion, one animal from the placebo group and one animal from the DOX+PMP+MTP group were euthanized under pentobarbital anesthesia. After dissection, the whole brain tissue was taken out and fixed in 4% paraformaldehyde for 72 hours or more. Brain tissue was taken from the infarcted area of the cerebral hemisphere on the surgical side, and brain tissue was taken from the corresponding area of the cerebral hemisphere on the non-surgical side. 14 coronal brain slices (4 mm thick) were prepared using MSM-2500 brain matrix. The areas around cerebral infarction in temporal lobe and precentral gyrus were sequentially dehydrated, waxed, embedded, and sectioned (3 slices, 5 µm/slice) for GFAP immunohistochemical staining. Paraffin slices were routinely dewaxed to water, then subjected to EDTA hot water bath for antigen repair for 20 min, and then cooled to room temperature; followed by PBS washing and incubation with 3% H₂O₂ at room temperature for 20 min; and dropwise addition of normal goat serum after PBS washing, and sealing at 37°C for 20 min. Primary antibody (GFAP, 1:250) was added dropwise subsequently, for incubation overnight at 4°C. After PBS washing, reaction enhancement solution was added dropwise for incubation at 37°C for 20 min; after PBS washing, secondary antibody was added dropwise for incubation at 37°C for 20 min; and after PBS washing, DAB color developing agent was added dropwise for color development at room temperature, with control under microscope, and distilled water was used to stop the color development. Hematoxylin restaining was performed for 2 min, followed by dehydration with alcohol, rendering transparent with xylene; and sealing with neutral gum. Negative control was set in each experiment, and PBS solution was used instead of primary antibody. Other steps were exactly the same. Three immunohistochemical slices were taken under optical microscope to quantify GFAP protein. Two infarction peripheral areas were selected for each slice, and images were collected under 200 times visual field. Image Pro plus 6.0 morphological analysis software was used to quantify GFAP protein expression in six infarction peripheral areas.

### 1.3.2 Caring of stroke rhesus monkeys

MCAO rhesus monkeys were transferred to neurological ICU for care immediately after surgery, and their heart rate, blood oxygen saturation, blood pressure, electrocardiogram, urine volume and body temperature were monitored regularly. Biochemical and hematological examinations were performed at 3 h after MCAO surgery (before MRI examination) and at 16 h, 27 h, 48 h, 72 h, 96 h, 7 d (Day 7), 14 d (Day 14), and 28 d (Day 28) after surgery. No drugs to reduce intracranial pressure or brain edema were used in the test.

The 12 MCAO rhesus monkeys selected in this test did not die during the 28-day test period, and all 12 animals survived.

### 1.3.3 Data processing

Statistical analysis software, the quantitative data were expressed as mean±standard deviation. One-way analysis of variance was employed for cerebral infarction volume and neurological defect symptom score. P<0.05 was defined as significant difference.

### 1.4 Experimental results and discussion

### 1.4.1 Effects on brain edema/infarction volume and brain tissue at various time points after stroke in subjects

Changes of brain edema/infarction volume at various time points after stroke in subjects of each group are shown in FIG. 5.

In the model control group (N=6), the mean volume of DWI cerebral edema/infarction on the surgical side (right side) 3-4 hours after reperfusion was 8.98±1.14 cm³, the mean volume of cerebral edema/infarction when the peak of edema was reached within 75 h after stroke was 21.57±4.31 cm³, and the edema volume accounted for about 40% of the hemisphere volume, with a significant midline shift. MRI imaging examination showed lesions in frontal lobe, parietal lobe and temporal lobe on the surgical side (right side). At Day 7 (D7) after stroke, the mean volume of T2 FLAIR cerebral edema/infarction was 20.60±6.45 cm³, and at the same time, some brain tissue in frontal lobe area showed necrotic liquefaction lesion. From Day 14 (D14) to Day 28 (D28) after stroke, the volume of brain tissue edema/infarction gradually decreased to 10.34±3.91 cm³ and 7.93±3.39 cm³, respectively, and at the same time, it could be seen that the boundaries between liquefied necrosis area and infarction area and healthy (left side) brain tissue was clearer.

DOX+PMP+MTP treatment group (N=6): 6 MCAO rhesus monkeys were administered orally about 5 hours after stroke for 28 consecutive days, and the brain tissue edema/infarction volume on the surgical side (right side) decreased significantly at 75 h, Day 7, Day 14, and Day 28 after stroke (p<0.05). The mean volumes of T2 FLAIR brain edema/infarction at the peak of edema at 75 h and Day 7 (D7) after stroke were 13.86±3.63 cm³ and 11.62±3.63 cm³, and the mean volumes of T2 FLAIR brain edema/infarction at Day 14 (D14) and Day 28 (D28) after stroke were 5.83±2.24 cm³ and 4.34±1.20 cm³.

The above data showed that the composition containing three drugs could significantly reduce the brain edema/infarction volume in the 90-minute-MCAO reperfusion rhesus monkey model, when administered 5 hours after stroke for 28 consecutive days.

### 1.4.2 Effects on NHPSS neurobehavioral score at various time points after stroke in subjects

Changes of NHPSS neurobehavioral scores at various time points after stroke in subjects of each group were shown in FIG. 6.

Model control group (N=6): NHPSS scores were 15.4±2.9, 12.0±1.6, and 7.4±2.6 at 24 h, 72 h and Day 7 (D7) after stroke, which mainly showed weak defense response, decreased strength of upper and lower limbs on the contralateral side (affected side) compared with the healthy limbs, weakness of the affected arm preventing it from lifting above the head, neurological deficits in the affected fingers preventing them from grasping food, bradykinesia, and a significant reduction in the ability to balance and make rotational movement. The NHPSS scores at Day 14 (D14) and Day 28 (D28) after stroke decreased gradually, which were 3.6±1.8 and 1.8±0.4, respectively, and the fine function of fingers and arm strength on the affected side (left side) of MCAO rhesus monkeys at Day 28 (D28) were still slightly worse than those on the healthy side (right side).

DOX+PMP+MTP group (N=6): the NHPSS scores at 24 h, 72 h, and Day 7 (D7) after stroke were 10.3±3.1, 5.3±2.4 and 2.7±1.9, which were significantly lower than those of the model control group at the same time point (p<0.05 or p<0.01). The NHPSS scores at Day 14 (D14) and Day 28 (D28) after stroke were 1.2±0.4 and 0.7±0.5, respectively.

As could be seen from the above, the DOX+PMP+MTP treatment group, when administered 5 hours after stroke, significantly reduced the neurobehavioral scores of MCAO rhesus monkeys and significantly improved the neurological deficit in stroke animals, and recovered finger function and limb strength of affected limbs significantly faster than the model control group, and the imaging results were highly correlated with NHPSS scores.

### 1.4.3 Effect on GFAP protein expression

The results are shown in FIG. 7. In the placebo group, the brain tissue GFAP protein expression in the peri-infarction area increased significantly 30 days after stroke, with a significant increase of 3-4 times compared to the GFAP protein expression in the corresponding area of the cerebral hemisphere on the non-surgical side (p<0.01), the GFAP protein expression was 9.79±1.93, and at the same time, morphological changes of reactive astrocytes were observed, with the cell bodies being hypertrophic, swollen, and having more protrusions.

Compared with the placebo group, the brain tissue GFAP expression in the peri-infarction area in the DOX+PMP+MTP group decreased significantly at Day 30 after stroke (p<0.01), and the GFAP expression was 4.21±0.75. Treatment with DOX+PMP+MTP composition for 30 days significantly inhibited the increase of GFAP protein expression in brain tissues in the peri-infarction area of stroke rhesus monkeys, and inhibited neuroinflammation.

### 1.5 Safety tolerance

The safe dose ranges and toxic side effects of DOX, PMP, and MTP are known. The dosage of DOX tablets commonly used is 4-8 mg/time, twice a day. The recommended individual dose of PMP is 0.375 mg to 4.5 mg per day. The initial dose for treating idiopathic Parkinson's disease is 0.375 mg per day, and then the dose is increased once every 5-7 days, with the maximum dose of 1.5 mg. The incidence of drowsiness increased when the daily dose is higher than 1.5 mg. The recommended dose of MTP is 100-200 mg/day, which may reach 300 mg/day or 400 mg/day if necessary. The known adverse reaction is hypotension.

In the test, the human dosage calculated based on the dosage of rhesus monkeys is lower than the maximum value of the recommended dosage, so in theory, the combined use of DOX+PMP+MTP of the present invention has very good safety.

During the above experiment, no adverse events related to drug administration, such as intracranial hemorrhage, were seen on magnetic resonance examination throughout the drug administration period in the all 6 animals in the DOX+PMP+MTP group, which further confirmed that the combined use of DOX+PMP+MTP of the present invention has good safety.

### 2. Example 2

The inventors evaluated, using retinal ischemia/reperfusion (I/R) rhesus monkey models, the effect of pharmaceutical compositions of doxazosin+pramipexole+metoprolol, compositions of doxazosin+metoprolol+bromocriptine, compositions of doxazosin+metoprolol, doxazosin alone, and pramipexole alone in the prevention, treatment, and/or improvement of optic neuropathy and retinopathy, particularly ischemic optic neuropathy.

The main efficacy indexes: 1) fundus photography (FP) and optical coherence tomography (OCT) were used to quantitatively and dynamically evaluate the effects on retinal structure at D7, D21, D35, and D63 after retinal I/R injury in rhesus monkeys, including the changes of retinal thickness and nerve fiber layer (RNFL) thickness; 2) flash visual evoked potential (FVEP) and full-field electroretinogram (FERG) were used to dynamically evaluate optic nerve conduction function and overall retinal function; and 3) immunohistochemistry was used to quantify the number of retinal ganglion cells (RGC) and the expression of glial fibrillary acidic protein (GFAP) and Quantitative Fluorescence Real-time PCR was used to detect inflammatory factor expression in ischemic retinopathy, at D63 after retinal I/R injury.

### 2.1 Experimental materials

### Test product 1:

Name or abbreviation (English name): Doxazosin mesylate (DOX)
Purity: 99.6% HPLC
Produced by Zhejiang Xinsaike Pharmaceutical Co., Ltd.

### Test product 2:

Name or abbreviation (English name): Pramipexole 2HCL Monohydrate (PMP)
Purity: 99.55% HPLC
Produced by Shanghai Ziqi Biotechnology Co., Ltd.

### Test product 3

Name or abbreviation (English name): Metoprolol tartrate salt (MTP)
Purity: 100% HPLC
Produced by Sun Pharmaceutical Industries Ltd., India

### Test product 4

Name or abbreviation (English name): Bromocriptine Mesilate (BRM)
Specification: 2.5 mg/tablet

Produced by Gedeon Richter Ltd. The initial clinical dose of bromocriptine mesilate was 1.25 mg, 2-3 times a day. The routine dose of bromocriptine mesilate tablets is 4-16 tablets per day (10-40 mg of bromocriptine mesilate).

### 2.2 Specific experimental scheme

### 2.2.1 Laboratory animals

23 healthy rhesus monkeys aged 4-6 years were selected, Macaca mulatta (Rhesus Macaque), general grade, provided by Primed Primate Research Center. The feeding environment grade in the experimental period: ordinary grade. Temperature: 18-26 °C. Relative humidity: 40% to 70%. Ventilation: no less than 8 air changes per hour, using 100% fresh air (no air circulation). Illumination time: automatic illumination, alternating light and dark every 12 hours. Animal cage: 850*900*2365 mm double-layer stainless steel cage. Feeding density: 1 per cage. All procedures involving animals complied with ARVO's statement on the use of animals in ophthalmic and visual research, and all operations had been reviewed and approved by the IACUC Committee of the Primed Primate Research Center.

### 2.2.2 Retinal ischemia/reperfusion injury (I/R) rhesus monkey model

The preparation of rhesus monkeys with acute panretinal ischemia/reperfusion injury (I/R) is shown in FIG. 8. After anesthesia with 8 mg/kg compound ketamine, unilateral retinal ischemia was induced in the right eye, while the contralateral eye served as non-ischemic control. One drop of compound tropicamide eye drops (Santen Pharmaceutical) was added to the right eye, and then the rhesus monkey was placed in a dark room for mydriasis. When the pupil of the right eye was larger than 6 mm, the anterior chamber was prepared to be filled with water and pressurized for modeling. 0.9% sodium chloride injection (Sichuan Kelun Pharmaceutical Co., Ltd) was injected into a 250 ml infusion bag (Sichuan Kangning Medical Equipment Co., Ltd.), the end of the infusion tube was connected with a 30G needle (BD Microlance^{®}, BD), and the needle was inserted into the anterior chamber of the right eye to inject 0.9% sterile saline. With the saline bag rising to 1.9 meters, the intraocular pressure (IOP) of the rhesus monkey rose acutely to 140 mmHg, and the ischemia lasted for 90 minutes. By observing the corneal edema, it was considered as a sign of successful induction of retinal ischemia. After ischemia was completed, the saline reservoir was slowly lowered, the needle was pulled out from the eye, and IOP was restored to form retinal reperfusion.

### 2.2.3 Grouping and administration

23 rhesus monkeys with acute retinal I/R were divided into 6 groups: I/R control group (n=6), I/R+DOX+MTP+PMP group (n=6), I/R+DOX+MTP group (n=3), I/R+DOX+MTP+BRM group (n=3), I/R+DOX group (n=3), and I/R+PMP group (n=2). Animals in each administration group were administered orally 4-5 hours after retinal I/R injury (pulling the needle out from the eye was taking as the starting point of I/R injury) for 63 consecutive days, and some animals were euthanized to complete pathology after the experiment.

The following was the dosage and frequency of each administration:
I/R control group: administered with fruit.
I/R+DOX+PMP+MTP group: DOX: 0.266 to 0.532 mg/kg; PMP: 0.002 to 0.004 mg/kg; MTP: 1.667 mg/kg; 2-3 times a day.
I/R+DOX+MTP group: DOX: 0.266 to 0.532 mg/kg; MTP: 1.667 to 3.33 mg/kg; 2-3 times a day.
I/R+DOX group: 0.266 to 0.532 mg/kg, 2-3 times a day.
I/R+PMP group: 0.002 to 0.004 mg/kg, 2-3 times a day.
I/R+DOX+MTP+BRM group: ratios: DOX: 0.266 to 0.532 mg/kg; MTP: 1.667 mg/kg; BRM: 0.03 mg/kg, 2-3 times a day.

**Table 2 Test drugs and doses thereof**

| | Drug name | Dose administered to monkeys at a time (mg/kg) | Approximate equivalence of daily human clinical dose (mg) |
|---|---|---|---|
| Test product 1 | Doxazosin (DOX) | 0.266, 0.532 | 2 to 8 |
| Test product 2 | Pramipexole (PMP) | 0.002, 0.004 | 0.0625 to 0.125 |
| Test product 3 | Metoprolol (MTP) | 1.677, 3.33 | 20 to 100 |
| Test product 4 | Bromocriptine | 0.03 | 2 |

| | | | |
|---|---|---|---|
| Note: monkey doses can be dose-converted to human doses using the body surface area method or in vivo drug concentration exposure. | | | |

### 2.3 Observation index and monitoring method

### 2.3.1 Examination of retinal thickness/retinal nerve fiber layer thickness by FP/OCT

Before modeling, both eyes were scanned with Kowa VX-20 fundus camera and Heidelberg SPECTRALIS plus OCT at Day 7 (D7), Day 21 (D21), Day 35 (D35), and Day 63 (D63) after I/R injury, respectively. The right eye was I/R injured eye and the left eye was non-ischemic control eye.

Methods: rhesus monkeys were anesthetized by intramuscular injection of 8 mg/kg compound ketamine. 1-2 drops of compound tropicamide eye drops were added to both eyes for mydriasis. If the pupil was larger than 6 mm under light after mydriasis, the fundus could be examined. The right eye and the left eye were opened sequentially with an eyelid opener, positioning with optic disc, fundus camera (Kowa VX-20, Japan) was used to take fundus photography, and then OCT examination was performed. The thickness of the EDTRS region of the retinal 9-room map and the thickness of the retinal nerve fiber layer (RNFL) in each quadrant were measured using OCT. The analysis method was shown in FIG. 10. The average thickness of RNFL was calculated by averaging the thickness values measured over 360 degrees. The thickness of each area was calculated by averaging the temporal (T; 315 to 45 degrees), superior temporal (TS; 45 to 90 degrees), inferior temporal (TI; 90 to 135 degrees), nasal (N; 135 to 225 degrees), superior nasal (NS; 225 to 270 degrees) and inferior nasal (NI; 270 to 315 degrees) measurement values, according to the instrument vendor's instructions.

### 2.3.2 Dynamic evaluation of optic nerve conduction function and overall retinal function by FERG and FVEP

Acute retinal ischemia may cause neuroretinal damage, including photoreceptors, whose function can be evaluated by FERG and FVEP. Flash visual evoked potential (FVEP) and full-field electroretinogram (FERG) were performed dynamically using an ocular electrophysiologic instrument (Roland Consult Stasche & Finger GmbH, Ganzfeld Q450 SC) at Day 7 (D7), Day 21 (D21), and Day 63 (D63) after I/R injury. All detection parameters were set with reference to the latest standards of International Society for Clinical Electrophysiology of Vision (ISCEV).

Methods: the anesthesia method was the same as that in the FP/OCT examination, and the sites that required to be connected with electrodes were sterilized with 75% alcohol. The eyelid was opened with an eyelid opener, it was recorded that the position where the electrode was placed was 2-3 cm of the occipital trochanter, the position where the reference electrode was placed was the middle of the forehead, the position where the grounding electrode was placed was the root of the tail, the non-tested eye was covered, and FVEP was recorded in the order of the right eye followed by the left eye. After the FVEP recording, the eyes were fully mydriated with compound tropicamide eye drops, the grounding electrode was connected to the middle of forehead, and the reference electrode was placed in the outer canthus of both eyes. After Benoxil was used for eye surface anesthesia, two drops of hydroxymethylcellulose sodium eye drops were dropped onto corneal contact electrodes, which were then put into both eyes to ensure complete contact with cornea. The light-adapted 3.0 ERG, light-adapted 30HZ oscillatory potentials were recorded sequentially. The recording electrode for detecting ERG was annular corneal electrode, and the reference electrode and grounding electrode were needle-like electrodes (rhesus monkey). VEP electrode was a needle-like electrode. The impedance of each electrode was less than 5 KΩ.

### 2.3.3 Immunohistochemistry and partial retinal ganglion cell count

16 rhesus monkeys were euthanized at Day 63 (D63) after retinal I/R injury, including: I/R control group (n=3, 6 eyes), I/R+DOX+MTP+PMP group (n=3, 6 eyes), I/R+DOX+MTP group (n=3, 6 eyes), I/R+DOX+MTP+BRM group (n=3, 6 eyes), I/R+DOX group (n=3, 6 eyes), and I/R+PMP group (n=1, 2 eyes).

Methods: Eyeballs were taken out and placed in F.A.A fixative solution. 72 hours later, the eyeballs were taken out, and retina was cut according to the range shown in FIG. 10 (red box). A rectangular (about 0.8 cm×1.6 cm) eyeball wall tissue including optic disc and macular area was taken for immunohistochemical staining. The sampled retina was embedded and then sliced. The fovea of the macula was continuously sliced at about 0.5 mm=500 µm, for about 100 slices (5 µm/slice), in 10 rounds (10 slices/round), and from every 50 µm (i.e., each round), one slice was taken for Brn-3a staining and GFAP protein staining continuously, and 8-10 immunohistochemical slices were taken for ganglion cell counting and GFAP protein quantification. The number of temporal and nasal ganglion cells within 1.5 mm from the fovea was counted for each slice. The operation flow is shown in FIG. 9. The number of RGC was calculated for each slice. The number of RGCs in retina of ischemic injured eye (OD) was subtracted by the number of RGC in retina of contralateral unischemic eye (OS) of the same rhesus monkey, and the result was divided by the number of RGCs in the contralateral eye (OS) to obtain the percentage of RGC loss. All quantitation procedures were conducted by two researchers under double-blind conditions.

### 2.3.4 Detection of inflammatory factor expression in ischemic retinopathy by Quantitative Fluorescence Real-time PCR

Methods: RNA was extracted from retinal tissue using a total RNA extraction kit (Foregene) and its absorbance value was measured. 2 ug of total RNA was used as a template for reverse transcription reaction, and relative quantitative PCR detection was performed with primers from Table 3, EvaGreen Express 2×qPCR MasterMix-No Dye fluorescent dye (abm), and β-Actin as an internal reference. Reaction conditions: pre-denaturation at 95°C for 10 min; 95°C for 10 S, 60°C for 30 S, totally 40 cycles; melting curve (rising from 60 °C to 95°C, and the temperature rose by 0.3°C every 15s). Primer Premier 5.0 software was used for primer design, and the primers were synthesized by Shanghai Sangon Bioengineering Technology Service Co., Ltd.

**Table 3 Primer sequence**

| Gene | Species | Direction | Primer sequence (5'-3') | Product length (bp) | Annealing temperature (°C) |
|---|---|---|---|---|---|
| β-Actin | Rhesus monkey | FORWARD | TCGTGCGTGACATTAAGGAGAAGC | 136 | 60 |
| | | REVERSE | TCGTTGCCAATGGTGATGACCTG | | |
| IFN-γ | Rhesus monkey | FORWARD | CGAATGTCCAACGCAAAGCAGTAC | 113 | 60 |
| | | REVERSE | TGCTCTTCGACCTCGAAACATCTG | | |
| IL-1β | Rhesus monkey | FORWARD | CTTACTACAGCGGCAACGAGGATG | 103 | 60 |
| | | REVERSE | CCACCACCCAGAGGGCAGAG | | |
| TNF-α | Rhesus monkey | FORWARD | AATGGCGTGGAGCTGACAGATAAC | 136 | 60 |
| | | REVERSE | CGATGCGGCTGATGGTGTGG | | |

### 2.3.5 Statistical analysis

All the results were expressed as mean±SEM. Statistical analysis was made using Graphpad Prism (8.0 version) by double-tailed T test. p<0.05 was considered to be of statistical significance.

### 2.4 Experimental results and discussion

### 2.4.1 Effect on retinal ganglion cells

Histopathological Brn3a immunohistochemistry was performed at Day 63 (D63) after retinal I/R injury to evaluate the number of retinal ganglion cells (RGC). The results of each group are shown in FIG. 13. Compared with OS eyes without ischemia injury, the average loss of RGC cells in I/R eyes (N=3) in the I/R control group at Day 63 (D63) was 75.7±7.5%. Compared with the I/R control group, the I/R+DOX+MTP+PMP group (N=3) with the I/R eyes being treated for 63 days exhibited significantly increased survival rate of retinal ganglion cells (RGC) (p<0.01), with an average loss of RGC cells being 13.4±5.5%.

The average losses of RGC cells in I/R eyes in the I/R+DOX+MTP+BRM group, I/R+DOX+MTP group, I/R+DOX, group and I/R+PMP group were 73.3±6.0%, 60.0±14.6%, 63.9±14.1% and 60.0%, respectively, having no statistically significant difference from the I/R control group.

### 2.4.2 Effect on GFAP protein expression

The results are shown in FIG. 14. The retinal GFAP protein expression in the non-ischemic OS control eyes (N=7) was quantified as 2.04±0.13, and GFAP staining was only observed in RGC and nerve fiber layer. Compared with non-ischemic OS control eyes, the retinal GFAP protein expression in the I/R eyes (N=3) in the I/R control group increased significantly to 10.66±1.41 (p<0.01), and the signal reached the inner plexiform layer. Compared with the I/R eyes in the I/R control group, the I/R+DOX+MTP+PMP group (N=3) significantly inhibited GFAP protein overexpression in the I/R eyes after 63 days of treatment (p<0.01), and GFAP was quantified as 3.29±0.36.

As could be seen from the above, after 63 days of treatment, the DOX+MTP+PMP group significantly improved the survival rate of ganglion cells (RGC) after retinal I/R injury, and significantly inhibited I/R-induced increase in GFAP protein expression in retinal tissue and inhibited neuroinflammation. At the same time, the experimental data showed that the DOX+MTP+BRM group, DOX+MTP group, DOX group, and PMP group had weak protection on retinal ganglion cells.

### 2.4.3 Changes in retinal thickness

The results of the quantitative analysis of the mean thickness of the EDTRS area of the retina in a 9-room map after I/R injury using OCT are shown in Table 4, FIG. 10 and FIG. 11. Compared with the case before modeling, the average thickness of the retinal EDTRS area of the I/R eyes (N=6) in the I/R control group decreased significantly at Day 7 (D7), Day 21 (D21), Day 35 (D35), and Day 63 (D63) (p<0.05), and decreased by 78±24 microns at Day 63 (D63), indicating severe degeneration and severe structural injury in all layers of the retina. Compared with the I/R control group, the I/R+DOX+MTP+PMP group (N=6) significantly prevented the thickness reduction in the retinal EDTRS area of the I/R eye (p<0.01). The average thickness of the retinal EDTRS area decreased by 7±6 microns, 19±13 microns, 21±15 microns, and 21±19 microns at Day 7 (D7), Day 21 (D21), Day 35 (D35), and Day 63 (D63), respectively.

In addition, compared with the case before modeling, the average thickness of retinal EDTRS area in the I/R+DOX+MTP+BRM group, I/R+DOX+MTP group, I/R+DOX group, and I/R+PMP group decreased significantly at each time point, and the magnitude of decrease at each time point was not statistically different from that in the I/R control group.

### 2.4.4 Changes in nerve fiber layer (RNFL) thickness

The OCT quantification results are shown in Table 5 and FIG. 10.

Compared with the case before modeling, the I/R eyes in the I/R control group mainly exhibited edema in the retinal NFL and RGCs layers at Day 7 (D7), then gradual thinning of retinal RNFL thickness at Day 21 (D21), Day 35 (D35), and Day 63 (D63), and at Day 63 (D63), the Global average thickness of retinal nerve fiber layer (RNFL) decreased averagely by 32±17 microns. Compared with I/R eyes in the I/R control group, I/R+DOX+MTP+PMP group significantly prevented the retinal RNFL from decreasing significantly at Day 63 (D63) of the treatment (p<0.05), and decreased averagely by 5±4 microns at Day 63 (D63).

In addition, compared with I/R eyes in the I/R control group, the average retinal RNFL Global thickness in the I/R+DOX+MTP+BRM group, I/R+DOX+MTP group, I/R+DOX group, and I/R+PMP group decreased significantly at Day 7 (D7), Day 21 (D21), Day 35 (D35), and Day 63 (D63), and the magnitude of decrease at each time point was not statistically different from that in the I/R control group.

### 2.4.5 Full-field electroretinogram (FERG) detection results

I/R induced neuronal injury and visual impairment in different retinal diseases. The light-adapted 3.0 ERG response was that the degree of abnormality when a-wave amplitude reached its peak reflected the function of the photoreceptor, and the degree of abnormality when b-wave amplitude reached its peak reflected the function of cone cells. The results are shown in Table 6. The light-adapted 3.0 ERG response a-wave and b-wave amplitudes both decreased at D7 and D21 after I/R injury in the I/R control group (N=6), compared with the uninjured eyes OS in the group, the retinal function recovered to some extent at Day 63 (D63), but still decreased significantly. There was no obvious change in the uninjured eyes during the test period.

Compared with the I/R control group, the light-adapted 3.0 ERG response a-wave and b-wave amplitudes significantly increased at Day 7 (D7) and Day 21 (D21) after I/R injury in the DOX+MTP+PMP group (N=6) (p<0.05). The composition significantly inhibited the decrease of retinal function after I/R injury. Compared with the I/R control group, the light-adapted 3.0 ERG response a-wave and b-wave amplitudes both significantly decreased in the I/R+DOX+MTP+BRM group (N=3) at each time point after injury, and there was no significant difference. The retinal function recovered somewhat at Day 63 (D63), but still decreased.

### 2.4.6 Optical visual evoked potential (FVEP) detection results

FVEP reflects the conduction function of the entire visual pathway for visual signals from retinal ganglion cells to occipital visual cortex of the brain. The results are shown in Table 7.

The P2 amplitude in the I/R eyes (N=4) in the I/R control group decreased at Day 21 (D21) and Day 63 (D63), compared with the non-injured eyes in the group. Compared with the I/R control group, the P2 amplitude (N2-P2) of I/R eyes (N=4) in the I/R+DOX+MTP+PMP group increased significantly at Day 21 (D21) and Day 63 (D63) after drug administration (p<0.05). The composition significantly improved retinal function.

### 2.4.7 Effect on inflammatory factors after I/R injury of rhesus monkey retina

The results are shown in FIG. 15. Compared with control eyes without ischemic injury (N=6), Day 63 (D63) results of I/R eyes (N=3) in the I/R control group showed significant up-regulation of TNF-α, IL-1β, and IFN-γ levels induced by retinal I/R (p<0.01 or p<0.05). Compared with I/R eyes in the I/R control group, the DOX+MTP+PMP group (N=3) significantly decreased the increase of TNF-α, IL-1β, and IFN-γ expression levels induced by retinal I/R in I/R eyes (N=3) (p<0.05), and there was no significant difference in expression levels compared with non-ischemic control eyes.

The above showed that the DOX+MTP+PMP composition protected against RGC cell loss and GFAP overexpression after panretinal I/R injury in rhesus monkeys, significantly reduced the increase of TNF-α, IL-1β, and IFN-γ expression levels caused by retinal I/R, maintained retinal thickness and reduced neuronal injury, alleviated optic nerve function reduction caused by I/R, including FERG and FVEP destruction, and preserved retinal overall function.

**Table 4 Effect on the mean thickness of the 9-room retinal EDTRS area after retinal ischemia-reperfusion (I/R) in rhesus monkeys (unit: µm)**

| Eyeball | Group | N | Before modeling (baseline) | Day 7 | | Day 21 | | Day 35 | | Day 63 | |
|---|---|---|---|---|---|---|---|---|---|---|---|
| | | | | Δ | P | Δ | P | Δ | P | Δ | P |
| OD (I/R injury) | I/R control group | 6 | 320±17 | -41±18 | / | -64±19 | / | -73±23 | / | -78±24 | / |
| | I/R+DOX+MTP+PM P | 6 | 308±9 | -7±6 | 0.003 | -19±13 | 0.001 | -21±15 | 0.002 | -21±19 | 0.003 |
| | I/R+DOX+MTP+BR M | 3 | 332±20 | -58±8 | 0.209 | -108±5 | 0.011 | -115±6 | 0.016 | -117±6 | 0.085 |
| | I/R+DOX+MTP | 3 | 305±10 | -34±11 | 0.653 | -61±7 | 0.793 | -67±9 | 0.707 | -72±11 | 0.709 |
| | I/R+DOX | 3 | 325±13 | -26±0 | 0.260 | -48±7 | 0.250 | -54±10 | 0.275 | -58±9 | 0.260 |
| | I/R+PMP | 2 | 327±4 | -50±14 | / | -79±15 | / | -85±14 | / | -88±18 | / |
| OS (Non-ischemic contralateral eye) | Control group | 6 | 321±18 | -4±4 | / | -3±5 | / | -5±7 | / | -6±8 | / |
| | DOX+MTP+PMP | 6 | 308±10 | -2±2 | 0.442 | -2±1 | 0.607 | -2±2 | 0.261 | -2±3 | 0.372 |
| | DOX+MTP+BRM | 3 | 334±19 | -4±4 | 0.864 | -4±3 | 0.901 | -4±2 | 0.318 | -6±2 | 0.747 |
| | DOX+MTP | 3 | 304±10 | 0±1 | 0.222 | -1±1 | 0.570 | -2±2 | 0.492 | -2±3 | 0.539 |
| | DOX | 3 | 324±11 | -5±4 | 0.698 | -3±4 | 0.904 | -3±3 | 0.586 | -3±5 | 0.678 |
| | PMP | 2 | 325±3 | -3±5 | / | -6±3 | / | -2±4 | / | 3±3 | / |

| | | | | | | | | | | | |
|---|---|---|---|---|---|---|---|---|---|---|---|
| Note: 1. Δ: Retinal change value: the average thickness of retinal EDTRS area after drug administration (micron)-the value before modeling (micron); "negative numbers" means decrease in retinal thickness, and "positive numbers" means thickening of retina | | | | | | | | | | | |

**Table 5 Effect on the Global mean thickness of retinal nerve fiber layer (RNFL) after retinal ischemia-reperfusion (I/R) in rhesus monkeys (unit: µm)**

| Eyeball | Group | N | Before molding (baseline) | Day 35 | | Day 63 | |
|---|---|---|---|---|---|---|---|
| | | | | Δ | P | Δ | P |
| OD (I/R injury) | I/R control group | 6 | 109±7 | -34±19 | / | -32±17 | / |
| | I/R+DOX+MTP+PMP | 6 | 102±7 | -3±4 | 0.005 | -5±4 | 0.013 |
| | I/R+DOX+MTP+BRM | 3 | 106±1 | -34±18 | 0.239 | -35±19 | 0.853 |
| | I/R+DOX+MTP | 3 | 107±1 | -20±14 | 0.581 | -21±15 | 0.760 |
| | I/R+DOX | 3 | 111±7 | -14±4 | 0.846 | -21±7 | 0.385 |
| | I/R+PMP | 2 | 106±1 | -41±8 | / | -46±3 | / |
| OS (Non-ischemic contralateral eye) | Control group | 6 | 110±8 | 0±1 | / | 1±1 | / |
| | DOX+MTP+PMP | 6 | 101±8 | 1±1 | 0.401 | 1±2 | 0.874 |
| | DOX+MTP+BRM | 3 | 108±3 | -1±1 | 0.177 | -1±2 | 0.399 |
| | DOX+MTP | 3 | 106±4 | 2±2 | 0.135 | 2±4 | 0.625 |
| | DOX | 3 | 109±8 | 1±2 | 0.733 | 1±1 | 0.502 |
| | PMP | 2 | 108±0 | -2±1 | / | 2±1 | / |

| | | | | | | | |
|---|---|---|---|---|---|---|---|
| Note: 1. Δ: RNFL change value: the average thickness of RNFL after drug administration (micron)-the value before modeling (micron); "negative numbers" means decrease in RNFL thickness, and "positive numbers" means thickening of RNFL. | | | | | | | |

**Table 6 Effect on FERG after retinal ischemia-reperfusion (I/R) in rhesus monkeys**

| Eyeball | Group | N | a wave (µV) | | | b wave (µV) | | |
|---|---|---|---|---|---|---|---|---|
| | | | Day / | Day 21 | Day 63 | Day / | Day 2 1 | Day 63 |
| OD (I/R injury) | I/R control group | 6 | 8.2±5.4 | 7.2±5.6 | 10.3±5.8 | 24.3±13.1 | 26.5±14.6 | 33.2±22.1 |
| | I/R+DOX+MTP+PMP | 6 | 17.1±6.0* | 20.0±7.4* | 13.9±6.3 | 51.7±17.3* | 57.6±25.6* | 47.9±21.0 |
| | I/R-DOX +MTP+BRM | 3 | 4.8±0.6 | 6.0±1.8 | 10.2±3.0 | 11.2±7.4 | 13.34±2.6 | 26.3=1.6 |
| OS (Non-ischemic contralateral eye) | Control group | 6 | 22.2±8.1 | 17.0±4.3 | 18.7±4.8 | 58.8±10.2 | 55.9+8.0 | 60.4±11.3 |
| | DOX+MTP+PMP | 6 | 18.1±5.0 | 19.9+6.0 | 16.4±3.8 | 63.0±12.3 | 65.9±20.0 | 58.4±12.3 |
| | DOX+MTP+BRM | 3 | 23.5±2.1 | 24.8+2.1 | 19.3±5.9 | 55.6±4.3 | 65.5±3.4 | 50.6±17.4 |

| | | | | | | | | |
|---|---|---|---|---|---|---|---|---|
| Note: 1. *, p<0.05 compared with **the I/R control group.** | | | | | | | | |

**Table 7 Effect on FVEP after retinal ischemia-reperfusion (I/R) in rhesus monkeys**

| Eyeball | Group | N | N2-P2 (µV) | | | P2 (ms) | | |
|---|---|---|---|---|---|---|---|---|
| | | | Day 7 | Day 21 | Day 63 | Day 7 | Day 21 | Day 63 |
| OD (I/R injury) | I/R control group | 4 | 32.5±11.6 | 13.8±7.1 | 10.0±3.8 | 122.8±9.0 | 123.2±13.7 | 120.0±11.1 |
| | I/R+DOX+MTP+P MP | 4 | 58.7±20.4 | 52.0±17.5* | 51.9±2.38* | 112.3±9.5 | 112.6±8.0 | 116.2±12.1 |
| OS (Non-ischemic contralateral eye) | Control group | 4 | 34.2±14.1 | 40.9±19.5 | 23.4±9.3 | 119.8±6.2 | 112.7±7.5 | 117.3±3.6 |
| | DOX+MTP+PMP | 4 | 46.5±8.6 | 47.4±18.8 | 51.9±18.9 | 121.3±6.4 | 114.7±9.9 | 116.7±11.7 |

| | | | | | | | | |
|---|---|---|---|---|---|---|---|---|
| Note: 1. *, p<0.05 compared with **the I/R control group.** | | | | | | | | |

### 3. Example 3

The inventors evaluated the prevention, treatment, and/or improvement effects of doxazosin (DOX), pramipexole (PMP), and metoprolol (MTP) in combination on neurodegenerative diseases, using models of cognitive dysfunction caused by reperfusion after 180-minute middle cerebral artery M1 occlusion in rhesus monkeys. Reperfusion after 180-minute M1 segment occlusion in middle cerebral artery in rhesus monkeys may form a wider range of infarction, showing an edema/infarction volume accounting for about 70% of the hemisphere at the peak of edema, which may lead to long-term obvious cognitive decline, and may be used to evaluate the efficacy of neuroprotective agents in preventing and improving cognitive function. Evaluation indexes include:
1) using 3.0 T GE MRI to dynamically observe edema/infarction trajectories at 3-4 h, 72-75 h, Day 7, Day 14, and Day 28 after reperfusion; MRI imaging sequence including T1WI, T2 FLAIR, DWI, MRA, and DSC, wherein T1WI may show the anatomical structure of brain, differentiate between gray matter area and white matter area, diagnose hemorrhage and ischemia, etc., T2 FLAIR and DWI may quantify edema/necrosis volume, and T2 FLAIR may suggest increased water content within the tissue, and is used to show inflammation, ischemia, edema and other lesions; and DSC may show the cerebral tissue perfusion condition and sensitive response to cerebral ischemia area after acute ischemic stroke, and may quantitatively evaluate the volume of ischemic core area of brain tissue;
2) performing MRA scanning 3-4 hours after stroke (reperfusion after ischemia for 180 minutes), which accurately shows whether blood vessels are reperfused and whether collateral circulation is formed, wherein DWI shows ischemic infarction lesions in anterior circulation middle cerebral artery similar to those in clinical patients 3-4 hours after stroke, and they are used as the criteria for model success, reducing variability of the models;
3) employing the design in which the composition is administered 7 hours after stroke, which can be transformed to clinical practice better than prophylactic administration;
4) according to NHPSS, dynamically evaluating improvement for neurologic deficits at 16 h, 24 h, 48 h, 72 h, 96 h, Day 5, Day 7, Day 14, Day 21, and Day 28 after stroke; and
5) performing spatial delayed response test 3 months after stroke to evaluate the improvement of cognitive dysfunction.

### 3.1 Experimental materials

The experimental materials were the same as those described in Example 1.1.

### 3.2 Specific experimental scheme

### 3.2.1 Laboratory animals

9 healthy rhesus monkeys (*Macaca mulatta (Rhesus Macaque)*) aged 5-7 years, general grade, weight: 6-9 kg, provided by Primed Primate Research Center, were selected. The feeding environment grade in the experimental period: ordinary grade. Temperature: 18-26 °C. Relative humidity: 40% to 70%. Ventilation: no less than 8 air changes per hour, using 100% fresh air (no air circulation). Illumination time: automatic illumination, alternating light and dark every 12 hours. Animal cage: 850*900*2365 mm double-layer stainless steel cage. Feeding density: 1 per cage. All operations had been reviewed and approved by the IACUC Committee of the Primed Primate Research Center.

### 3.2.2 Preparation of rhesus monkey 180-minute middle cerebral artery occlusion reperfusion models (MCAO)

The method of making MCAO models was the same as that described in Example 1.2.2, except that the time of clipping M1 segment of middle cerebral artery in Example 3 was 180 minutes.

### 3.2.3 Grouping and administration

9 model animals formed by reperfusion after 180-minute MCAO in rhesus monkeys were selected and quantified by DWI for edema volume 3-4 hours after reperfusion, and grouped. 9 rhesus monkeys were scanned by 3.0 T GEMI 3h-4h after 180 minute-MCAO reperfusion. The bilateral middle cerebral arteries were obviously visible by magnetic resonance angiography (MRA) and CT angiography (CTA), and it was determined that reperfusion succeeded (see FIG. 17). 9 MCAO rhesus monkeys were divided into model control group (5) and DOX+PMP+MTP group (4). The mean volumes of edema/infarction areas quantified by DWI in the two groups were 14.61±2.63 cm³ and 13.43±3.14 cm³. There was no statistical difference between the two groups.

Animals in the administration group were administered orally about 7 hours after stroke (i.e., the time point of implementing aneurysm clipping artery was the time point of stroke attack), and were administered 2-4 times a day at intervals for 60 consecutive days. The following was the dosage and mode of each administration:
Model control group (Placebo group): administered with empty capsules.
DOX+PMP+MTP treatment group: DOX: 0.266 mg/kg to 0.532 mg/kg; PMP: 0.002 to 0.004 mg/kg; MTP: 1.667 mg/kg to 3.334 mg/kg.

### 3.2.4 Test procedure

MRI scans were performed at 3-4 h, 72-75 h, Day 7, Day 14, and Day 28 after MCAO-180 minute reperfusion to measure brain edema/infarction volume, and neurobehavioral scoring (NHPSS) was performed. At the same time, in order to investigate long-term neurological deficit, spatial delayed response test was used 3 months after stroke to evaluate cognitive function.

### 3.3 Observation index and monitoring method

### 3.3.1 Main efficacy indexes

### 3.3.1.1 Brain tissue scanning and quantification of brain edema/infarction volume

Same as described in Example 1.3.1.1.

### 3.3.1.2 NHPSS neurobehavioral score

Same as described in Example 1.3.1.2.

### 3.3.1.3 Quantitative evaluation of perfusion of brain tissue microcirculation and sensitive response cerebral ischemia area

Dynamic susceptibility contrast enhanced magnetic resonance imaging (MRI-DSC) is an examination method of obtaining a series of dynamic images by using rapid scanning sequence imaging when a paramagnetic contrast agent administered via intravenous bolus injection passes through the examined tissue for the first time. It is a functional MRI imaging technique reflecting the distribution of tissue microvessels and blood perfusion, and provides an important imaging basis for patients with ischemic cerebrovascular disease to choose a therapeutic regimen and determine the efficacy of treatment.

MRI-DSC imaging, after workstation post-processing, analysis and calculation of cerebral hemodynamic parameters, can produce four main indexes reflecting regional blood supply: 1) Regional cerebral blood volume (rCBV) refers to blood volume present in a certain amount of brain tissue vascular structure. 2) Regional cerebral blood flow (rCBF) refers to blood flow through a certain amount of brain tissue vascular structure in a unit time. The smaller the value, the lower the brain tissue blood flow. 3) Regional mean transit time (rMTT) refers to the time it takes for a contrast agent to pass through the capillaries, when blood flows through the vascular structure (the time it takes for blood to flow in from the arteries to flow out of the veins). This value being large indicates that microcirculation is not smooth. 4) Regional Time to peak (rTTP) refers to the time it takes for a contrast agent to reach the peak in ROI in brain. The larger the value, the later the peak of the contrast agent reaches the brain tissue. 75 hours after stroke, the volume of ischemic core area was quantified in MRI-DSC imaging to evaluate the curative effect of improving ischemia.

The area where CBV and CBF decreased significantly and MTT and TTP prolonged significantly was selected as a non-perfusion area (ischemic core area) (see FIG. 18, dark region of CBV, CBF, MTT and TTP). Advantage Workstation 4.7 workstation software for GE magnetic resonance was used to determine the area of the nonperfused area in each layer of the scanned CBV, and the cerebral ischemic volume could be obtained by summing the area of ischemic area of each layer and multiplying the sum by the layer thickness. (Wintermark M, Sesay M, Barbier E, et al. Comparative overview of brain perfusion imaging techniques.[J]. Journal of Neuroradiology, 2005, 32(9):294-314.; Shih L C, Saver J L, Alger J R, et al. Perfusion-Weighted Magnetic Resonance Imaging Thresholds Identifying Core, Irreversibly Infarcted Tissue[J]. Stroke, 2003, 34(6):1425-1430.; Oppenheim C, Peeters A, Cosnard G, et al. Which MR-derived perfusion parameters are the best predictors of infarct growth in hyperacute stroke? Comparative study between relative and quantitative measurements.[J]. Radiology, 2002, 223(2):361-370.; JING Yanping, LUO Bin, GAO Zhengrong, et al. Application value of DSC-PWI in ischemic cerebrovascular diseases [J], Journal of Chinese Physician, 2020, 22(3): 6).

### 3.3.1.4 Cognitive function test

The cognitive functions of monkeys were evaluated by Spatial Delayed Response testing, including learning and memory ability, visuospatial ability and ability to understand and judge (Bo, Zhang, Fei, et al. Chronic phencyclidine treatment impairs spatial working memory in rhesus monkeys.[J]. Psychopharmacology, 2019.; Buccafusco J J . Working Memory Delayed Response Tasks in Monkeys -- Methods of Behavior Analysis in Neuroscience[J]. CRC Press, 2009.).

A modified Wisconsin device (as shown in FIG. 16) was used for spatial delayed response testing. Food was randomly placed in one of two containers under the monkey's watchful eye, a cover was put thereon, and then a baffle was lowered to block the monkey's view. After a certain time delay, the animal was allowed to choose the container with food in it. Animals had to memorize in which container the food was placed in order to make a right choice and get the food. The delay time was 1s, 5s, 10s, and 30s respectively. Three months after stroke, 6 animals from the model control group (N=3) and DOX+PMP+MTP group (N=3) were tested 30 times a day. The delay time was extended sequentially if the animals had a correct rate of 80% or more in the tests in 2 consecutive days. The curative effect indexes were cognitive function indexes, i.e., the correct rate of the test completed at different delay times and the longest time interval that memory could reach.

In previous studies, the inventors selected 10 healthy rhesus monkeys aged 5-7 years to complete the spatial delayed response testing, and the longest delay time of all animals was ≥30s. When the interval time was 30s or more, the animal's inattention affected the accuracy of the test, so in this example, the inventors selected the test with a maximum delay of 30 seconds.

### 3.3.2 Caring of rhesus monkeys

Same as described in Example 1.3.2.

### 3.3.3 Data analysis

Same as described in Example 1.3.3.

### 3.4 Experimental results and discussion

### 3.4.1 Effect on cerebral edema/infarction volume

The results are shown in FIG. 17.

Model control group (N=5): DWI of the surgical side (right side) 3-4 hours after reperfusion showed a mean volume of cerebral edema/infarction of 14.61±2.63 cm³. T2 FLAIR after stroke showed that the peak of edema was reached 75 h later, and the average volume of cerebral edema/infarction was 29.77±2.82 cm³. The volume of edema accounted for about 70% of the hemisphere volume (the whole brain volume of an adult rhesus monkey is about 90 cm³), with a significant midline shift. MRI imaging examination showed lesions in frontal lobe, parietal lobe, temporal lobe and occipital lobe on the surgical side (right side). At Day 7 (D7) after stroke, T2 FLAIR showed that the average volume of brain edema/infarction was 25.91±4.2 cm³, and the volumes of brain tissue edema/infarction decreased gradually from Day 14 (D14) to Day 28 (D28) after stroke, which were 16.87±3.60 cm³ and 12.02±3.71 cm³, respectively. In addition, at D28, the boundary of brain tissue necrosis area was clearer, and the necrosis area included cognition and memory-related brain areas such as temporal lobe and hippocampal area. Moreover, due to the involvement of the anterior gyrus of the brain, the functions of the contralateral hand were affected, especially the function of opposite finger movement of the thumb and index finger.

DOX+PMP+MTP treatment group (N=4): DWI of the surgical side (right side) 3-4 hours after reperfusion showed a mean volume of cerebral edema/infarction of 13.43±3.14 cm³. Four MCAO rhesus monkeys were administered with drugs orally about 7 hours after stroke, and were treated continuously for 60 days. Compared with the model control group, the brain tissue edema/infarction volume of the surgical side (right side) decreased significantly at 75 h, Day 7, Day 14, and Day 28 after stroke (p<0.05 or p<0.01). The mean volumes of T2 FLAIR brain edema/infarction at 75 h and Day 7 (D7) after stroke were 17.56±5.95 cm³ and 15.05±5.17 cm³, and the mean volumes of T2 FLAIR brain edema/infarction at Day 14 (D14) and Day 28 (D28) after stroke were 8.16±2.12 cm³ and 5.92±2.01 cm³.

### 3.4.2 Quantitative evaluation of volume of cerebral ischemic core area

The results are shown in FIG. 18.

MRI-DSC scan on the brains of normal animals showed that the perfusion conditions of CBF, CBV, MTT, and TTP in the left and right hemispheres were the same, and there was no ischemia.

Four animals in the model control (Placebo) group underwent DSC scanning at 75 h after stroke, and obvious non-perfusion area could be observed on the surgical side (right side). The volume of the non-perfusion area (ischemic core area) measured in CBV image was 17031±6355 pixel² (3.41±1.27 cm³ after conversion).

Four animals in the DOX+PMP+MTP treatment group underwent DSC scanning at 75 h after stroke. The volume of the non-perfusion area measured in CBV image of the surgical side (right side) was 4747±3210 pixel² (0.95±0.64 cm³ after conversion). The volume of the non-perfusion area was significantly smaller than that in the model control group (P<0.05), which showed that DOX+PMP+MTP significantly reduced the volume of ischemic core area 75 h after stroke, and significantly improved the ischemic state in acute stage after stroke.

The above pixel value (pixel²) was converted to volume (cm³) by the formula: volume of rCBV non-perfusion area (cm³) = pixel value (pixel²) ÷ 1000 × 0.2.

Explanation of conversion formula: Advantage Workstation 4.7 of GE MRI was used for image processing. In each scanning layer image, the boundary of the high signal region of the brain tissue on the surgical side was manually delineated as the region of interest (ROI). The software automatically calculated the area of ROI, and its unit was pixel². When the unit was cm², the numerical value was 1000 times larger than the numerical value when the unit was pixel². Therefore, in the conversion formula, the pixel value (pixel²) was divided by 1000. The layer thickness of each layer of scanned image was 0.2 cm, and the volume of cerebral infarction was obtained by multiplying the ROI area of each layer by the layer thickness. Therefore, in the conversion formula, the volume of cerebral infarction was obtained by multiplying the ROI area of each layer by 0.2.

### 3.4.3 Effect on NHPSS neurobehavioral score

The results were shown in FIG. 19.

Model control group (N=5): NHPSS scores were 16.8±1.8, 16.0±1.4 and 10.4±1.3 at 24 h, 72 h and Day 7 (D7) after stroke, which mainly showed weak defense response, decreased strength of upper and lower limbs on the contralateral side (affected side) compared with the healthy limbs, weakness of the affected arm preventing it from lifting above the head, bradykinesia of the affected hand, and a significant reduction in the ability to balance and make rotational movement. The NHPSS scores from Day 14 (D14) to Day 28 (D28) after stroke decreased gradually, which were 6.4±0.9 and 6.2±1.3, respectively, and the fine function of fingers and arm strength on the affected side (left side) of MCAO rhesus monkeys at Day 28 (D28) were still worse than those on the healthy side (right side). Three months after stroke, 5 animals lost the opposite finger movement of thumb and index finger on the affected hand, and could not complete the fine task of peeling peanuts with both hands cooperatively.

DOX+PMP+MTP group (N=4): the NHPSS scores at 24 h, 72 h and D7 after stroke were 14.3±2.9, 11.5±1.9 and 6.0±1.8, which were significantly lower than those of the model control group at the same time point (p<0.05 or p<0.01). The NHPSS scores from Day 14 (D14) to Day 28 (D28) after stroke were 4.0±0.8 and 2.5±1.3, respectively. Three months after stroke, 4 animals were not affected in terms of the opposite finger movement of thumb and index finger on the affected hand, and could complete the fine task of peeling peanuts with both hands.

### 3.4.4 Effect on cognitive ability

The results are shown in Table 8.

Three rhesus monkeys in the model control group underwent the spatial delayed response testing 3 months after stroke, and their cognitive ability decreased significantly. Two of the animals failed the test with a correct rate of 0% in the 1s delay test, suggesting that their cognitive ability was significantly impaired, including memory, visual space, and comprehension judgment. At the same time, massive necrosis of hippocampus and temporal lobe cortex, which were important cognitive function-related areas, on the affected side (right side) could be observed in MRI scan of two of the animals at Day 28 (D28). The remaining one animal could pass the 1-second delay test, but failed the 5-second delay test (with a correct rate of 63%).

Three animals in the DOX+PMP+MTP group successfully completed the 1s, 5s, 10s and 30s delay task tests in the spatial delayed response testing 3 months after stroke, suggesting that DOX+PMP+MTP treatment for 60 days after stroke could significantly improve the cognitive ability of stroke animals (P<0.05 vs. the model control group).

60 days of oral administration of DOX+PMP+MTP composition in models of cognitive dysfunction due to recanalization for 180-minute middle cerebral artery occlusion in rhesus monkeys could significantly reduce brain edema/infarction volume and neurobehavioral score, significantly improve cognitive function, including memory, visual space and comprehension judgment, and prevent and impede progression of dementia.

### 3.4.5 Safety tolerance

The safe dose ranges and toxic side effects of DOX, PMP and MTP are known. The dosage of DOX tablets commonly used is 4-8 mg/time, twice a day. The recommended individual dose of PMP is 0.375 mg to 4.5 mg per day. The initial dose for treating idiopathic Parkinson's disease is 0.375 mg per day, and then the dose is increased once every 5-7 days, with the maximum dose of 1.5 mg. The incidence of drowsiness increased when the daily dose is higher than 1.5 mg. The recommended dose of MTP is 100-200 mg/day, which may reach 300 mg/day or 400 mg/day if necessary. The known adverse reaction is hypotension.

In the test, the human dosage calculated based on the dosage of rhesus monkeys is lower than the maximum value of the recommended dosage, so in theory, the combined use of DOX+PMP+MTP of the present invention has very good safety.

During the above experiment, no adverse events related to drug administration, such as intracranial hemorrhage, were seen on magnetic resonance imaging examination throughout the drug administration period in the all 4 animals in the DOX+PMP+MTP group, which further confirmed that the combined use of DOX+PMP+MTP of the present invention had good safety, and will not cause or aggravate the risk of bleeding. In clinical application, the treatment method of the present invention can be combined with intravenous rt-PA thrombolysis or/and intravascular treatment for treating subjects.

**Table 8 Results of spatial delayed response testing in rhesus monkeys**

| Group | Serial number of animal | 1s delay | | 5s delay | | 10s delay | | 30s delay | |
|---|---|---|---|---|---|---|---|---|---|
| | | Number of tests | Correct rate | Number of tests | Correct rate | Number of tests | Correct rate | Number of tests | Correct rate |
| Model control group (n=3) | M1 | 90 times | 0% | / | / | / | / | / | / |
| | M2 | 60 times | 83% | 90 times | 63% | / | / | / | / |
| | M3 | 90 times | 0% | / | / | / | / | / | / |
| DOX+PMP+MTP Group (n=3) | M6 | 60 times | 92% | 60 times | 90% | 60 times | 82% | 90 times | 83% |
| | M7 | 60 times | 83% | 60 times | 84% | 60 times | 81% | 90 times | 80% |
| | M8 | 60 times | 85% | 60 times | 81% | 60 times | 85% | 90 times | 81% |
| P (Chi-square test vs. model control group) | | 0.083 | | 0.014 | | 0.014 | | 0.014 | |

| | | | | | | | | | |
|---|---|---|---|---|---|---|---|---|---|
| Note: /, not tested | | | | | | | | | |

### 4. Example 4

The inventors created retinal vein occlusion (RVO) rhesus monkey animal models using laser photocoagulation, and observed the changes in the structure of the retina and the retinal nerve fiber layer (RNFL) to evaluate the effect of the pharmaceutical composition of doxazosin + pramipexole + metoprolol in reducing the degree of retinal edema and protecting the optic nerve, so as to evaluate the effect of the pharmaceutical composition in preventing, treating and/or improving retinal vein occlusion.

### 4.1 Experimental materials

The experimental materials were the same as those described in Example 2.1.

### 4.2 Specific experimental scheme

### 4.2.1 Laboratory animals

6 healthy rhesus monkeys (*Macaca mulatta (Rhesus Macaque)*) aged 4-6 years, general grade, provided by Primed Primate Research Center, were selected. The feeding environment grade in the experimental period: ordinary grade. Temperature: 18-26 °C. Relative humidity: 40% to 70%. Ventilation: no less than 8 air changes per hour, using 100% fresh air (no air circulation). Illumination time: automatic illumination, alternating light and dark every 12 hours. Animal cage: 850*900*2365 mm double-layer stainless steel cage. Feeding density: 1 per cage. All operations involving animals complied with ARVO's statement on the use of animals in ophthalmic and visual research, and all operations had been reviewed and approved by the IACUC Committee of the Primed Primate Research Center.

### 4.2.2 Creation of RVO rhesus monkey animal models by laser

Six healthy male rhesus monkeys aged 3-5 years were first subjected to slit lamp, fundus examination, OCT examination and fundus fluorescence angiography, and after confirming the absence of ocular disorders, the superior trunk vein around the optic disc of the left eye of the rhesus monkey was closed using yellow light of laser to make a RVO model, and a fundus fluorescence angiography was performed immediately to confirm the formation of RVO. Examination was made 7 days, 14 days, and 42 days after surgery.

Modeling method: after anesthesia by intramuscular injection of ketamine hydrochloride (20 mg/kg) and dexmedetomidine (0.015 mg/kg), one drop of compound tropicamide eye drops was added to each eye for mydriasis. After both eyes were fully mydriated, the rhesus monkeys were placed prone in front of the laser (Vitra 532nm, Quantel Medical), with their heads fixed on a head frame, and on the modeled eye, a hand-held funduscope (Area Centralis, Volk Optical, Inc.) was used and the slit lamp (TOKA TSL-5, Wenzhou Raymond Photoelectricity Tech. Co., Ltd.) was adjusted to view the retinal structure of the monkey. Laser photocoagulation started from the distal end of the superior retinal branch vein (about 2 optic disc diameters from the edge of optic disc) and gradually moved to the proximal end, and the laser occlusion length was about 1 optic disc diameter. Success was marked by whitening of blood vessels, no blood reflux, and tortuous dilatation of distal end of veins. After surgery, fundus photography and angiography were performed to confirm that the superior retinal branch vein was completely occluded. The results are shown in FIG. 20.

### 4.2.3 Grouping and administration

The RVO models created by laser photocoagulation in the left eyes of 6 rhesus monkeys were examined, by FFA, to be successful. RVO rhesus monkey models showed typical signs such as retinal hemorrhage, cystic edema and no perfusion area after surgery, which were similar to those in clinical patients. They were divided into two groups 3-5 hours after surgery, and were administered orally for 42 consecutive days. The dosage and frequency of the composition were as follows:
Placebo group (n=3, 3 eyes): administered with fruit.
DOX+PMP+MTP group (n=3, 3 eyes): DOX: 0.266 to 0.532 mg/kg; PMP: 0.002 to 0.004 mg/kg; MTP: 1.667 mg/kg; 2-3 times a day.

### 4.3 Observation index and monitoring method

### 4.3.1 Main efficacy indexes

The main efficacy indexes included: (1) OCT quantification evaluation of retinal edema around the occlusion site; (2) OCT quantification evaluation of changes in the thickness of nerve fiber layer (RNFL) around the optic disc; (3) FP examination of retinal hemorrhage, venous tortuosity, dilatation, cotton wool spots, hard exudation, optic disc edema, vascular sheathing, etc.; and (4) FFA examination of fluorescence leakage of retinal vessels, non-perfusion area of retina, decrease in retinal capillaries, etc.

Detection frequency: before and immediately after modeling, 7 days, 14 days, and 42 days after modeling.

Detection methods: description on the FP and OCT methods were the same as that in FP/OCT examination of retinal thickness/retinal nerve fiber layer thickness in Example 2.

The specific steps of FFA fundus fluorescein angiography were as follows: after animals were anesthetized, sterilizing the skin of lower limbs, penetrating a syringe with 4 ml normal saline into the vein of the lower limb on the dorsal part close to the ankle j oint, after blood reflux was confirmed, replacing it with 3ml of 20% fluorescein sodium for rapid injection, and then acquiring angiographic pictures at different times and sites.

### 4.3.2 Statistical analysis

All the results were expressed as Mean±SD by statistical treatment. Statistical analysis was made using Graphpad Prism (8.0 version) by double-tailed T test, and p<0.05 was considered to be of statistical significance.

### 4.4 Experimental results and discussion

### 4.4.1 Results of FP and FFA examination

FP and FFA were performed immediately after modeling, the results showed that RVO rhesus monkey models were successfully created by laser photocoagulation in the superior branch trunk vein around the optic disc of the left eye; fundus color photographs showed obvious venous dilatation and tortuosity in retina, whitening of the vein, and no blood reflux; and FFP fluorescein angiography showed delayed filling and fluorescence leakage near the vein occlusion area. See FIG. 20.

Placebo group: at Day 7 of administration (modeling D7), retinal flame-shaped hemorrhage, venous dilatation and large-area capillary fluorescence leakage around the non-perfusion area and occlusion site could be observed by FP and FFA examinations in the placebo group, which were similar to those in RVO patients.

DOX+MTP+PMP group: at Day 7 of treatment of RVO rhesus monkeys, fluorescence leakage of capillaries around the retinal occlusion site, which was obviously milder than that in the placebo group, and retinal flame-shaped hemorrhage (milder than that in the placebo group) could be observed. See FIG. 21.

### 4.4.2 OCT quantification evaluation of retinal edema around occlusion site

The results are shown in FIG. 22.

At Day 14 of administration, retinal edema around the occlusion site of superior retinal branch vein was severe in the placebo group, and the thickness increased on average by 229±11 µm compared with that before modeling; and compared with the placebo group, DOX+MTP+PMP group significantly reduced retinal edema around the occlusion site of superior retinal branch vein (p<0.05) after 14 days treatment, and the thickness increased on average by 160±24 µm compared with that before modeling.

### 4.4.3 OCT quantification evaluation of retinal nerve fiber layer (RNFL)

The results are shown in FIG. 23.

At Day 42 of administration, the average thickness of retinal nerve fiber layer (RNFL) in the placebo group (N=3) decreased by 10±4 µm compared with that before modeling. Compared with the placebo group, the DOX+MTP+PMP group (N=3) significantly inhibited the decrease of RNFL (p<0.05) at Day 42 of administration, and the average thickness of RNFL decreased by only 2±2 µm compared with that before modeling.

The above showed that DOX+MTP+PMP treatment of RVO rhesus monkeys for 42 days significantly reduced RVO-induced retinal edema at the occlusion site, protected the retinal structure, inhibited the reduction of RNFL, and showed protective effect on the optic nerve.

### 5. Example 5

The inventors evaluated the efficacy and safety tolerance of the pharmaceutical composition of doxazosin + pramipexole + metoprolol for diabetic peripheral neuropathy by using neuroelectrophysiological examination (electromyograph)-nerve conduction function examination through 58 days of treatment for rhesus monkey experimental models with spontaneous diabetic peripheral neuropathy. In the experiment, epalrestat was used as positive control.

### 5.1 Experimental materials

**Table 9 Test drugs and doses thereof**

| | Drug name | Mode of action | Dose for monkeys (mg/kg) | Approximate equivalence of human clinical dose (mg) |
|---|---|---|---|---|
| Test product 1 | Doxazosin (DOX) | α1-AR blocker | 0.133, 0.266 | 1 to 4 |
| Test product 2 | Pramipexole (PMP) | D3 receptor agonist | 0.002, 0.004 | 0.0625 to 0.125 |
| Test product 3 | Metoprolol (MTP) | β receptor blocker | 0.833, 1.677 | 10 to 50 |

| | | | | |
|---|---|---|---|---|
| Note: monkey doses can be dose-converted to human doses using the body surface area method or in vivo drug concentration exposure. | | | | |

### Test product 1:

Name or abbreviation (English name): Doxazosin mesylate (DOX)
Purity: 98% HPLC
Produced by Shanghai Ziqi Biotechnology Co., Ltd.

### Test product 2:

Name or abbreviation (English name): Pramipexole 2HCL Monohydrate (PMP)
Purity: 99.55% HPLC
Produced by Shanghai Ziqi Biotechnology Co., Ltd.

### Test product 3

Name or abbreviation (English name): Metoprolol tartrate salt (MTP)
Purity: 98% HPLC
Produced by Aladdin (Shanghai) Reagent Co., Ltd.
Positive control drug
Name or abbreviation: epalrestat
Batch No.: RS20200323
Physical and chemical properties: white powder
Purity: 98%

Produced by TOKYO CHEMICAL INDUSTRY CO., LTD.

### 5.2 Specific experimental scheme

### 5.2.1 Laboratory animals

Animal species: Rhesus monkeys, Macaca mulatta (Rhesus Macaque)
Grade: ordinary grade. The rhesus monkeys were qualified in quarantine prior to testing, including physical examination, 2 tests of Mycobacterium tuberculosis, parasite, Salmonella, Shigella, and B virus examination.
Animal identification: stainless steel number plate engraved with Arabic numerals on neck ring and tattoo on chest
Supplier: Ya'an Primed Biotechnology Co., Ltd. (Primed Primate Research Center)
Production License No.: SCXK (Sichuan) 2019-027

The test system followed the requirements of AAALAC

### 5.2.2 Selection criteria for experiment

A total of 20 rhesus monkeys with spontaneous DPN were selected for the experiment. The selection criteria were as follows:
1) 20 males or females aged 13 to 25 years (equivalent to human adults aged 40 to 75 years); 13 males; 7 females.
2) 3-7 years of diabetes course: fasting plasma glucose (FPG): greater than 5 mmol/L, vs age-matched controls (4.1±0.3 mmol/L);
3) chronic sensorimotor distal, symmetrical neuropathy (DSPN):
   Nerve electromyography (EMG) was used for neurophysiological examination to evaluate the ability of peripherally myelinated thick fiber nerves to conduct electrical signals. They were selected and diagnosed as distal, symmetrical diabetic peripheral polyneuropathy (a common type of DNP), with nerve damage persisting for at least 6 months. Nerve detection included: 1) sensory nerve conduction function: median nerve (left and right sides), ulnar nerve (left and right sides), sural nerve (left and right sides), superficial peroneal nerve (left and right sides); and 2) motor nerve conduction function: median nerve (left and right sides), ulnar nerve (left and right sides), common peroneal nerve (left and right sides). The criteria of abnormal nerve conduction velocity (SNCV) in this testing were consistent with the DPN criteria of the WHO international collaborative research of the diabetic peripheral neuropathy (WHOPNTF).

Those complying with the criteria shown in Table 10 below were nerve conduction abnormalities:

**Table 10 Criteria for nerve conduction abnormalities**

| **Motor nerve conduction velocity (m/s)** | **Sensory nerve conduction velocity (m/s)** |
|---|---|
| Median nerve ≤50 | Median nerve ≤44 |
| Ulnar nerve ≤49 | Ulnar nerve ≤44 |
| Common peroneal nerve ≤41 | Sural nerve ≤41 |
| | Superficial peroneal nerve ≤44 |

For comparison, Table 11 below shows reference values of nerve conduction velocity for healthy rhesus monkeys (non-diabetic) (data from measured average values of 20 age-matched healthy rhesus monkeys).

**Table 11 Nerve function parameters of age-matched controls of non-diabetic monkeys**

| | | **Nerve conduction velocity (m/s) in healthy rhesus monkeys (n=20, age: 13±4 vears, 8-19 years)** |
|---|---|---|
| Motor nerve conduction MCV (m/s) | Median nerve (N=40 nerve) | 67.2±6.6 |
| | Ulnar nerve (N=40 nerve) | 69.2±7.6 |
| | Common peroneal nerve (N=40 nerve) | 67.7±7.1 |
| Sensory nerve conduction SVC (m/s) | Median nerve (N=40 nerve) | 55.7±6.3 |
| | Ulnar nerve (N=40 nerve) | 63.4±7.4 |
| | Sural nerve (N=40 nerve) | 51.1±5.9 |
| | Superficial peroneal nerve (N=40 nerve) | 70.2±7.7 |

### 5.2.3 Grouping and administration

Twenty rhesus monkeys with spontaneous DPN were divided into six groups. Specific grouping is shown in Table 12 below. Table 13 provides abnormality detection of nerve conduction parameters in nerve electromyography of tested rhesus monkeys with spontaneous DPN in each group (judged according to the criteria in Table 10).

**Table 12 Grouping of DPN rhesus monkeys and basic data**

| **Group** | **Number of animal** | **Age (years)** | **Gender** | **Weight (kg)** | **Course of diabetes (years)** |
|---|---|---|---|---|---|
| **Placebo group (n=4)** | M1 | 17 | Female | 7.21 | 6 |
| | M2 | 13 | Female | 7.36 | 4 |
| | M3 | 20 | Female | 6.97 | 7 |
| | M4 | 17 | Female | 4.94 | 4 |
| | Average | 17±3 | | 6.62±1.13 | 5±2 |
| DOX+PMP+M TP | M5 | 22 | Male | 7.97 | 7 |
| | M6 | 24 | Male | 8.05 | 5 |
| **Experimental group (n=4)** | M7 | 25 | Male | 10.69 | 5 |
| | M8 | 25 | Male | 10.42 | 5 |
| | Average | 24±1 | | 9.28±1.47 | 6±1 |
| **Epalrestat group (n=4)** | M9 | 24 | Male | 8.05 | 5 |
| | M10 | 25 | Male | 10.42 | 5 |
| | M11 | 25 | Male | 10.69 | 5 |
| | M12 | 14 | Female | 5.36 | 3 |
| | Average | 22±5 | | 8.63±2.48 | 5±1 |
| **PMP Group (n=3)** | M13 | 25 | Male | 10.63 | 6 |
| | M14 | 17 | Female | 7.57 | 7 |
| | M15 | 25 | Male | 10.44 | 6 |
| | Average | 22±5 | | 9.55±1.71 | 6±1 |
| **DOX+MTP group (n=3)** | M16 | 15 | Male | 7.54 | 4 |
| | M17 | 24 | Male | 7.6 | 6 |
| | M18 | 16 | Female | 5.37 | 4 |
| | Average | 18±5 | | 6.84±1.27 | 5±1 |
| **DOX+PMP group (n=2)** | M19 | 25 | Male | 11.08 | 6 |
| | M20 | 25 | Male | 10.69 | 6 |
| | Average | 25±0 | | 10.89±0.28 | 6±0 |

**Table 13 Baseline period - abnormality detection in nerve electromyography of tested rhesus monkeys with spontaneous DPN in each group**

| **Group** | **Num ber of anim at** | **Motor nerve conduction velocity (MCV)** | | | | | | **Sensory nerve conduction velocity (SCV)** | | | | | | | |
|---|---|---|---|---|---|---|---|---|---|---|---|---|---|---|---|
| | | Right median nerve | Left median nerve | Right ulnar nerve | Left ulnar nerve | Right common peroneal nerve | Left common peroneal nerve | Right median nerve | Left median nerve | Right ulnar nerve | Left ulnar nerve | Right sural nerve | Left sural nerve | Right superfi cial perone al **nerve** | Left superfi cial peroneal nerve |
| **Placebo group (n=4)** | M1 | Normal | Normal | Normal | Normal | Normal | Normal | Normal | Normal | Normal | Normal | **Abnor mal** | **Abnor mal** | Normal | Normal |
| | M2 | Normal | Normal | Normal | Normal | Normal | Normal | Normal | Normal | Normal | Normal | **Abnor mal** | Normal | Normal | Normal |
| | M3 | Normal | Normal | Normal | Normal | Normal | **Abnor mal** | Normal | Normal | Normal | Normal | **Abnor mal** | **Abnor mal** | Normal | Normal |
| | M4 | Normal | Normal | Normal | Normal | Normal | Normal | Normal | Normal | Normal | Normal | **Abnor mal** | **Abnor mal** | Normal | Normal |
| **DOX+PMP+ MTP treatment group (n=4)** | M5 | **Abnor mal** | **Abnor mal** | **Abnor mal** | **Abnor mal** | **Abnor mal** | **Abnor mal** | **Abnor mal** | **Abnor mal** | **Abnor mal** | Normal | **Abnor mal** | **Abnor mal** | Normal | Normal |
| | M6 | Normal | **Abnor mal** | **Abnor mal** | **Abnor mal** | Normal | Normal | **Abnor mal** | Normal | Normal | **Abnor mal** | **Abnor mal** | **Abnor mal** | Normal | Normal |
| | M7 | Normal | Normal | Normal | Normal | Normal | Normal | Normal | **Abnor mal** | Normal | **Abnor mal** | **Abnor mal** | **Abnor mal** | Normal | Normal |
| | M8 | **Abnor mal** | Normal | Normal | Normal | Normal | **Abnor mal** | **Abnor mal** | **Abnor mal** | Normal | Normal | **Abnor mal** | **Abnor mal** | Normal | Normal |
| **Epalrestat group (n=4)** | M9 | Normal | Normal | **Abnor mal** | **Abnor mal** | Normal | Normal | **Abnor mal** | Normal | Normal | Abnor mal | **Abnor mal** | **Abnor mal** | Normal | Normal |
| | M10 | Normal | Normal | Normal | Normal | Normal | **Abnor mal** | **Abnor mal** | **Abnor mal** | **Abnor mal** | Normal | **Abnor mal** | **Abnor mal** | Normal | Normal |
| | M11 | Normal | Normal | Normal | Normal | Normal | Normal | Normal | **Abnor mal** | Normal | Normal | **Abnor mal** | **Abnor mal** | Normal | Normal |
| | M12 | Normal | Normal | Normal | Normal | Normal | Normal | Normal | Normal | Normal | Normal | **Abnor mal** | Normal | Normal | Normal |
| **PMP group (n=3)** | M13 | Normal | Normal | Normal | Normal | Normal | Normal | Normal | **Abnor mal** | Normal | Normal | **Abnor mal** | Normal | Normal | Normal |
| | M14 | Normal | Normal | Normal | Normal | Normal | Normal | Normal | Normal | Normal | Normal | **Abnor mal** | **Abnor mal** | Normal | Normal |
| | M15 | Normal | **Abnor mal** | Normal | Normal | Normal | Normal | **Abnor mal** | **Abnor mal** | Normal | Normal | **Abnor mal** | **Abnor mal** | Normal | Normal |
| **DOX+MTP group** (n=3) | M16 | Normal | Normal | Normal | Normal | Normal | Normal | **Abnor mal** | **Abnor mal** | Normal | Normal | **Abnor mal** | **Abnor mal** | Normal | Normal |
| | M17 | Normal | Normal | **Abnor mal** | Normal | Normal | Normal | **Abnor mal** | Normal | Normal | Normal | **Abnor mal** | **Abnor mal** | Normal | Normal |
| | M18 | Normal | Normal | Normal | Normal | Normal | Normal | Normal | Normal | Normal | Normal | **Abnor mal** | **Abnor mal** | Normal | Normal |
| **DOX+PMP group (n=2)** | M19 | Normal | Normal | Normal | Normal | Normal | Normal | **Abnor mal** | **Abnor mal** | Normal | Normal | **Abnor mal** | Normal | Normal | Normal |
| | M20 | Normal | **Abnor** | Normal | Normal | Normal | Normal | **Abnor** | **Abnor** | Normal | Normal | Normal | **Abnor** | Normal | Normal |

This test involved DOX+PMP+MTP group (n=4), epalrestat group (n=4), DOX+MTP group (n=3), PMP group (n=3), DOX+PMP group (n=2) and placebo group (n=4). The administration scheme of each group was described below, wherein the administration route was oral administration. The baseline period was 1 month, followed by continuous oral administration for 58 days to evaluate the effect of 58 days of treatment on nerve conduction velocity and the ability to reduce the continuous deterioration of nerve function. The following was the grouping and dosage information:
DOX+PMP+MTP group: 4 animals
D0 to D14: DOX+PMP+MTP: 0.133+0.002+0.833 mg/kg, twice a day
D15 to D28: DOX+PMP+MTP: 0.133+0.002+1.667 mg/kg, twice a day
D29 to D58: DOX+PMP+MTP: 0.266+0.004+1.667 mg/kg, twice a day
Epalrestat group: 4 animals; the dose was 2.5 mg/kg (equivalent to clinical equivalent dose of 40-80 mg), given daily at D0-D58, once a day in the first-second weeks and twice a day in the third-eighth weeks.
DOX+MTP group: 3 animals
D0 to D28: DOX+MTP: 0.266 mg/kg+1.667 mg/kg, twice a day
D29 to D58: DOX+MTP: 0.53 mg/kg+3.3 mg/kg, twice a day
PMP group: 3 animals
D0 to D58: 0.004 mg/kg, twice a day
DOX+PMP group: 2 animals
D0 to D28: 0.133 mg/kg + 0.002 mg/kg, twice a day
D29 to D58: 0.266 mg/kg + 0.004 mg/kg, twice a day
Placebo group: 4 animals, given drinking water or fruit, and observed continuously for 58 days.
Route of administration: food-induced administration.
Dosage calculation: calculating the dosage of the next week according to the weight weighed each time.

### 5.3 Observation index and monitoring method

### 5.3.1 Main efficacy indexes

### 5.3.1.1 Nerve electromyogram

Detection method: compound ketamine was injected intramuscularly. After anesthesia, a receiving electrode, a reference electrode, and a ground wire were pasted to the prepared skin of limbs, and the designated site was stimulated using a stimulating electrode. Current was increased until the image was stabilized and then saved, and the distance between the stimulating electrode and the receiving electrode (sensory conduction) or two stimulation points (motor conduction) was measured. During detection, the environment should be kept quiet and the temperature of the detection environment should be controlled at 20 °C-25 °C. The testers needed to be double-blind and were not aware of the animal dosing information.
Test items: see Table 14.
Detection frequency: once before administration and once after administration (58 days of administration).
Detection instrument: Haishen NDI-092 type electromyography/evoked potential apparatus.

**Table 14 Indexes of myopotential detection**

| **Measured nerve** | **Measured site** | **Type** |
|---|---|---|
| Median nerve | Upper limb | Motor conduction |
| Ulnar nerve | | |
| Common peroneal nerve | Lower limb | |
| Median nerve | Upper limb | Sensory conduction |
| Ulnar nerve | | |
| Superficial peroneal nerve | Lower limb | |
| Sural nerve | | |

### 5.3.2 Other efficacy indexes

### 5.3.2.1 Glycolipid metabolism indexes and blood biochemical indexes

Blood sample collection method: see Table 15, specifically: the animals were fasted overnight the day before the blood collection operation, and blood was collected at 08:00 the next day without anesthesia, from forearm vein.

**Table 15 Sample collection and processing information**

| **Sampling method** | **Sampling quantity and temporary storage condition** | **Type of collecting tube** | **Sample processing method** | **Sample use** |
|---|---|---|---|---|
| Fasting, non-anesthesia | 1ml/2ml (when a full set of blood biochemistry was included) mixture of ice and water | EDTA2K anticoagulant vacuum blood collection tube | 3000 rpm, 10 min, centrifugation at 4 °C, separation of plasma | Glycolipid metabolism/ blood biochemistr y |

Detection index and method: see Table 16
Detection frequency: once before administration and once at Day 58 after administration
Detection instrument: Roche cobas6000 analyzer series C501 module was used for detection

**Table 16 Blood biochemical detection items**

| **Tested item** | **Unit** | **Testing method** |
|---|---|---|
| Blood glucose (GLU) | mmol/L | Hexokinase method |
| Fructosamine (FRA) | µmol/L | Rate method |
| Total cholesterol (TC) | mmol/L | Enzyme colorimetry |
| Low density lipoprotein (LDL) | mmol/L | Homogeneous enzyme colorimetry |
| High density lipoprotein (HDL) | mmol/L | Homogeneous enzyme colorimetry |
| Triglyceride (TG) | mmol/L | Enzyme colorimetry |
| Alanine transaminase (ALT) | IU/L | IFCC rate method |
| Aspartate transaminase (AST) | IU/L | Colorimetry |
| Alkaline phosphatase (ALP) | IU/L | Colorimetry, rate method |
| γ-glutamyl transpeptidase (GGT) | IU/L | Enzyme colorimetry |
| Total bilirubin (TBIL) | µmol/L | Diazo method |
| Direct bilirubin (DBIL) | µmol/L | Diazo method, two point endpoint method |
| Total protein (TP) | g/L | Colorimetry, two point endpoint method |
| Albumin (ALB) | g/L | Colorimetry |
| Urea nitrogen (BUN) | mg/dL | Colorimetry, rate method |
| Creatinine (CR) | mg/dL | Enzyme colorimetry |
| Creatine phosphokinase (CK) | IU/L | Colorimetry, rate method |

### 5.3.2.2 Observation of clinical symptoms

Observation times: once a day.
Observation contents: skin, coat, eyes, ears, nose, mouth, chest, abdomen, genitourinary part, limbs, etc., and breathing, movement, urination, defecation, and behavior changes.

### 5.3.3 Data processing

The results were expressed in the form of individual data. Microsoft Office Excel 2016 software was used for data processing and statistical analysis. Measurement data was expressed by "Mean±SD". Taking the latest data before administration as the baseline value, the indexes before and after administration were statistically analyzed by repeated measurement, and P<0.05 meant that the difference was of statistical significance.

### 5.4 Experimental results and discussion

### 5.4.1 Effect on improvement of neurological function

### 5.4.1.1 Effect on sensory nerve conduction velocity

Sensory nerves labeled as "abnormal" in Table 13 were subjected to measurement by electromyography before administration (baseline) and at Day 58 (D58) after administration to evaluate the effect of the tested drug on sensory nerve conduction velocity in rhesus monkeys with diabetic peripheral neuropathy. The results are shown in Table 17 and FIG. 24 and FIG. 25.

**Placebo group** (n=4): after administration, no significant change was observed in conduction velocity of each nerve, which proved that the model was relatively stable. The change in conduction velocity measurements of seven abnormal nerves at D58 relative to baseline was - 0.4±1.0 m/s.

**Epalrestat group** (n=4): compared with the baseline value, 13 nerves with abnormal conduction velocities administered for 58 days increased significantly in SCV and benefited therefrom, with a mean increase of 4.2±4.0 m/s, which was increased extremely significantly compared with the placebo group (P<0.01 vs placebo group).

**DOX+PMP+MTP group** (n=4): compared with the baseline value, 17 nerves with abnormal conduction velocities administered for 58 days increased significantly in SCV and benefited therefrom, with a mean increase of 4.3±3.4 m/s (P<0.05 vs baseline value), which was increased extremely significantly compared with the placebo group (P<0.01 vs placebo group).

In addition, compared with the placebo group, no significant improvement in abnormal sensory nerve conduction velocity was observed in the DOX+MTP group, PMP group, and DOX+PMP group, and there was no statistically significant difference compared with the placebo group (P>0.05 vs placebo group).

**Table 17 Effect of 58-day administration on sensory nerve conduction velocity in rhesus monkeys with diabetic peripheral neuropathy**

| Group | Animal number | Nerve name | Baseline value (m/s) | Administration D58 (m/s) | Variation value (m/s) | Count of the total number of abnormal nerves | P (change value of each treatment group vs change value of placebo group) |
|---|---|---|---|---|---|---|---|
| Placebo group (n=4) | M1 | Right sural nerve | 35.5 | 35.6 | 0.1 | 7 | NA |
| | | Left sural nerve | 40.2 | 38.8 | -1.4 | | |
| | M2 | Right sural nerve | 42.1 | 41.6 | -0.5 | | |
| | M3 | Right sural nerve | 28.1 | 29.6 | 1.5 | | |
| | | Left sural nerve | 29.6 | 29.2 | -0.4 | | |
| | M4 | Right sural nerve | 40.2 | 40.0 | -0.2 | | |
| | | Left sural nerve | 39.8 | 38.2 | -1.6 | | |
| | Mean±SD | | **36.5±5.6** | **36.1±5.0** | **-0.4±1.0** | | |
| DOX + PMP + MTP group (n=4) | M5 | Right median nerve | 29.7 | 36.4 | 6.7 | 17 | 0.0017 |
| | | Left median nerve | 36.5 | 37.9 | 1.4 | | |
| | | Right ulnar nerve | 39.6 | 45.6 | 6.0 | | |
| | | Right sural nerve | 30.0 | 32.4 | 2.4 | | |
| | | Left sural nerve | 29.5 | 30.3 | 0.8 | | |
| | M6 | Right median nerve | 37.6 | 40.9 | 3.3 | | |
| | | Left ulnar nerve | 43.0 | 41.8 | -1.2 | | |
| | | Right sural nerve | 32.7 | 41.7 | 9.0 | | |
| | | Left sural nerve | 32.0 | 40.0 | 8.0 | | |
| | M7 | Left median nerve | 40.0 | 43.8 | 3.8 | | |
| | | Left ulnar nerve | 40.4 | 52.6 | 12.2 | | |
| | | Right sural nerve | 35.1 | 35.6 | 0.5 | | |
| | | Left sural nerve | 37.5 | 41.9 | 4.4 | | |
| | M8 | Right median nerve | 24.7 | 28.1 | 3.4 | | |
| | | Left median nerve | 35.6 | 40.0 | 4.4 | | |
| | | Right sural nerve | 33.9 | 36.7 | 2.8 | | |
| | | Left sural nerve | 34.4 | 40.0 | 5.6 | | |
| | Mean±SD | | **34.8±4.7** | **39.2±5.8*** | **4.3±3.4** | | |
| Epalrestat group (n=4) | M9 | Right median nerve | 43.4 | 48.2 | 4.8 | 13 | 0.0094 |
| | | Left ulnar nerve | 42.4 | 50.7 | 8.3 | | |
| | | Right sural nerve | 34.6 | 43.9 | 9.3 | | |
| | | Left sural nerve | 35.3 | 41.4 | 6.1 | | |
| | M10 | Right median nerve | 25.8 | 25.1 | -0.7 | | |
| | | Left median nerve | 38.5 | 40 | 1.5 | | |
| | | Right ulnar nerve | 39.2 | 48.4 | 9.2 | | |
| | | Right sural nerve | 40.9 | 41.3 | 0.4 | | |
| | | Left sural nerve | 40.4 | 39.3 | -1.1 | | |
| | M11 | Left median nerve | 42.6 | 51.2 | 8.6 | | |
| | | Right sural nerve | 36.8 | 39.6 | 2.8 | | |
| | | Left sural nerve | 39.6 | 45.2 | 5.6 | | |
| | M12 | Right sural nerve | 44.4 | 43.8 | -0.6 | | |
| | Mean±SD | | **38.8±4.7** | **43.6±7.3** | **4.2±4.0** | | |
| PMP group(n=3) | M13 | Left median nerve | 44.0 | 42.1 | -1.9 | 8 | 0.2752 |
| | | Right sural nerve | 40.0 | 39.6 | -0.4 | | |
| | M14 | Right sural nerve | 39.0 | 41.1 | 2.1 | | |
| | | Left sural nerve | 40.5 | 40.2 | -0.3 | | |
| | M15 | Right median nerve | 26.2 | 25.6 | -0.6 | | |
| | | Left median nerve | 39.2 | 40.0 | 0.8 | | |
| | | Right sural nerve | 41.2 | 42.6 | 1.4 | | |
| | | Left sural nerve | 38.5 | 40.4 | 1.9 | | |
| | Mean±SD | | **38.6±4.9** | **39.0±5.1** | **0.4±1.3** | | |
| DOX+ MTP group (n=3) | M16 | Right median nerve | 43.5 | 44.4 | 0.9 | 9 | 0.3711 |
| | | Left median nerve | 40.9 | 38.4 | -2.5 | | |
| | | Right sural nerve | 37.2 | 36.6 | -0.6 | | |
| | | Left sural nerve | 41.0 | 36.8 | -4.2 | | |
| | M17 | Right median nerve | 43.4 | 41.3 | -2.1 | | |
| | | Right sural nerve | 39.5 | 41.3 | 1.8 | | |
| | | Left sural nerve | 40.4 | 38.6 | -1.8 | | |
| | M18 | Right sural nerve | 38.1 | 36.4 | -1.7 | | |
| | | Left sural nerve | 38.6 | 38.6 | 0.0 | | |
| | Mean±SD | | **40.3±2.1** | **39.2±2.5** | **-1.1±1.7** | | |
| DOX+ PMP group(n=2) | M19 | Right median nerve | 41.2 | 44.0 | 2.8 | 6 | 0.6604 |
| | | Left median nerve | 42.1 | 44.0 | 1.9 | | |
| | | Right sural nerve | 35.6 | 34.6 | -1.0 | | |
| | M20 | Right median nerve | 23.1 | 25.0 | 1.9 | | |
| | | Left median nerve | 39.2 | 33.9 | -5.3 | | |
| | | Left sural nerve | 41.2 | 42.0 | 0.8 | | |
| | Mean ± SD | | **37.1±6.6** | **37.3±6.9** | **0.2±2.7** | | |

| | | | | | | | |
|---|---|---|---|---|---|---|---|
| Note: *, p<0.05 vs baseline value. Value of change = end of administration-baseline value. | | | | | | | |

### 5.4.1.2 Effect on sensory conduction velocity of sural nerve

The degree and rate of sensory nerve conduction velocity (SCV) abnormality in patients with DPN were more severe than those of motor nerve conduction velocity (MCV) abnormality, and the patients' neurological abnormalities were usually more severe in the lower limbs (sural nerve) than in the upper limbs. The disease phenotype characteristics of DPN rhesus monkeys were similar to those of DPN patients. Therefore, evaluating whether sensory conduction velocity of sural nerve in DPN rhesus monkeys 58 days after administration was improved was the primary efficacy index. The results of effect of the tested drugs on the sensory conduction velocity of "abnormal" sural nerve in DPN rhesus monkeys are shown in Table 18, FIG. 26 and FIG. 27. Compared with the baseline, after 58 days of administration, the nerve conduction velocity returning to normal or increasing by 2-5 m/s was diagnosed as being effective, or increasing by more than 5 m/s was determined to be significantly effective.

**Placebo group (n=4):** no significant changes in conduction velocity were observed in each nerve after administration, and the average change in conduction velocity (SCV) measurements of 7 abnormal nerves after 58 days of administration relative to the baseline value was -0.4±1.0 m/s, demonstrating that the model was relatively stable. Compared with the baseline, 0/7 nerve was effective.

**Epalrestat group** (n=4): compared with the baseline value, the sensory nerve conduction velocity (SCV) of sural nerves of 7 abnormal nerves increased averagely by 3.2±3.9 m/s after 58 days of administration, which was increased significantly compared with the placebo group (P<0.05 vs placebo group). Compared with the baseline, 4/7 nerves were determined to be effectively improved in conduction velocity.

DOX+PMP+MTP (n=4): compared with the baseline value, the sensory nerve conduction velocity (SCV) of 8 abnormal sural nerves increased significantly 58 days after administration, with an average increase of 4.6±3.9 m/s (P<0.05 vs baseline value), which was increased extremely significantly compared with the placebo group (P<0.01 vs placebo group). Compared with the baseline, 6/8 (75%) nerves were determined to be effectively improved in conduction velocity.

In addition, compared with the placebo group, no significant improvement in abnormal sural sensory nerve conduction velocity was observed in the DOX+MTP group, PMP group and DOX+PMP group, and there was no statistically significant difference compared with the placebo group (P>0.05 vs placebo group).

**Table 18 Effect of 58-day administration on sural sensory nerve conduction velocity in DNP rhesus monkeys**

| Group | Number of animal | Name of nerve | Baseline value (m/s) | D58 of administration (m/s) | Change value (m/s) | Calculation of the total number of abnormal nerves | P (change value of treatment group vs change value in placebo group) |
|---|---|---|---|---|---|---|---|
| Placebo group (n=4) | M1 | Right sural nerve | 35.5 | 35.6 | 0.1 | 7 | NA |
| | | Left sural nerve | 40.2 | 38.8 | -1.4 | | |
| | M2 | Right sural nerve | 42.1 | 41.6 | -0.5 | | |
| | M3 | Right sural nerve | 28.1 | 29.6 | 1.5 | | |
| | | Left sural nerve | 29.6 | 29.2 | -0.4 | | |
| | M4 | Right sural nerve | 40.2 | 40.0 | -0.2 | | |
| | | Left sural nerve | 39.8 | 38.2 | -1.6 | | |
| | Mean±SD | | 36.5±5.6 | 36.1±5.0 | 0.4±1.0 | | |
| DOX+ PMP+ MTP group (n=4) | M5 | Right sural nerve | 30.0 | 32.4 | 2.4 | 8 | 0.0031 |
| | | Left sural nerve | 29.5 | 30.3 | 0.8 | | |
| | M6 | Right sural nerve | 32.7 | 41.7 | 9.0 | | |
| | | Left sural nerve | 32.0 | 40.0 | 8.0 | | |
| | M7 | Right sural nerve | 35.1 | 35.6 | 0.5 | | |
| | | Left sural nerve | 37.5 | 41.9 | 4.4 | | |
| | M8 | Right sural nerve | 33.9 | 36.7 | 2.8 | | |
| | | Left sural nerve | 34.4 | 40.0 | 5.6 | | |
| | Mean±SD | | 33.1±2.7 | 37.3±4.3* | 4.6±3.9 | | |
| Epalrestat group (n=4) | M9 | Right sural nerve | 34.6 | 43.9 | 9.3 | 7 | 0.0380 |
| | | Left sural nerve | 35.3 | 41.4 | 6.1 | | |
| | M10 | Right sural nerve | 40.9 | 41.3 | 0.4 | | |
| | | Left sural nerve | 40.4 | 39.3 | -1.1 | | |
| | M11 | Right sural nerve | 36.8 | 39.6 | 2.8 | | |
| | | Left sural nerve | 39.6 | 45.2 | 5.6 | | |
| | M12 | Right sural nerve | 44.4 | 43.8 | -0.6 | | |
| | Mean±SD | | 38.9±3.2 | 42.1±2.1 | 3.2±3.9 | | |
| PMP group (n=3) | M13 | Right sural nerve | 40.0 | 39.6 | -0.4 | 5 | 0.0722 |
| | M14 | Right sural nerve | 39.0 | 41.1 | 2.1 | | |
| | | Left sural nerve | 40.5 | 40.2 | -0.3 | | |
| | M15 | Right sural nerve | 41.2 | 42.6 | 1.4 | | |
| | | Left sural nerve | 38.5 | 40.4 | 1.9 | | |
| | Mean±SD | | 39.8±1.0 | 40.8±1.0 | 0.9±1.1 | | |
| DOX+ MTP group (n=3) | M16 | Right sural nerve | 37.2 | 36.6 | -0.6 | 6 | 0.4197 |
| | | Left sural nerve | 41.0 | 36.8 | -4.2 | | |
| | M17 | Right sural nerve | 39.5 | 41.3 | 1.8 | | |
| | | Left sural nerve | 40.4 | 38.6 | -1.8 | | |
| | M18 | Right sural nerve | 38.1 | 36.4 | -1.7 | | |
| | | Left sural nerve | 38.6 | 38.6 | 0.0 | | |
| | Mean±SD | | 39.1±1.3 | 38.1±1.7 | -1.1±1.8 | | |
| DOX+ PMP group (n=2) | M19 | Right sural nerve | 35.6 | 34.6 | -1.0 | 2 | 0.6327 |
| | M20 | Left sural nerve | 41.2 | 42.0 | 0.8 | | |
| | Mean±SD | | 38.4±2.8 | 38.3±3.7 | -0.1±0.9 | | |

| | | | | | | | |
|---|---|---|---|---|---|---|---|
| Note: *, p<0.05 vs baseline value. Value of change = end of administration-baseline value. | | | | | | | |

### 5.4.1.3 Effect on motor nerve conduction velocity

The number of nerves abnormal in motor nerve conduction velocity (MCV) in each group of DPN rhesus monkeys enrolled in this test was small and did not meet the statistical criteria. Therefore, the effect on motor nerve conduction velocity was not studied in this test. In addition, since the degree and rate of sensory nerve conduction velocity (SCV) abnormality in patients with DPN were usually more severe than those of motor nerve conduction velocity (MCV) abnormality, the effect of SCV was more important in pharmacodynamic studies.

### 5.4.2 Safety of composition

The safe dose ranges and toxic side effects of DOX, PMP, and MTP are known. The dosage of DOX tablets commonly used is 4-8 mg/time, twice a day. The recommended individual dose of PMP is 0.375 mg to 4.5 mg per day. The initial dose for treating idiopathic Parkinson's disease is 0.375 mg per day, and then the dose is increased once every 5-7 days, with the maximum dose of 1.5 mg. The incidence of drowsiness increased when the daily dose is higher than 1.5 mg. The recommended dose of MTP is 100-200 mg/day, which may reach 300 mg/day or 400 mg/day if necessary. The known adverse reaction is hypotension.

In the test, the human dosage calculated based on the dosage of rhesus monkeys is lower than the maximum value of the recommended dosage, so in theory, the combined use of DOX+PMP+MTP of the present invention has very good safety.

During the above experiment, no adverse events (including blood biochemical indexes and clinical observations) related to drug administration were seen throughout the drug administration period in the animals in the DOX+PMP+MTP group, which further confirmed that the combined use of DOX+PMP+MTP of the present invention had good safety.

### 5.4.3 Discussion of results

The above experimental results showed that combined use of three drugs (DOX+PMP+MTP) for oral administration for 58 days could significantly improve nerve conduction velocity in rhesus monkey models with spontaneous DPN, especially sural nerve sensory nerve conduction velocity, control and alleviate DPN progression, and show good safety during administration, with an intensity of efficacy comparable to that of the commonly used first-line clinical drug, epalrestat.

### 6. Example 6

Example of pharmaceutical composition: for 10 kg monkeys, 1.3 mg of Doxazosin mesylate (DOX) bulk drug powder, 0.02 mg of Pramipexole Dihydrochloride (PMP) bulk drug powder, and 8.3 mg of Metoprolol tartrate salt (MTP) bulk drug powder could be uniformly mixed to obtain the pharmaceutical composition example. The pharmaceutical composition could be directly mixed into food for feeding animals, or could be prepared into oral tablets after being mixed with appropriate excipients or additives.

All patent disclosures and patent applications referred to in this specification are incorporated herein by reference to the same extent that each individual patent disclosure or patent application is specifically and individually shown to be incorporated by reference.

Although preferred embodiments of the present invention have been shown and described herein, it will be apparent to those skilled in the art that these embodiments are provided by way of example only. It is intended that the following claims define the scope of the present invention and thereby cover methods and structures within the scope of these claims and their equivalents.

### Incorporation by reference

Various publications are cited herein, the disclosures of which are incorporated herein by reference in their entirety:
(1) Wu, S. et al. Stroke in China: advances and challenges in epidemiology, prevention, and management. Lancet Neurol. 18, 394-405 (2019). Kim, A.S., Cahill, E. & Cheng, N.T. Global Stroke Belt: Geographic Variation in Stroke Burden Worldwide. Stroke 46, 3564-3570 (2015);
(2) Gg A, Cq A, Lfa B. Prevention of dementia in an aging world: Evidence and biological rationale. Ageing Research Reviews, 2020, 64: 101045;
(3) Jca B, Gl B, Ar B, et al. Alzheimer's disease drug development pipeline: 2019. Alzheimer's & Dementia: Translational Research & Clinical Interventions, 2019, 5:272-293;
(4) Panza F, Lozupone M, Logroscino G, et al. A critical appraisal of amyloid-β-targeting therapies for Alzheimer disease. Nature Reviews Neurology, 2019, 15(2): 73-88;
(5) Khanh Vu TH, Chen H, Pan L, Cho KS, Doesburg D, Thee EF, Wu N, Arlotti E, Jager MJ, Chen DF. CD4+ T-Cell Responses Mediate Progressive Neurodegeneration in Experimental Ischemic Retinopathy. Am J Pathol. 2020 Aug; 190(8): 1723-1734;
(6) Soares R O S, Losada D M, Jordani M C, et al. Ischemia/Reperfusion Injury Revisited: An Overview of the Latest Pharmacological Strategies[J]. International Journal of Molecular Sciences, 2019, 20(20): 5034;
(7) Ardura-Fabregat A, Boddeke E, Boza-Serrano A, et al. Targeting Neuroinflammation to Treat Alzheimer's Disease. CNS Drugs, 2017, 31(6005).;
(8) Hz B, Tz A, Dw C, et al. IFN-γ regulates the transformation of microglia into dendritic-like cells via the ERK/c-myc signaling pathway during cerebral ischemia/reperfusion in mice. Neurochemistry International, 2020, 141.;
(9) Belkhelfa M, Rafa H, Medjeber O, et al. IFN-γ and TNF-α are involved during Alzheimer disease progression and correlate with nitric oxide production: a study in Algerian patients. Journal of interferon & cytokine research: the official journal of the International Society for Interferon and Cytokine Research, 2014, 34(11): 839-847.;
(10) Proescholdt M G, Chakravarty S, Foster J A, et al. Intracerebroventricular but not intravenous interleukin-1beta induces widespread vascular-mediated leukocyte infiltration and immune signal mRNA expression followed by brain-wide glial activation. Neuroscience, 2002, 112(3):731-749.;
(11) Ferrari C C, Godoy M, Tarelli R, et al. Progressive neurodegeneration and motor disabilities induced by chronic expression of IL-1β in the substantia nigra. Neurobiology of Disease, 2006, 24(1):183-193.;
(12) Li M, Li Z, Yao Y, et al. Astrocyte-derived interleukin-15 exacerbates ischemic brain injury via propagation of cellular immunity. Proc Natl Acad Sci USA, 2017, 114(3): E396-E405.;
(13) Yongmei Chen and Raymond A. Swanson, Astrocytes and Brain Injury, Journal of Cerebral Blood Flow and Metabolism: Official Journal of the International Society of Cerebral Blood Flow and Metabolism 23, no. 2 (February 2003): 137-49;
(14) Chen Y H, Lin R R, Tao Q Q. The role of P2X7R in neuroinflammation and implications in Alzheimer's disease[J]. Life Sciences, 2021, 271:119187.;
(15) Cook DJ, Teves L, Tymianski M. Treatment of stroke with a PSD-95 inhibitor in the gyrencephalic primate brain. Nature. 2012 Feb 29;483(7388):213-7.;
(16) Adams HP Jr, Davis PH, Leira EC, et al. Baseline NIH Stroke Scale score strongly predicts outcome after stroke: A report of the Trial of Org 10172 in Acute Stroke Treatment (TOAST). Neurology. 1999 Jul;53(1):126-131.;
(17) Morgan, J. E. Optic nerve head structure in glaucoma: Astrocytes as mediators of axonal damage. Eye 14, 437-444 (2000);
(18) Tang, Y. et al. Therapeutic Targeting of Retinal Immune Microenvironment With CSF-1 Receptor Antibody Promotes Visual Function Recovery After Ischemic Optic Neuropathy. Front. Immunol. 11, 585918 (2020).;
(19) Wintermark M , Sesay M , Barbier E , et al. Comparative overview of brain perfusion imaging techniques. [J]. Journal of Neuroradiology, 2005, 32(9):294-314.;
(20) Shih L C , Saver J L , Alger J R , et al. Perfusion-Weighted Magnetic Resonance Imaging Thresholds Identifying Core, Irreversibly Infarcted Tissue[J]. Stroke, 2003, 34(6):1425-1430.;
(21) Oppenheim C , Peeters A , Cosnard G, et al. Which MR-derived perfusion parameters are the best predictors of infarct growth in hyperacute stroke? Comparative study between relative and quantitative measurements.[J]. Radiology, 2002, 223(2):361-370.;
(22) JING Yanping, LUO Bin, GAO Zhengrong, et al. Application value of DSC-PWI in ischemic cerebrovascular diseases [J], Journal of Chinese Physician, 2020, 22(3):6.;
(23) Bo, Zhang, Fei, et al. Chronic phencyclidine treatment impairs spatial working memory in rhesus monkeys.[J]. Psychopharmacology, 2019.;
(24) Buccafusco J J . Working Memory Delayed Response Tasks in Monkeys -- Methods of Behavior Analysis in Neuroscience[J]. CRC Press, 2009.;
(25) Louis R Pasquale et al. Development of Primary Open Angle Glaucoma-Like Features in a Rhesus Macaque Colony From Southern China. [J]. Transl Vis Sci Technol. 2021 Aug 2;10(9):20.;
(26) Brott TG, Adams HP Jr, Olinger CP, et al. Measurements of acute cerebral infarction: a clinical examination scale. Stroke 1989; 20: 864-70.;
(27) Emberson J, Lees KR, Lyden P, et al; Stroke Thrombolysis Trialists' Collaborative Group. Effect of treatment delay, age, and stroke severity on the effects of intravenous thrombolysis with alteplase for acute ischaemic stroke: a meta-analysis of individual patient data from randomised trials. Lancet. 2014 Nov 29;384(9958):1929-35.;
(28) Gupta R, Jovin TG. Endovascular management of acute ischemic stroke: advances in patient and treatment selection. Expert Rev Neurother. 2007 Feb;7(2):143-53.;
(29) Wu S, Wu B, Liu M, et al; China Stroke Study Collaboration. Stroke in China: advances and challenges in epidemiology, prevention, and management. Lancet Neurol. 2019 Apr;18(4):394-405.;
(30) Kim AS, Cahill E, Cheng NT. Global Stroke Belt: Geographic Variation in Stroke Burden Worldwide. Stroke. 2015 Dec;46(12):3564-70.;
(31) Kern TS, Du Y, Tang J, et al. Regulation of Adrenergic, Serotonin, and Dopamine Receptors to Inhibit Diabetic Retinopathy: Monotherapies versus Combination Therapies. Mol Pharmacol. 2021 Nov;100(5):470-479.;
(32) Palczewski K, Chen Y, Maeda A. Systems pharmacology for treating ocular disorders, US10117868[P]. 2018.;
(33) Roitberg B , Khan N , Tuccar E , et al. Chronic ischemic stroke model in cynomolgus monkeys: Behavioral, neuroimaging and anatomical study[J]. Neurological Research, 2003, 25(1):68-78.;
(34) A S E K . Clinical interpretation and use of stroke scales. [J]. The Lancet Neurology, 2006, 5(7):603-612.;
(35) Galgano M, Toshkezi G, Qiu X, et al. Traumatic Brain Injury: Current Treatment Strategies and Future Endeavors[J]. Cell Transplantation, 2017, 26(7):1118-1130.;
(36) Bourque PR, Chardon JW, Massie R. Autoimmune peripheral neuropathies. Clin Chim Acta. 2015 Sep 20;449:37-42.;
(37) Menorca RM, Fussell TS, Elfar JC. Peripheral Nerve Trauma: Mechanisms of Injury and Recovery. Hand Clin. 2013;29(3):317-330.;
(38) Blaes F. Diagnosis and therapeutic options for peripheral vasculitic neuropathy. Ther Adv Musculoskelet Dis. 2015;7(2):45-55.;
(39) LIN Shihe. Ischemic peripheral neuropathy [J]. Journal of New Medicine, 2001, 32 (1): 13.;
(40) Hotta, Nigishi, Akanuma et al. Long-Term Clinical Effects of Epalrestat, an Aldose Reductase Inhibitor, on Diabetic Peripheral Neuropathy.[J]. Diabetes Care, 2006.;
(41) Zakin E, Abrams R, Simpson D M. Diabetic Neuropathy[J]. Seminars in Neurology, 2019, 39(05):560-569.;
(42) Neurocomplications Group of Chinese Diabetes Society, Expert consensus on diagnosis and treatment of diabetic neuropathy (2021 Edition). Chinese Journal of Diabetes, 2021, 13(06):540-557;
(43) Sato K , Yama K , Yu M , et al. Epalrestat increases intracellular glutathione levels in Schwann cells through transcription regulation[J]. Redox Biology, 2014.;
(44) Epalrestat. Reactions Weekly, 2011, 1369(1):18-18;
(45) Fujise Y, Koda M, Kato J, et al. Drug-induced hepatic injury caused by an aldose reductase inhibitor, epalrestat[J]. Kanzo, 2011, 52(6):351-355.;
(46) LI Yongli, YAN Fengshan, DOU Shewei, et al., Clinical comparative application of arterial proton spin labeling and dynamic magnetic susceptibility contrast-enhanced perfusion imaging in the evaluation of cerebral microcirculation in patients with cerebral ischemia, Journal of Zhengzhou University: Medical Edition, 2013 (4): 4;
(47) JING Yanping, LUO Bin, GAO Zhengrong, et al. Application value of DSC-PWI in ischemic cerebrovascular diseases, Journal of Chinese Physician, 2020, 22(3): 6.;
(48) YUAN Long, LI Xuesong, MENG Chunyang, et al., Research progress on the relationship between inflammation and neuropathic pain. Journal of Chinese Physician, 2020, 22 (2): 5.;
(49) JIA Jianping, CHEN Shengdi. Neurology. 8th Edition. Beijing: People's Medical Publishing House, 2018.;
(50) Tesfaye S, Selvarajah D, Gandhi R, Greig M, Shillo P, Fang F, Wilkinson ID. Diabetic peripheral neuropathy may not be as its name suggests: evidence from magnetic resonance imaging. Pain. 2016;157 Suppl 1: S72-80;
(51) Selvarajah D, Wilkinson ID, Maxwell M, Davies J, Sankar A, Boland E, Gandhi R, Tracey I, Tesfaye S. Magnetic resonance neuroimaging study of brain structural differences in diabetic peripheral neuropathy. Diabetes Care. 2014;37(6):1681-8.;
(52) Sloan G, Shillo P, Selvarajah D, Wu J, Wilkinson ID, Tracey I, Anand P, Tesfaye S. A new look at painful diabetic neuropathy. Diabetes Res Clin Pract. 2018 Oct;144:177-191. doi: 10.1016/j.diabres.2018.08.020. Epub 2018 Sep 7. PMID: 30201394.;
(53) Gordon S , Pallai S , Dinesh S , et al. A new look at painful diabetic neuropathy[J]. Diabetes Research and Clinical Practice, 2018, 144:177-191;
(54) Cornblath D R , Hillman M A , Striffler J S , et al. Peripheral neuropathy in diabetic monkeys[J]. Diabetes, 1989, 38(11):1365-1370.; Cornblath D R , Dellon AL, Mackinnon S E . Spontaneous diabetes mellitus in a rhesus monkey: Neurophysiological studies[J]. Muscle & Nerve, 1989, 12(3):233-235.;
(55) Tien Y. Wong, Ingrid U. Scott. Retinal-Vein Occlusion. N Engl J Med 2010; 363: 2135-44.;
(56) Peter A. Campochiaro. Anti-Vascular Endothelial Growth Factor Treatment for Retinal Vein Occlusions. Ophthalmologica 2012;227(suppl 1):30-35.;
(57) Ursula Schmidt-Erfurth, a José Garcia-Arumi, b Bianca S. Gerendas, et al. Guidelines for the Management of Retinal Vein Occlusion by the European Society of Retina Specialists (EURETINA). Ophthalmologica 2019;242:123-162.;
(58) Tong Zhao, Qiang Lu, Yong Tao, et al. Effects of apelin and vascular endothelial growth factor on central retinal vein occlusion in monkey eyes intravitreally injected with bevacizumab: a preliminary study. Molecular Vision 2011; 17:1044-1055.;
(59) Park SP, Ahn JK. Changes of aqueous vascular endothelial growth factor and pigment epithelium-derived factor following intravitreal bevacizumab for macular oedema secondary to branch retinal vein occlusion. Clin Experiment Ophthalmol 2009; 37:490-5.;
(60) Hidetaka Noma , Kanako Yasuda, Masahiko Shimura. Cytokines and Pathogenesis of Central Retinal Vein Occlusion. J. Clin. Med. 2020, 9, 3457.;
(61) Matsumoto Y, Freund KB, Peiretti E, Cooney MJ, Ferrara DC, Yannuzzi LA. Rebound macular edema following bevacizumab (Avastin) therapy for retinal venous occlusive disease. Retina. 2007, 27(4):426~31.;
(62) Rosenfeld PJ, Shapiro H, Tuomi L, et al. Characteristics of patients losing vision after 2 years of monthly dosing in the phase III ranibizumab clinical trials. Ophthalmology. 2011, 118(3):523~30.;
(63) Yan Z, An J, Shang Q, et al. YC-1 Inhibits VEGF and Inflammatory Mediators Expression on Experimental Central Retinal Vein Occlusion in Rhesus Monkey. Current Eye Research, 2018:1-8.;
(64) QIAN Feng. Clinical analysis of Alprostadil combined with mecobalamin in the treatment of diabetic neuropathy [J]. Journal of Huaihai Medicine, 2018, 36(4):2.

## Claims

1. A method for modulating neuropathy, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

2. The method of claim 1, wherein the method inhibits neuroinflammation.

3. The method of claim 2, wherein the method treats, prevents, or improves a disease, disorder, or condition associated with neuroinflammation.

4. The method of claim 3, wherein the disease, disorder, or condition comprises ischemic cerebral stroke, hemorrhagic cerebral stroke, transient ischemic attack, craniocerebral injury, vascular dementia, Alzheimer's disease, Lewy body dementia, frontotemporal dementia, glaucoma, ischemic optic neuropathy, optic neuritis, optic nerve tumor, traumatic optic neuropathy, retinal artery occlusion, retinal vein occlusion, and retinopathy of prematurity.

5. The method of claim 1, wherein the method repairs nerve injury.

6. The method of claim 1, 2, or 5, wherein the method treats, prevents, or improves a disease, disorder, or condition associated with neuropathy.

7. The method of claim 6, wherein the disease, disorder, or condition is a central nervous system disease, disorder, or condition.

8. The method of claim 7, wherein the central nervous system disease, disorder, or condition is a cerebrovascular disease.

9. The method of claim 8, wherein the cerebrovascular disease is stroke.

10. The method of claim 9, wherein the stroke is ischemic stroke.

11. The method of claims 1-10, wherein the subject is a subject within 7 hours after a stroke attack.

12. The method of claims 1-10, wherein the subject has a National Institute of Health stroke scale (NIHSS) of 20 or within 20.

13. The method of claims 1-10, wherein the subject has a National Institute of Health stroke scale (NIHSS) of 15 or within 15.

14. The method of claims 1-10, wherein the subject has a National Institute of Health stroke scale (NIHSS) of 8 to 15.

15. The method of claims 1-14, wherein the subject is a subject with moderate to severe stroke and persistent neurological impairment.

16. The method of claims 1-15, wherein the subject is a subject with anterior circulation large vessel occlusion.

17. The method of claims 1-16, wherein the subject has a symptom of occlusion in the M1 segment of the middle cerebral artery.

18. The method of claims 1-16, wherein the subject has a symptom of occlusion in the M2 segment of the middle cerebral artery.

19. The method of claims 1-18, wherein the subject is a subject with ischemia-reperfusion injury.

20. The method of claim 19, wherein the reperfusion is performed by intravascular therapy.

21. The method of claim 19, wherein the reperfusion is performed by administration of a thrombolytic agent.

22. The method of claim 19, wherein the reperfusion is performed by angioplasty.

23. The method of claims 1-18, wherein the subject is an ischemic non-reperfusion subject.

24. The method of claims 1-18, wherein the subject is a subject with a massive cerebral infarction.

25. The method of claims 10-24, wherein the method improves cerebral tissue perfusion in the subject in an acute phase after stroke.

26. The method of claims 10-24, wherein the method inhibits a reduction in regional cerebral blood volume (rCBV) around cerebral tissue infarction.

27. The method of claims 10-24, wherein the method improves ischemic condition in the acute phase after stroke.

28. The method of claims 10-24, wherein the method saves ischemic penumbra.

29. The method of claims 10-24, wherein the method reduces brain edema/infarction volume in the subj ect.

30. The method of claims 10-24, wherein the method inhibits central nervous inflammation.

31. The method of claims 10-24, wherein the method inhibits overexpression of glial fibrillary acidic protein (GFAP).

32. The method of claims 10-24, wherein the method reduces stroke neurobehavioral score.

33. The method of claims 10-24, wherein the method improves neurological deficits in the subj ect.

34. The method of claims 10-24, wherein the method improves and restores finger function and limb strength of an affected limb in the subject.

35. The method of claims 10-24, wherein the method reduces disability rate of the subject.

36. The method of claims 10-24, wherein the method prevents, treats, and improves cognitive dysfunction in the subject, including aspects of memory, visual space, and comprehension judgment.

37. The method of claims 10-24, wherein the method prevents and impedes development of dementia.

38. The method of claims 10-24, wherein the method treats destructive effect of ischemia on the central nervous system of the subject.

39. The method of claim 9, wherein the stroke is hemorrhagic stroke.

40. The method of claim 39, wherein the hemorrhagic stroke is a condition of cerebral hemorrhage.

41. The method of claim 39, wherein the hemorrhagic stroke is a condition of subarachnoid hemorrhage.

42. The method of claim 8, wherein the cerebrovascular disease is a transient ischemic attack.

43. The method of claim 42, wherein the method improves cerebral tissue perfusion in the subject in an acute phase after stroke.

44. The method of claim 42, wherein the method inhibits reduction in regional cerebral blood volume (rCBV) in an injured brain tissue area.

45. The method of claim 42, wherein the method improves ischemic condition in the acute phase after stroke.

46. The method of claim 42, wherein the method saves ischemic penumbra.

47. The method of claim 42, wherein the method inhibits central nervous inflammation.

48. The method of claim 42, wherein the method inhibits overexpression of glial fibrillary acidic protein (GFAP).

49. The method of claim 42, wherein the method reduces stroke neurobehavioral score.

50. The method of claim 42, wherein the method improves neurological deficits in the subj ect.

51. The method of claim 42, wherein the method improves and restores finger function and limb strength of an affected limb in the subject.

52. The method of claim 42, wherein the method reduces disability rate of the subject.

53. The method of claim 42, wherein the method prevents, treats, and improves cognitive dysfunction in the subject, including aspects of memory, visual space, and comprehension judgment.

54. The method of claim 42, wherein the method prevents and impedes development of dementia.

55. The method of claim 42, wherein the method treats destructive effect of ischemia on the central nervous system of the subject.

56. The method of Claim 7, wherein the central nervous system disease, disorder, or condition is craniocerebral injury.

57. The method of claim 56, wherein the craniocerebral injury is traumatic brain injury.

58. The method of claim 57, wherein the method inhibits central nervous inflammation.

59. The method of claim 57, wherein the method inhibits GFAP overexpression.

60. The method of Claim 7, wherein the central nervous system disease, disorder, or condition is a neurodegenerative disease.

61. The method of claim 60, wherein the neurodegenerative disease is dementia.

62. The method of claim 61, wherein the dementia is vascular dementia.

63. The method of claim 61, wherein the dementia is Alzheimer's disease.

64. The method of claim 61, wherein the dementia is Lewy body dementia.

65. The method of claim 61, wherein the dementia is frontotemporal dementia.

66. The method of claims 61-65, wherein the method prevents, treats, and improves cognitive dysfunction in the subject, including aspects of memory, visual space, and comprehension judgment, in particular cognitive dysfunction in the aspect of visual space.

67. The method of claims 61-65, wherein the method prevents and impedes development of dementia.

68. The method of claims 61-65, wherein the method improves cerebral tissue perfusion in the subject.

69. The method of claims 61-65, wherein the method inhibits reduction in regional cerebral blood volume (rCBV) around cerebral tissue infarction.

70. The method of claims 61-65, wherein the method improves a condition of ischemia.

71. The method of claims 61-65, wherein the method saves ischemic penumbra.

72. The method of claims 61-65, wherein the method reduces brain edema/infarction volume in the subj ect.

73. The method of claims 61-65, wherein the method inhibits central nervous inflammation.

74. The method of claims 61-65, wherein the method inhibits GFAP overexpression.

75. The method of claims 61-65, wherein the method reduces neurobehavioral score.

76. The method of claims 61-65, wherein the method improves neurological deficits.

77. The method of claims 61-65, wherein the method reduces the overall disability rate of the subject.

78. The method of claim 7, wherein the central nervous system disease, disorder, or condition is optic neuropathy.

79. The method of claim 78, wherein the method inhibits or relieves retinal ganglion cell (RGC) injury in the subject.

80. The method of claim 78, wherein the method inhibits or relieves severe axonal injury of ganglion cells in the subject.

81. The method of claims 79-80, wherein the method treats, prevents, or improves a disease, disorder, or condition associated with retinal ganglion cell (RGC) injury or severe axonal injury of ganglion cells.

82. The method of claim 81, wherein the disease, disorder, or condition includes glaucoma, ischemic optic neuropathy, optic neuritis, optic nerve tumor, traumatic optic neuropathy, retinal artery occlusion, retinal vein occlusion, and retinopathy of prematurity.

83. The method of claim 78, wherein the optic neuropathy is glaucoma.

84. The method of claim 83, wherein the glaucoma is primary glaucoma.

85. The method of claim 84, wherein the primary glaucoma is open-angle glaucoma.

86. The method of claim 84, wherein the primary glaucoma is angle-closure glaucoma.

87. The method of claim 84, wherein the primary glaucoma is a special type of glaucoma.

88. The method of claim 83, wherein the glaucoma is secondary glaucoma.

89. The method of claim 83, wherein the glaucoma is developmental glaucoma.

90. The method of claim 78, wherein the optic neuropathy is ischemic optic neuropathy.

91. The method of claim 90, wherein the ischemic optic neuropathy is anterior ischemic optic neuropathy.

92. The method of claim 90, wherein the ischemic optic neuropathy is posterior ischemic optic neuropathy.

93. The method of claims 83-92, wherein the method mitigates loss of retinal ganglion cells (RGC).

94. The method of claims 83-92, wherein the method protects visual function.

95. The method of claims 83-92, wherein the method inhibits or reduces severity of secondary neuronal injury.

96. The method of claims 83-92, wherein the method mitigates internal retinal injury.

97. The method of claims 83-92, wherein the method reduces retinal edema.

98. The method of claims 83-92, wherein the method mitigates retinal thickness reduction.

99. The method of claims 83-92, wherein the method reduces edema of retinal nerve fiber layer.

100. The method of claims 83-92, wherein the method maintains thickness of retinal nerve fiber layer.

101. The method of claims 83-92, wherein the method prevents thinning of retinal nerve fiber layer.

102. The method of claims 83-92, wherein the method inhibits optic nerve inflammation.

103. The method of claims 83-92, wherein the method inhibits GFAP overexpression.

104. The method of claims 83-92, wherein the method inhibits the expression level of at least one inflammatory factor associated with nerve cell injury, the inflammatory factors comprising IFN-γ, IL-1β, and TNF-α.

105. The method of claims 83-92, wherein the method relieves optic nerve function decline.

106. The method of claims 83-92, wherein the method improves a functional index of full-field electroretinogram (FERG).

107. The method of claims 83-92, wherein the method improves a functional index of flash visual evoked potential (FVEP).

108. The method of claims 83-92, wherein the method protects overall retinal function.

109. The method of claim 78, wherein the optic neuropathy is optic neuritis.

110. The method of claim 78, wherein the optic neuropathy is an optic nerve tumor.

111. The method of claim 78, wherein the optic neuropathy is traumatic optic neuropathy.

112. The method of claims 109-111, wherein the method inhibits or relieves retinal ganglion cell (RGC) injury in the subject.

113. The method of claims 109-111, wherein the method inhibits or relieves severe axonal injury of ganglion cells in the subject.

114. The method of claims 109-111, wherein the method inhibits GFAP overexpression.

115. The method of claims 109-111, wherein the method inhibits I/R-induced overexpression of GFAP.

116. The method of claims 109-111, wherein the method inhibits the expression level of at least one inflammatory factor associated with nerve cell injury, the inflammatory factors comprising IFN-γ, IL-1β, and TNF-α.

117. The method of claim 7, wherein the central nervous system disease, disorder, or condition is retinopathy.

118. The method of claim 117, wherein the method inhibits or relieves retinal ganglion cell (RGC) injury in the subject.

119. The method of claim 117, wherein the method inhibits or relieves severe axonal injury of ganglion cells in the subject.

120. The method of claims 118-119, wherein the method treats, prevents, or improves a disease, disorder, or condition associated with retinal ganglion cell (RGC) injury or severe axonal injury of ganglion cells.

121. The method of claim 120, wherein the disease, disorder, or condition includes glaucoma, ischemic optic neuropathy, optic neuritis, optic nerve tumor, traumatic optic neuropathy, retinal artery occlusion, retinal vein occlusion, and retinopathy of prematurity.

122. The method of claim 117, wherein the retinopathy is a retinal vascular occlusion disease.

123. The method of claim 122, wherein the retinal vascular occlusion disease is retinal artery occlusion.

124. The method of claim 123, wherein the retinal artery occlusion is central artery occlusion.

125. The method of claim 123, wherein the retinal artery occlusion is branch artery occlusion.

126. The method of claim 123, wherein the retinal artery occlusion is ciliary artery occlusion.

127. The method of claim 123, wherein the retinal artery occlusion is retinal precapillary arteriolar occlusion.

128. The method of claim 122, wherein the retinal vascular occlusion disease is retinal vein occlusion.

129. The method of claim 128, wherein the retinal vein occlusion is central vein occlusion.

130. The method of claim 128, wherein the retinal vein occlusion is branch vein occlusion.

131. The method of claim 117, wherein the retinopathy is retinopathy of prematurity.

132. The method of claims 123-127 and 131, wherein the method mitigates loss of retinal ganglion cells (RGC).

133. The method of claims 123-127 and 131, wherein the method protects visual function.

134. The method of claims 123-127 and 131, wherein the method inhibits or reduces severity of secondary neuronal injury.

135. The method of claims 123-127 and 131, wherein the method mitigates internal retinal injury.

136. The method of claims 123-127 and 131, wherein the method reduces retinal edema.

137. The method of claims 123-127 and 131, wherein the method mitigates retinal thickness reduction.

138. The method of claims 123-127 and 131, wherein the method reduces edema of retinal nerve fiber layer.

139. The method of claims 123-127 and 131, wherein the method maintains thickness of retinal nerve fiber layer.

140. The method of claims 123-127 and 131, wherein the method prevents thinning of retinal nerve fiber layer.

141. The method of claims 123-127 and 131, wherein the method inhibits optic nerve inflammation.

142. The method of claims 123-127 and 131, wherein the method inhibits GFAP overexpression.

143. The method of claims 123-127 and 131, the method inhibits the expression level of at least one inflammatory factor associated with nerve cell injury, the inflammatory factors comprising IFN-γ, IL-1β and TNF-α.

144. The method of claims 123-127 and 131, wherein the method relieves optic nerve function decline.

145. The method of claims 123-127 and 131, wherein the method improves a functional index of full-field electroretinogram (FERG).

146. The method of claims 123-127 and 131, wherein the method improves a functional index of flash visual evoked potential (FVEP).

147. The method of claims 123-127 and 131, wherein the method protects overall retinal function.

148. The method of claim 6, wherein the disease, disorder, or condition is peripheral neuropathy.

149. The method of claim 148, wherein the peripheral neuropathy is diabetic peripheral neuropathy.

150. The method of claim 149, wherein the diabetic peripheral neuropathy is diabetic distal symmetrical polyneuropathy.

151. The method of claim 149, wherein the diabetic peripheral neuropathy is diabetic mononeuropathy or multiple mononeuropathy.

152. The method of claims 148-151, wherein the subject exhibits symptoms selected from a group consisting of: symmetrical symptom manifestation, distal limb sensory abnormalities, both lower limb sensory abnormalities, stocking anesthesia, glove anesthesia, median nerve, ulnar nerve, common peroneal nerve involvement, sensory dysfunction, motor dysfunction, insensitivity to trauma, and neuropathic pain.

153. The method of claim 149, wherein the diabetic peripheral neuropathy is diabetic painful peripheral neuropathy.

154. The method of claim 153, wherein the sleep of the subject is affected by pain.

155. The method of claim 153 or 154, wherein the subject suffers from pain-induced depression and anxiety.

156. The method of claims 148-155, wherein the method improves peripheral nerve conduction velocity.

157. The method of claims 148-156, wherein the method significantly improves sensory nerve conduction velocity of sural nerve.

158. The method of claims 148-155, wherein the method produces effects on the subject selected from a group consisting of: improving microcirculation, inhibiting neuroinflammation, inhibiting central nervous inflammation, repairing damaged nerve tissue, relieving symptoms of peripheral neuropathy for a long term, controlling the progression of neuropathy for a long term, and controlling the progression of neuropathic pain for a long term.

159. The method of claims 1-158, wherein the subject is a mammal.

160. The method of claim 159, wherein the mammal is a primate.

161. The method of claim 160, wherein the primate is a human.

162. The method of claims 1-161, wherein the method comprises administering to a subject a therapeutically effective amount of doxazosin, a pharmaceutically acceptable salt or an acceptable form thereof, in combination with a therapeutically effective amount of pramipexole, a pharmaceutically acceptable salt or an acceptable form thereof, and further in combination with a therapeutically effective amount of metoprolol, a pharmaceutically acceptable salt or an acceptable form thereof.

163. The method of claims 1-161, wherein a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof are administered to the subject simultaneously.

164. The method of claims 1-161, wherein a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof are administered to the subject separately.

165. The method of claims 1-161, wherein the therapeutically effective amount of doxazosin, a pharmaceutically acceptable salt or an acceptable form thereof, the therapeutically effective amount of pramipexole, a pharmaceutically acceptable salt or an acceptable form thereof, and the therapeutically effective amount of metoprolol, a pharmaceutically acceptable salt or an acceptable form thereof are formulated into a single pharmaceutical composition.

166. The method of claims 1-165, wherein the therapeutically effective amount of doxazosin, a pharmaceutically acceptable salt or an acceptable form thereof, the therapeutically effective amount of pramipexole, a pharmaceutically acceptable salt or an acceptable form thereof, the therapeutically effective amount of metoprolol, a pharmaceutically acceptable salt or an acceptable form thereof, or the formulated single pharmaceutical composition is formulated as an oral dosage form, an intravenous dosage form, a subcutaneous dosage form, or a fundal injection dosage form.

167. The method of claims 1-165, wherein the therapeutically effective amount of doxazosin, a pharmaceutically acceptable salt or an acceptable form thereof, the therapeutically effective amount of pramipexole, a pharmaceutically acceptable salt or an acceptable form thereof, the therapeutically effective amount of metoprolol, a pharmaceutically acceptable salt or an acceptable form thereof, or the formulated single pharmaceutical composition is formulated as an oral dosage form.

168. The method of claims 1-167, wherein the therapeutically effective amount of doxazosin, a pharmaceutically acceptable salt or an acceptable form thereof, the therapeutically effective amount of pramipexole, a pharmaceutically acceptable salt or an acceptable form thereof, the therapeutically effective amount of metoprolol, a pharmaceutically acceptable salt or an acceptable form thereof, or the formulated single pharmaceutical composition is formulated into tablets, capsules, syrups, and suspensions; intravenous injection, subcutaneous injection, intramuscular injection, and intraperitoneal injection; creams, jellies, powders, patches; inhalation powders, sprays, suspensions or rectal suppositories.

169. The method of claims 1-167, wherein the therapeutically effective amount of doxazosin, a pharmaceutically acceptable salt or an acceptable form thereof, the therapeutically effective amount of pramipexole, a pharmaceutically acceptable salt or an acceptable form thereof, the therapeutically effective amount of metoprolol, a pharmaceutically acceptable salt or an acceptable form thereof, or the formulated single pharmaceutical composition is formulated into tablets, capsules, syrups, and suspensions.

170. The method of claims 1-167, wherein the therapeutically effective amount of doxazosin, a pharmaceutically acceptable salt or an acceptable form thereof, the therapeutically effective amount of pramipexole, a pharmaceutically acceptable salt or an acceptable form thereof, the therapeutically effective amount of metoprolol, a pharmaceutically acceptable salt or an acceptable form thereof, or the formulated single pharmaceutical composition is formulated into tablets.

171. The method of claims 1-170, wherein the therapeutically effective amount of doxazosin, a pharmaceutically acceptable salt or an acceptable form thereof, the therapeutically effective amount of pramipexole, a pharmaceutically acceptable salt or an acceptable form thereof, the therapeutically effective amount of metoprolol, a pharmaceutically acceptable salt or an acceptable form thereof, or the formulated single pharmaceutical composition is administered to the subject orally, intravenously, subcutaneously, or intramuscularly.

172. The method of claims 1-170, wherein the therapeutically effective amount of doxazosin, a pharmaceutically acceptable salt or an acceptable form thereof, the therapeutically effective amount of pramipexole, a pharmaceutically acceptable salt or an acceptable form thereof, the therapeutically effective amount of metoprolol, a pharmaceutically acceptable salt or an acceptable form thereof, or the formulated single pharmaceutical composition is administered orally to the subject.

173. The method of claims 1-172, wherein the pharmaceutically acceptable salt is selected from hydrochloride, sulfate, phosphate, pyrophosphate, hydrobromide, nitrate, citrate, fumarate, maleate, malate, ascorbate, succinate, tartrate, benzoate, acetate, methanesulfonate, ethanesulfonate, salicylate, stearate, benzenesulfonate, or p-toluenesulfonate.

174. The method of claims 1-173, wherein the pharmaceutically acceptable salt form of doxazosin is methanesulfonate.

175. The method of claims 1-173, wherein the pharmaceutically acceptable salt form of pramipexole is hydrochloride.

176. The method of claims 1-173, wherein the pharmaceutically acceptable salt form of metoprolol is tartrate.

177. The method of claims 1-176, wherein the daily dosage of doxazosin ranges from 0.4 mg to 16 mg.

178. The method of claims 1-176, wherein the daily dosage of doxazosin ranges from 0.5 mg to 8 mg.

179. The method of claims 1-176, wherein the daily dosage of pramipexole ranges from 0.03 mg to 4.5 mg.

180. The method of claims 1-176, wherein the daily dosage of pramipexole ranges from 0.1 mg to 1mg.

181. The method of claims 1-176, wherein the daily dosage of metoprolol ranges from 2 mg to 200 mg.

182. The method of claims 1-176, wherein the daily dosage of metoprolol ranges from 10 mg to 100 mg.

183. The method of claims 1-182, wherein the therapeutically effective amount of doxazosin, a pharmaceutically acceptable salt or an acceptable form thereof, the therapeutically effective amount of pramipexole, a pharmaceutically acceptable salt or an acceptable form thereof, and the therapeutically effective amount of metoprolol, a pharmaceutically acceptable salt or an acceptable form thereof are administered to a subject at a frequency of 1, 2, 3, or 4 times per day.

184. The method of claims 1-183, wherein the therapeutically effective amount of doxazosin, a pharmaceutically acceptable salt or an acceptable form thereof, the therapeutically effective amount of pramipexole, a pharmaceutically acceptable salt or an acceptable form thereof, and the therapeutically effective amount of metoprolol, a pharmaceutically acceptable salt or an acceptable form thereof are administered to a subject in a form of a long-acting oral preparation.

185. A method for treating, preventing, or improving cerebrovascular disease, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

186. The method of claim 185, wherein the cerebrovascular disease is stroke.

187. The method of claim 186, wherein the stroke is ischemic stroke.

188. The method of claims 185-187, wherein the subject is a subject within 7 hours after a stroke attack.

189. The method of claims 185-188, wherein the subject has a National Institute of Health stroke scale (NIHSS) of 20 or within 20.

190. The method of claims 185-188, wherein the subject has a National Institute of Health stroke scale (NIHSS) of 15 or within 15.

191. The method of claims 185-188, wherein the subject has a National Institute of Health stroke scale (NIHSS) of 8 to 15.

192. The method of claims 185-191, wherein the subject is a subject with moderate to severe stroke and persistent neurological impairment.

193. The method of claims 185-192, wherein the subject is a subj ect with anterior circulation large vessel occlusion.

194. The method of claims 185-193, wherein the subject has a symptom of occlusion in the M1 segment of the middle cerebral artery.

195. The method of claims 185-193, wherein the subject has a symptom of occlusion in the M2 segment of the middle cerebral artery.

196. The method of claims 185-195, wherein the subject is a subject with ischemia-reperfusion injury.

197. The method of claim 196, wherein the reperfusion is performed by intravascular therapy.

198. The method of claim 196, wherein the reperfusion is performed by administration of a thrombolytic agent.

199. The method of claim 196, wherein the reperfusion is performed by angioplasty.

200. The method of claims 185-195, wherein the subject is an ischemic non-reperfusion subj ect.

201. The method of claims 185-195, wherein the subject is a subject with a massive cerebral infarction.

202. The method of claims 185-201, wherein the method improves cerebral tissue perfusion in the subject in an acute phase after stroke.

203. The method of claims 185-201, wherein the method inhibits reduction in regional cerebral blood volume (rCBV) around cerebral tissue infarction.

204. The method of claims 185-201, wherein the method improves ischemic condition in the acute phase after stroke.

205. The method of claims 185-201, wherein the method saves ischemic penumbra.

206. The method of claims 185-201, wherein the method reduces brain edema/infarction volume in the subject.

207. The method of claims 185-201, wherein the method inhibits central nervous inflammation.

208. The method of claims 185-201, wherein the method inhibits overexpression of glial fibrillary acidic protein (GFAP).

209. The method of claims 185-201, wherein the method reduces stroke neurobehavioral score.

210. The method of claims 185-201, wherein the method improves neurological deficits in the subject.

211. The method of claims 185-201, wherein the method improves and restores finger function and limb strength of an affected limb in the subject.

212. The method of claims 185-201, wherein the method reduces disability rate of the subj ect.

213. The method of claims 185-201, wherein the method prevents, treats and improves cognitive dysfunction in the subject, including aspects of memory, visual space, and comprehension judgment.

214. The method of claims 185-201, wherein the method prevents and impedes development of dementia.

215. The method of claims 185-201, wherein the method treats destructive effect of ischemia on the central nervous system of the subject.

216. The method of claim 186, wherein the stroke is hemorrhagic stroke.

217. The method of claim 216, wherein the hemorrhagic stroke is a condition of cerebral hemorrhage.

218. The method of claim 216, wherein the hemorrhagic stroke is a condition of subarachnoid hemorrhage.

219. The method of claim 185, wherein the cerebrovascular disease is a transient ischemic attack.

220. The method of claim 219, wherein the method improves cerebral tissue perfusion in the subject in an acute phase after stroke.

221. The method of claim 219, wherein the method inhibits a reduction in regional cerebral blood volume (rCBV) in an injured brain tissue area.

222. The method of claim 219, wherein the method improves ischemic condition in the acute phase after stroke.

223. The method of claim 219, wherein the method saves ischemic penumbra.

224. The method of claim 219, wherein the method inhibits central nervous inflammation.

225. The method of claim 219, wherein the method inhibits overexpression of glial fibrillary acidic protein (GFAP).

226. The method of claim 219, wherein the method reduces stroke neurobehavioral score.

227. The method of claim 219, wherein the method improves neurological deficits in the subj ect.

228. The method of claim 219, wherein the method improves and restores finger function and limb strength of an affected limb in the subject.

229. The method of claim 219, wherein the method reduces disability rate of the subject.

230. The method of claim 219, wherein the method prevents, treats, and improves cognitive dysfunction in the subject, including aspects of memory, visual space, and comprehension judgment.

231. The method of claim 219, wherein the method prevents and impedes development of dementia.

232. The method of claim 219, wherein the method treats destructive effect of ischemia on the central nervous system of the subject.

233. A method for treating, preventing, or improving craniocerebral injury, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

234. The method of claim 233, wherein the craniocerebral injury is traumatic brain injury.

235. The method of claim 234, wherein the method inhibits central nervous inflammation.

236. The method of claim 234, wherein the method inhibits GFAP overexpression.

237. A method for treating, preventing, or improving a neurodegenerative disease, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

238. The method of claim 237, wherein the neurodegenerative disease is dementia.

239. The method of claim 238, wherein the dementia is vascular dementia.

240. The method of claim 238, wherein the dementia is Alzheimer's disease.

241. The method of claim 238, wherein the dementia is Lewy body dementia.

242. The method of claim 238, wherein the dementia is frontotemporal dementia.

243. The method of claims 237-242, wherein the method prevents, treats, and improves cognitive dysfunction in the subject, including aspects of memory, visual space, and comprehension judgment, in particular cognitive dysfunction in the aspect of visual space.

244. The method of claims 237-242, wherein the method prevents and impedes development of dementia.

245. The method of claims 237-242, wherein the method improves cerebral tissue perfusion in the subj ect.

246. The method of claims 237-242, wherein the method inhibits reduction in regional cerebral blood volume (rCBV) around cerebral tissue infarction.

247. The method of claims 237-242, wherein the method improves a condition of ischemia.

248. The method of claims 237-242, wherein the method saves ischemic penumbra.

249. The method of claims 237-242, wherein the method reduces brain edema/infarction volume in the subject.

250. The method of claims 237-242, wherein the method inhibits central nervous inflammation.

251. The method of claims 237-242, wherein the method inhibits GFAP overexpression.

252. The method of claims 237-242, wherein the method reduces neurobehavioral score.

253. The method of claims 237-242, wherein the method improves neurological deficits.

254. The method of claims 237-242, wherein the method reduces the overall disability rate of the subject.

255. A method for treating, preventing, or improving glaucoma, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

256. The method of claim 255, wherein the glaucoma is primary glaucoma.

257. The method of claim 256, wherein the primary glaucoma is open-angle glaucoma.

258. The method of claim 256, wherein the primary glaucoma is angle-closure glaucoma.

259. The method of claim 256, wherein the primary glaucoma is a special type of glaucoma.

260. The method of claim 255, wherein the glaucoma is secondary glaucoma.

261. The method of claim 255, wherein the glaucoma is developmental glaucoma.

262. The method of claims 255-261, wherein the method mitigates loss of retinal ganglion cells (RGC).

263. The method of claims 255-261, wherein the method protects visual function.

264. The method of claims 255-261, wherein the method inhibits or reduces severity of secondary neuronal injury.

265. The method of claims 255-261, wherein the method mitigates internal retinal injury.

266. The method of claims 255-261, wherein the method reduces retinal edema.

267. The method of claims 255-261, wherein the method mitigates retinal thickness reduction.

268. The method of claims 255-261, wherein the method reduces edema of retinal nerve fiber layer.

269. The method of claims 255-261, wherein the method maintains thickness of retinal nerve fiber layer.

270. The method of claims 255-261, wherein the method prevents thinning of retinal nerve fiber layer.

271. The method of claims 255-261, wherein the method inhibits optic nerve inflammation.

272. The method of claims 255-261, wherein the method inhibits GFAP overexpression.

273. The method of claims 255-261, wherein the method inhibits the expression level of at least one inflammatory factor associated with nerve cell injury, the inflammatory factor being TNF-α, IL-1β, and/or IFN-γ.

274. The method of claims 255-261, wherein the method relieves optic nerve function decline.

275. The method of claims 255-261, wherein the method improves a functional index of full-field electroretinogram (FERG).

276. The method of claims 255-261, wherein the method improves a functional index of flash visual evoked potential (FVEP).

277. The method of claims 255-261, wherein the method protects overall retinal function.

278. A method for treating, preventing, or improving ischemic optic neuropathy, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

279. The method of claim 278, wherein the optic neuropathy is ischemic optic neuropathy.

280. The method of claim 279, wherein the ischemic optic neuropathy is anterior ischemic optic neuropathy.

281. The method of claim 279, wherein the ischemic optic neuropathy is posterior ischemic optic neuropathy.

282. The method of claims 278-281, wherein the method mitigates loss of retinal ganglion cells (RGC).

283. The method of claims 278-281, wherein the method protects visual function.

284. The method of claims 278-281, wherein the method inhibits or reduces severity of secondary neuronal injury.

285. The method of claims 278-281, wherein the method mitigates internal retinal injury.

286. The method of claims 278-281, wherein the method reduces retinal edema.

287. The method of claims 278-281, wherein the method mitigates retinal thickness reduction.

288. The method of claims 278-281, wherein the method reduces edema of retinal nerve fiber layer.

289. The method of claims 278-281, wherein the method maintains thickness of retinal nerve fiber layer.

290. The method of claims 278-281, wherein the method prevents thinning of retinal nerve fiber layer.

291. The method of claims 278-281, wherein the method inhibits optic nerve inflammation.

292. The method of claims 278-281, wherein the method inhibits GFAP overexpression.

293. The method of claims 278-281, wherein the method inhibits the expression level of at least one inflammatory factor associated with nerve cell injury, the inflammatory factors comprising TNF-α, IL-1β, and IFN-γ.

294. The method of claims 278-281, wherein the method relieves optic nerve function decline.

295. The method of claims 278-281, wherein the method improves a functional index of full-field electroretinogram (FERG).

296. The method of claims 278-281, wherein the method improves a functional index of flash visual evoked potential (FVEP).

297. The method of claims 278-281, wherein the method protects overall retinal function.

298. A method for treating, preventing, or improving optic neuritis, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

299. The method of claim 298, wherein the method inhibits or relieves retinal ganglion cell (RGC) injury or severe axonal injury in the subject.

300. The method of claim 298, wherein the method inhibits GFAP overexpression.

301. The method of claim 298, wherein the method inhibits I/R-induced overexpression of GFAP.

302. The method of claim 298, wherein the method inhibits the expression level of at least one inflammatory factor associated with nerve cell injury, the inflammatory factors comprising TNF-α, IL-1β, and IFN-γ.

303. A method for treating, preventing, or improving optic nerve tumors, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

304. The method of claim 303, wherein the method inhibits or relieves retinal ganglion cell (RGC) injury or severe axonal injury in the subject.

305. The method of claim 303, wherein the method inhibits GFAP overexpression.

306. The method of claim 303, wherein the method inhibits I/R-induced overexpression of GFAP.

307. The method of claim 303, wherein the method inhibits the expression level of at least one inflammatory factor associated with nerve cell injury, the inflammatory factors comprising TNF-α, IL-1β, and IFN-γ.

308. A method for treating, preventing, or improving traumatic optic neuropathy, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

309. The method of claim 308, wherein the method inhibits or relieves retinal ganglion cell (RGC) injury or severe axonal injury in the subject.

310. The method of claim 308, wherein the method inhibits GFAP overexpression.

311. The method of claim 308, wherein the method inhibits I/R-induced overexpression of GFAP.

312. The method of claim 308, wherein the method inhibits the expression level of at least one inflammatory factor associated with nerve cell injury, the inflammatory factors comprising TNF-α, IL-1β, and IFN-γ.

313. A method for treating, preventing, or improving retinopathy, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

314. The method of claim 313, wherein the method inhibits or relieves retinal ganglion cell (RGC) injury or severe axonal injury in the subject.

315. The method of claim 313, the method treats, prevents, or improves a disease, disorder, or condition associated with retinal ganglion cell (RGC) injury or severe axonal injury.

316. The method of claim 315, wherein the disease, disorder, or condition includes glaucoma, ischemic optic neuropathy, optic neuritis, optic nerve tumor, traumatic optic neuropathy, retinal artery occlusion, retinal vein occlusion, and retinopathy of prematurity.

317. The method of claim 313, wherein the retinopathy is a retinal vascular occlusion disease.

318. The method of claim 317, wherein the retinal vascular occlusion disease is retinal artery occlusion.

319. The method of claim 318, wherein the retinal artery occlusion is central artery occlusion.

320. The method of claim 318, wherein the retinal artery occlusion is branch artery occlusion.

321. The method of claim 318, wherein the retinal artery occlusion is ciliary artery occlusion.

322. The method of claim 318, wherein the retinal artery occlusion is retinal precapillary arteriolar occlusion.

323. The method of claim 318, wherein the retinal vascular occlusion disease is retinal vein occlusion.

324. The method of claim 323, wherein the retinal vein occlusion is central vein occlusion.

325. The method of claim 323, wherein the retinal vein occlusion is branch vein occlusion.

326. The method of claim 313, wherein the retinopathy is retinopathy of prematurity.

327. The method of claims 318-322 and 326, wherein the method mitigates loss of retinal ganglion cells (RGC).

328. The method of claims 318-322 and 326, wherein the method protects visual function.

329. The method of claims 318-322 and 326, wherein the method inhibits or reduces severity of secondary neuronal injury.

330. The method of claims 318-322 and 326, wherein the method mitigates internal retinal injury.

331. The method of claims 318-322 and 326, wherein the method reduces retinal edema.

332. The method of claims 318-322 and 326, wherein the method mitigates retinal thickness reduction.

333. The method of claims 318-322 and 326, wherein the method reduces edema of retinal nerve fiber layer.

334. The method of claims 318-322 and 326, wherein the method maintains thickness of retinal nerve fiber layer.

335. The method of claims 318-322 and 326, wherein the method prevents thinning of retinal nerve fiber layer.

336. The method of claims 318-322 and 326, wherein the method inhibits optic nerve inflammation.

337. The method of claims 318-322 and 326, wherein the method inhibits GFAP overexpression.

338. The method of claims 318-322 and 326, wherein the method inhibits the expression level of at least one inflammatory factor associated with nerve cell injury, the inflammatory factor being TNF-α, IL-1β, and/or IFN-γ.

339. The method of claims 318-322 and 326, wherein the method relieves optic nerve function decline.

340. The method of claims 318-322 and 326, wherein the method improves a functional index of full-field electroretinogram (FERG).

341. The method of claims 318-322 and 326, wherein the method improves a functional index of flash visual evoked potential (FVEP).

342. The method of claims 318-322 and 326, wherein the method protects overall retinal function.

343. A method for treating, preventing, or improving peripheral neuropathy, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

344. The method of claim 343, wherein the peripheral neuropathy is diabetic peripheral neuropathy.

345. The method of claim 344, wherein the diabetic peripheral neuropathy is diabetic distal symmetrical polyneuropathy.

346. The method of claim 344, wherein the diabetic peripheral neuropathy is diabetic mononeuropathy or multiple mononeuropathy.

347. The method of claims 343-346, wherein the subject exhibits symptoms selected from a group consisting of: symmetrical symptom manifestation, distal limb sensory abnormalities, both lower limb sensory abnormalities, stocking anesthesia, glove anesthesia, median nerve, ulnar nerve, common peroneal nerve involvement, sensory dysfunction, motor dysfunction, insensitivity to trauma, and neuropathic pain.

348. The method of claim 344, wherein the diabetic peripheral neuropathy is diabetic painful peripheral neuropathy.

349. The method of claim 348, wherein sleep of the subject is affected by pain.

350. The method of claim 344 or 348, wherein the subject suffers from pain-induced depression and anxiety.

351. The method of claims 343-350, wherein the method improves peripheral nerve conduction velocity.

352. The method of claims 343-351, wherein the method significantly improves sensory nerve conduction velocity of sural nerve.

353. The method of claims 343-350, wherein the method produces effects on the subject selected from a group consisting of: improving microcirculation, inhibiting neuroinflammation, inhibiting central nervous inflammation, repairing damaged nerve tissue, relieving symptoms of peripheral neuropathy for a long term, controlling the progression of neuropathy for a long term, and controlling the progression of neuropathic pain for a long term.

354. A method for inhibiting neuroinflammation, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

355. The method of claim 354, wherein the method treats, prevents, or improves a disease, disorder, or condition associated with neuroinflammation.

356. The method of claim 355, wherein the disease, disorder, or condition comprises ischemic cerebral stroke, hemorrhagic cerebral stroke, transient ischemic attack, craniocerebral injury, vascular dementia, Alzheimer's disease, Lewy body dementia, frontotemporal dementia, glaucoma, ischemic optic neuropathy, optic neuritis, optic nerve tumor, traumatic optic neuropathy, retinal artery occlusion, retinal vein occlusion, and retinopathy of prematurity.

357. A method for repairing nerve injury, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

358. A method for inhibiting or mitigating retinal ganglion cell (RGC) injury or severe axonal injury in a subject, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

359. The method of claim 358, wherein the method treats, prevents, or improves a disease, disorder, or condition associated with retinal ganglion cell (RGC) injury.

360. The method of claim 359, wherein the disease, disorder, or condition includes glaucoma, ischemic optic neuropathy, optic neuritis, optic nerve tumor, traumatic optic neuropathy, retinal artery occlusion, retinal vein occlusion, and retinopathy of prematurity.

361. Use of a pharmaceutical composition in the preparation of a medicament for modulating neuropathy, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

362. Use of a pharmaceutical composition in the preparation of a medicament for inhibiting neuroinflammation, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

363. Use of a pharmaceutical composition in the preparation of a medicament for repairing nerve injury, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

364. Use of a pharmaceutical composition in the preparation of a medicament for treating, preventing, or improving cerebrovascular diseases, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

365. Use of a pharmaceutical composition in the preparation of a medicament for treating, preventing, or improving craniocerebral injury, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

366. Use of a pharmaceutical composition in the preparation of a medicament for treating, preventing, or improving neurodegenerative diseases, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

367. Use of a pharmaceutical composition in the preparation of a medicament for treating, preventing, or improving glaucoma, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

368. Use of a pharmaceutical composition in the preparation of a medicament for treating, preventing, or improving ischemic optic neuropathy, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

369. Use of a pharmaceutical composition in the preparation of a medicament for treating, preventing, or improving optic neuritis, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

370. Use of a pharmaceutical composition in the preparation of a medicament for treating, preventing, or improving optic nerve tumors, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

371. Use of a pharmaceutical composition in the preparation of a medicament for treating, preventing, or improving traumatic optic neuropathy, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

372. Use of a pharmaceutical composition in the preparation of a medicament for treating, preventing, or improving retinopathy, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

373. Use of a pharmaceutical composition in the preparation of a medicament for inhibiting or mitigating retinal ganglion cell (RGC) injury or severe axonal injury in a subject, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

374. Use of a pharmaceutical composition in the preparation of a medicament for treating, preventing, or improving peripheral neuropathy, wherein the use comprises administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.

375. Use of a pharmaceutical composition in the preparation of a medicament for treating, preventing, or improving diabetic peripheral neuropathy, comprising administering to a subject a therapeutically effective amount of doxazosin, a pharmaceutically acceptable salt or an acceptable form thereof, a therapeutically effective amount of pramipexole, a pharmaceutically acceptable salt or an acceptable form thereof, and a therapeutically effective amount of metoprolol, a pharmaceutically acceptable salt or an acceptable form thereof.
